# EUROPEAN PATENT SPECIFICATION

(11) **EP 1 257 276 B1**
(45) Date of publication and mention of the grant of the patent: **28.12.2005**
(21) Application number: 01904970.9
(22) Date of filing: 19.01.2001
(51) Int. Cl.: A61K 31/495, C07D 471/04, A61P 31/00, A61P 31/18

(54) **ANTIVIRAL AZAINDOLE DERIVATIVES**
ANTIVIRALE AZAINDOLDERIVATE
DERIVES AZA-INDOLES, ANTIVIRAUX

(30) Priority: 22.02.2000 US 184004 P
(43) Date of publication of application: 20.11.2002
(73) Proprietor: Bristol-Myers Squibb Company, Princeton, NJ 08543-4000 (US)
(72) Inventor: WANG, Tao, Middletown, CT 06457 (US); WALLACE, Owen, B., Zionsville, IN 46077 (US); ZHANG, Zhongxing, Madison, CT 06443 (US); MEANWELL, Nicholas, A., East Hampton, CT 06424 (US); BENDER, John, A., Middletown, CT 06457 (US)
(74) Representative: Modiano, Micaela Nadia
(86) International application number: PCT/US2001/002009
(87) International publication number: WO 2001/062255

(56) References cited:
- ROMERO ET AL.: 'Bis(heteroaryl)piperazine (BHAP) reverse transcriptase inhibitors ..... a second-generation clinical candidate' J. MED. CHEM. vol. 36, 1993, pages 1505 - 1508, XP002939942

## Description

### Field of the Invention

This invention provides compounds having drug and bio-affecting properties, their pharmaceutical compositions and use. In particular, the invention is concerned with azaindole piperazine diamide derivatives that possess unique antiviral activity. More particularly, the present invention relates to compounds useful for the treatment of HIV and AIDS.

### Background Art

HIV-1 (human immunodeficiency virus -1) infection remains a major medical problem, with an estimated 33.6 million people infected worldwide. The number of cases of HIV and AIDS (acquired immunodeficiency syndrome) has risen rapidly. In 1999, 5.6 million new infections were reported, and 2.6 million people died from AIDS. Currently available drugs for the treatment of HIV include six nucleoside reverse transcriptase (RT) inhibitors (zidovudine, didanosine, stavudine, lamivudine, zalcitabine and abacavir), three non-nucleoside reverse transcriptase inhibitors (nevirapine, delavirdine and efavirenz), and five peptidomimetic protease inhibitors (saquinavir, indinavir, ritonavir, nelfinavir and amprenavir). Each of these drugs can only transiently restrain viral replication if used alone. However, when used in combination, these drugs have a profound effect on viremia and disease progression. In fact, significant reductions in death rates among AIDS patients have been recently documented as a consequence of the widespread application of combination therapy. However, despite these impressive results, 30 to 50% of patients ultimately fail combination drug therapies. Insufficient drug potency, non-compliance, restricted tissue penetration and drug-specific limitations within certain cell types (e.g. most nucleoside analogs cannot be phosphorylated in resting cells) may account for the incomplete suppression of sensitive viruses. Furthermore, the high replication rate and rapid turnover of HIV-1 combined with the frequent incorporation of mutations, leads to the appearance of drug-resistant variants and treatment failures when sub-optimal drug concentrations are present (Larder and Kemp; Gulick; Kuritzkes; Morris-Jones *et al;* Schinazi *et al;* Vacca and Condra; Flexner; Berkhout and Ren *et al;* (Ref. 6-14)). Therefore, novel anti-HIV agents exhibiting distinct resistance patterns, and favorable pharmacokinetic as well as safety profiles are needed to provide more treatment options.

Currently marketed HIV-1 drugs are dominated by either nucleoside reverse transcriptase inhibitors or peptidomimetic protease inhibitors. Non-nucleoside reverse transcriptase inhibitors (NNRTIs) have recently gained an increasingly important role in the therapy of HIV infections (Pedersen & Pedersen, Ref. 15). At least 30 different classes of NNRTI have been described in the literature (De Clercq, Ref. 16) and several NNRTIs have been evaluated in clinical trials. Dipyridodiazepinone (nevirapine), benzoxazinone (efavirenz) and bis(heteroaryl) piperazine derivatives (delavirdine) have been approved for clinical use. However, the major drawback to the development and application of NNRTIs is the propensity for rapid emergence of drug resistant strains, both in tissue cell culture and in treated individuals, particularly those subject to monotherapy. As a consequence, there is considerable interest in the identification of NNRTIs less prone to the development of resistance (Pedersen & Pedersen, Ref. 15).

Several indole derivatives including indole-3-sulfones, piperazino indoles, pyrazino indoles, and 5H-indolo[3,2-b][1,5]benzothiazepine derivatives have been reported as HIV-1 reverse transciptase inhibitors (Greenlee et al, Ref. 1; Williams et al, Ref. 2; Romero et al, Ref. 3; Font et al, Ref. 17; Romero et al, Ref. 18; Young et al, Ref. 19; Genin et al, Ref. 20; Silvestri et al, Ref. 21). Indole 2-carboxamides have also been described as inhibitors of cell adhesion and HIV infection (Boschelli et al, US 5,424,329, Ref. 4). Finally, 3-substituted indole natural products (Semicochliodinol A and B, didemethylasterriquinone and isocochliodinol) were disclosed as inhibitors of HIV-1 protease (Fredenhagen et al, Ref. 22).

Structurally related aza-indole amide derivatives have been disclosed previously (Kato et al, Ref. 23; Levacher et al, Ref. 24; Mantovanini et al, Ref. 5(a); Cassidy et al, Ref. 5(b); Scherlock et al, Ref. 5(c)). However, these structures differ from those claimed herein in that they are aza-indole mono-amides rather than unsymmetrical aza-indole piperazine diamide derivatives, and there is no mention of the use of these compounds for treating antiviral infections, particularly HIV. Nothing in these references can be construed to disclose or suggest the novel compounds of this invention and their use to inhibit HIV infection.

### REFERENCES CITED

### Patent documents

1. Greenlee, W.J.; Srinivasan, P.C. Indole reverse transcriptase inhibitors. U.S. Patent 5,124,327.
2. Williams, T.M.; Ciccarone, T.M.; Saari, W. S.; Wai, J.S.; Greenlee, W.J.; Balani, S.K.; Goldman, M.E.; Theohrides, A.D. Indoles as inhibitors of HIV reverse transcriptase. European Patent 530907.
3. Romero, D.L.; Thomas, R.C.; Preparation of substituted indoles as anti-AIDS pharmaceuticals. PCT WO 93 / 01181.
4. Boschelli, D.H.; Connor, D.T.; Unangst, P.C. Indole-2-carboxamides as inhibitors of cell adhesion. U.S. Patent 5,424,329.
5. (a) Mantovanini, M.; Melillo, G.; Daffonchio, L. Tropyl 7-azaindol-3-ylcarboxyamides as antitussive agents. PCT WO 95/04742 (Dompe Spa). (b) Cassidy, F.; Hughes, I.; Rahman, S.; Hunter, D. J. Bisheteroaryl-carbonyl and carboxamide derivatives with 5HT 2C/2B antagonists activity. PCT WO 96/11929. (c) Scherlock, M. H.; Tom, W. C. Substituted 1*H*-pyrrolopyridine-3-carboxamides. U. S. Patent 5,023,265.

### Other Publications

6. Larder, B.A.; Kemp, S.D. Multiple mutations in the HIV-1 reverse transcriptase confer high-level resistance to zidovudine (AZT). *Science,* **1989,** *246*, 1155-1158.
7. Gulick, R.M. Current antiretroviral therapy: An overview. *Quality of Life Research,* **1997,** *6*, 471-474.
8. Kuritzkes, D.R. HIV resistance to current therapies. *Antiviral Therapy,* **1997,** ***2*** (Supplement 3), 61-67.
9. Morris-Jones, S.; Moyle, G.; Easterbrook, P.J. Antiretroviral therapies in HIV-1 infection. *Expert Opinion on Investigational Drugs,* **1997,** *6*(8), 1049-1061.
10. Schinazi, R.F.; Larder, B.A.; Mellors, J.W. Mutations in retroviral genes associated with drug resistance. *International Antiviral News,* **1997,** *5*, 129-142.
11. Vacca, J.P.; Condra, J.H. Clinically effective HIV-1 protease inhibitors. *Drug Discovery Today,* **1997,** *2*, 261-272.
12. Flexner, D. HIV-protease inhibitors. *Drug Therapy,* **1998,** *338*, 1281-1292.
13. Berkhout, B. HIV-1 evolution under pressure of protease inhibitors: Climbing the stairs of viral fitness. *J. Biomed. Sci.,* **1999,** *6,* 298-305.
14. Ren; S.; Lien, E. J. Development of HIV protease inhibitors: A survey. *Prog. Drug Res.,* **1998,** *51,* 1-31.
15. Pedersen, O.S.; Pedersen, E.B. Non-nucleoside reverse transcriptase inhibitors: the NNRTI boom. *Antiviral Chem. Chemother.* **1999,** *10*, 285-314.
16. (a) De Clercq, E. The role of non-nucleoside reverse transcriptase inhibitors (NNRTls) in the therapy of HIV-1 infection. *Antiviral Research,* **1998**, *38*, 153-179. (b) De Clercq, E. Perspectives of non-nucleoside reverse transcriptase inhibitors (NNRTls) in the therapy of HIV infection. IL. *Farmaco,* **1999**, *54*, 26-45.
17. Font, M.; Monge, A.; Cuartero, A.; Elorriaga, A.; Martinez-Irujo, J.J.; Alberdi, E.; Santiago, E.; Prieto, I.; Lasarte, J.J.; Sarobe, P. and Borras, F. Indoles and pyrazino[4,5-*b*]indoles as nonnucleoside analog inhibitors of HIV-1 reverse transcriptase. *Eur. J. Med. Chem.,* **1995,** *30*, 963-971.
18. Romero, D.L.; Morge, R.A.; Genin, M.J.; Biles, C.; Busso, M,; Resnick, L.; Althaus, I.W.; Reusser, F.; Thomas, R.C and Tarpley, W.G. Bis(heteroaryl)piperazine (BHAP) reverse transcriptase inhibitors: structure-activity relationships of novel substituted indole analogues and the identification of 1-[(5-methanesulfonamido-1H-indol-2-yl)-carbonyl]-4-[3-[1-methylethyl)amino]-pyridinyl]piperazine momomethansulfonate (U-90152S), a second generation clinical candidate. *J. Med. Chem.,* **1993,** *36*, 1505-1508.
19. Young, S.D.; Amblard, M.C.; Britcher, S.F.; Grey, V.E.; Tran, L.O.; Lumma, W.C.; Huff, J.R.; Schleif, W.A.; Emini, E.E.; O'Brien, J.A.; Pettibone, D.J. 2-Heterocyclic indole-3-sulfones as inhibitors of HIV-reverse transcriptase. *Bioorg. Med. Chem. Lett.,* **1995,** *5*, 491-496.
20. Genin, M.J.; Poel, T.J.; Yagi, Y.; Biles, C.; Althaus, I.; Keiser, B.J.; Kopta, L.A.; Friis, J.M.; Reusser, F.; Adams, W.J.; Olmsted, R.A.; Voorman, R.L.; Thomas, R.C. and Romero, D.L. Synthesis and bioactivity of novel bis(heteroaryl)piperazine (BHAP) reverse transcriptase inhibitors: structure-activity relationships and increased metabolic stability of novel substituted pyridine analogs. *J*. *Med. Chem.,* **1996,** *39*, 5267-5275.
21. Silvestri, R.; Artico, M.; Bruno, B.; Massa, S.; Novellino, E.; Greco, G.; Marongiu, M.E.; Pani, A.; De Montis, A and La Colla, P. Synthesis and biological evaluation of 5*H*-indolo[3,2-*b*][1,5]benzothiazepine derivatives, designed as conformationally constrained analogues of the human immunodeficiency virus type 1 reverse transcriptase inhibitor L-737,126. *Antiviral Chem. Chemother.* **1998,** *9*, 139-148.
22. Fredenhagen, A.; Petersen, F.; Tintelnot-Blomley, M.; Rosel, J.; Mett, H and Hug, P. J. Semicochliodinol A and B: Inhibitors of HIV-1 protease and EGF-R protein Tyrosine Kinase related to Asterriquinones produced by the fungus *Chrysosporium nerdarium. Antibiotics,* **1997,** *50,* 395-401.
23. Kato, M.; Ito, K.; Nishino, S.; Yamakuni, H.; Takasugi, H. New 5-HT₃ (Serotonin-3) receptor antagonists. IV. Synthesis and structure-activity relationships of azabicycloalkaneacetamide derivatives. *Chem*. *Pharm. Bull.,* **1995,** *43*, 1351-1357.
24. Levacher, V.; Benoit, R.; Duflos, J; Dupas, G.; Bourguignon, J.; Queguiner, G. Broadening the scope of NADH models by using chiral and non chiral pyrrolo [2,3-*b*] pyridine derivatives. *Tetrahedron,* **1991,** *47*, 429-440.
25. (a) Mahadevan, I; Rasmussen, M. Synthesis of pyrrolopyridines (Azaindoles). *J. Het. Chem.,* **1992,** 29, 359-367. (b) Hands, D.; Bishop, B.; Cameron, M.; Edwards, J. S.; Cottrell, I. F.; Wright, S. H. B. A convient method for the preparation of 5-, 6- and 7-azaindoles and their derivatives. *Synthesis,* **1996,** 877-882. (c) Dobson, D.; Todd, A.; Gilmore, J. The Synthesis of 7-Alkoxyindoles. *Synth. Commun.* **1991**, 21, 611-617.
26. Sakamoto, T; Kondo, Y; Iwashita, S; Yamanaka, H Condensed Heteroaromatic Ring Systems. XII. Synthesis of Indole Derivatives from Ethyl 2-Bromocarbanilates. *Chem. Pharm. Bull.* **1987,** *35*, 1823-1828
27. Shadrina, L.P.; Dormidontov, Yu.P.; Ponomarev, V,G.; Lapkin, I.I. Reactions of organomagnesium derivatives of 7-aza- and benzoindoles with diethyl oxalate and the reactivity of ethoxalylindoles. *Khim. Geterotsikl. Soedin.,* **1987,** 1206-1209.
28. Sycheva, T.V.; Rubtsov, N.M.; Sheinker, Yu.N.; Yakhontov, L.N. Some reactions of 5-cyano-6-chloro-7-azaindoles and lactam-lactim tautomerism in 5-cyano-6-hydroxy-7-azaindolines. *Khim. Geterotsikl. Soedin.,* **1987**, 100-106.
29. Li, H.; Jiang, X.; Ye, Y.-H.; Fan, C.; Romoff, T.; Goodman, M. 3-(Diethoxyphosphoryloxy)-1,2,3-benzotriazin-4(3*H*)-one (DEPBT): A new coupling reagent with remarkable resistance to racemization. Organic *Lett.,* **1999,** *1*,91-93.
30. (a) Desai, M.; Watthey, J.W.H.; Zuckerman, M. A convenient preparation of 1-aroylpiperazines. *Org. Prep. Proced. lnt.,* **1976,** *8*, 85-86. (b) Adamczyk, M.; Fino, J.R. Synthesis of procainamide metabolites. N-acetyl desethylprocainamide and desethylprocainamide. *Org. Prep. Proced. Int.* **1996,** *28*, 470-474. (c) Rossen, K.; Weissman, S.A.; Sager, J.; Reamer, R.A.; Askin, D.; Volante, R.P.; Reider, P.J. Asymmetric Hydrogenation of tetrahydropyrazines: Synthesis of (S)-piperazine 2-tert-butylcarboxamide, an intermediate in the preparation of the HIV protease inhibitor Indinavir. *Tetrahedron Lett*., **1995,** *36*, 6419-6422. (d) Wang, T.; Zhang, Z.; Meanwell, N.A. Benzoylation of Dianions: Preparation of mono-Benzoylated Symmetric Secondary Diamines. *J. Org. Chem.,* **1999,** *64,* 7661-7662. (e) Wang, T.; Zhang, Z.; Meanwell, N.A. Regioselective mono-Benzoylation of Unsymmetrical Piperazines. *J*. *Org. Chem.* **2000,** *65*, 4740-4742.
31. Harada, N.; Kawaguchi, T.; Inoue, I.; Ohashi, M.; Oda, K.; Hashiyama, T.; Tsujihara, K. Synthesis and antitumor activity of quaternary salts of 2-(2'-oxoalkoxy)-9-hydroxyellipticines. *Chem. Pharm. Bull*., **1997,** *45,* 134-137.
32. Antonini, I.; Claudi, F.; Cristalli, G.; Franchetti, P.; Crifantini, M.; Martelli, S. Synthesis of 4-amino-1-β-D-ribofuranosyl-1*H*-pyrrolo[2,3-b]pyridine (1-Deazatubercidin) as a potential antitumor agent. *J. Med. Chem.,* **1982,** *25*, 1258-1261.
33. (a) Schneller, S. W.; Luo, J.-K. Synthesis of 4-amino-1*H*-pyrrolo[2,3-*b*]pyridine (1,7-Dideazaadenine) and 1*H*-pyrrolo[2,3-*b*]pyridin-4-ol (1,7-Dideazahypoxanthine). *J. Org. Chem.,* **1980**, *45*, 4045-4048. (b) Wozniak, M.; Grzegozek, M. Amination of 4-nitroquinoline with liquid methylamine/Potassium Permanganate *Chemistry of Heterocyclic Compounds* **1998**, 837-840.
34. Shiotani, S.; Tanigochi, K. Furopyridines. XXII [1]. Elaboration of the C-substitutents *alpha* to the heteronitrogen atom of furo[2,3-*b*]-, -[3.2-*b*]-, -[2.3-*c*]- and -[3,2-*c*]pyridine. *J. Heterocyclic. Chem.,* **1997**, 34, 901-907.
35. Minakata, S.; Komatsu, M.; Ohshiro, Y. Regioselective functionalization of 1*H*-pyrrolo[2,3-*b*]pyridine via its N-oxide. *Synthesis,* **1992,** 661-663.
36. Klemm, L. H.; Hartling, R. Chemistry of thienopyridines. XXIV. Two transformations of thieno[2,3-*b*]pyridine 7-oxide (1). *J. Heterocyclic Chem.,* **1976,** *13*, 1197-1200.
37. Shiotani, S.; Taniguchi, K. Furopyridunes. **XXIII** [1]. Synthesis and Reactions of Chloropyridine Derivatives of Furo[2,3-*b*]-, -[2,3-*c*]- and -[3,2-*c*]pyridine. *J*. *Heterocyclic Chem.* **1997**, *34*, 925-929.
38. Hayashida, M.; Honda, H.; Hamana, M. Deoxygenative 2-Alkoxylation of Quinoline 1-Oxide. *Heterocycles* **1990,** *31*, 1325-1331.
39. Miura, Y.; Takaku, S.; Fujimura, Y.; Hamana, M. Synthesis of 2,3-Fused quinolines from 3-Substituted Quinoline 1-Oxide. Part 1. *Heterocycles* **1992,** *34*, 1055-1063.
40. Solekhova, M.A.; Kurbatov, Yu. V. A New Reaction of Reductive Amination of Quinoline N-Oxide with 2-Aminopyridine. *Zh. Org. Khim.* **1996,** *32*, 956.
41. (a) Regnouf De Vains, J.B.; Papet, A.L.; Marsura, A. New symmetric and unsymmetric polyfunctionalized 2,2'-bipyridines. *J. Het. Chem.,* **1994,** *31,* 1069-1077. (b) Miura, Y.; Yoshida, M.; Hamana, M. Synthesis of 2,3-fused quinolines from 3-substituted quinoline 1-oxides. Part II, *Heterocycles,* **1993,** *36*, 1005-1016. (c) Profft, V.E.; Rolle, W. Uber 4-merkaptoverbindungendes 2-methylpyridins. *J. Prakt. Chem.,* **1960,** 283 (11), 22-34.
42. Nesi, R.; Giomi, D.; Turchi, S.; Tedeschi, P., Ponticelli, F. A new one step synthetic approach to the isoxazolo[4,5-*b*]pyridine system. *Synth. Comm.,* **1992,** *22*, 2349-2355.
43. (a) Walser, A.; Zenchoff, G.; Fryer, R.I. Quinazolines and 1,4-benzodiazepines. 75. 7-Hydroxyaminobenzodiazepines and derivatives. *J. Med. Chem*., **1976,** *19,* 1378-1381. (b) Barker, G.; Ellis, G.P. Benzopyrone. Part I. 6-Amino- and 6-hydroxy-2-subtituted chromones. *J. Chem*. *Soc.,* **1970,** 2230-2233.
44. Ayyangar, N.R.; Lahoti, R J.; Daniel, T. An alternate synthesis of 3,4-diaminobenzophenone and mebendazole. *Org. Prep. Proced. Int*., **1991,** *23*, 627-631.
45. Mahadevan, 1.; Rasmussen, M. Ambident heterocyclic reactivity: The alkylation of pyrrolopyridines (azaindoles, diazaindenes). *Tetrahedron,* **1993**, *49*, 7337-7352.
46. (a) Sakamoto, T.; Ohsawa, K. Palladium-catalyzed cyanation of aryl and heteroaryl iodides with copper(I) cyanide. *J. Chem. Soc, Perkin Trans 1* **1999,** 2323-2326. (b) Halley, F.; Sava, X. Synthesis of 5-cyanoindazole and 1-methyl and 1-aryl-5-cyanoindazoles. *Synth. Commun.* **1997,** *27*, 1199-1207. (c) Yamaguchi, S.; Yoshida, M.; Miyajima, I.; Araki, T.; Hirai, Y. The Synthesis of Benzofuroquinolines. **X.** Some Benzofuro[3,2-*c*]isoquinoline Derivatives. *J*. *Heterocyclic Chem.* **1995,** *32*,1517-1519. (d) Funhoff, D. J. H.; Staab, H. A. Cyclo[d.e.d.e.e.d.e.d.e.e.]decaakisbenzene, a New Cycloarene. *Angew Chem., Int. Ed. Engl.* **1986,** *25*, 742.
47. Klimesova, V.; Otcenasek, M.; Waisser, K. Potential antifungal agents. Synthesis and activity of 2-alkylthiopyridine-4-carbothioamides. *Eur. J. Med. Chem.* **1996,** *31*, 389-395.
48. Katritzky, A.; Rachwal, S.; Smith, T. P.; Steel, P. J. Synthesis and Reactivity of 2,6-Diamino-4-methyl-3-pyridinecarbonitrile. *J. Heterocyclic Chem.* **1995,** *32*, 979-984.
49. (a) Miletin, M.; Hartl, J.; Machacek, M. Synthesis of Some Anides of 2-Alkyl-4-pyridinecarboxylic Acids and Their Photosynthsis-Inhibiting Activity. *Collect. Czech. Chem. Commun.* **1997,** *62*, 672-678. (b) Shiotani, S.; Taniguchi, K. Furopyridines. **XVII** [1]. Cyanation, Chlorination and Nitration of Furo[3,2-b]pyridine *N*-Oxide. *J. Heterocyclic Chem.* **1996,** *33*, 1051-1056. (c) El Hadri, A.; Leclerc, G. A Convenient Synthesis of cis-4-(Sulfomethyl)-piperidine-2-carboxylic Acid: NMR Assignment. *J. Heterocyclic Chem.* **1993,** *30*, 631-635.
50. (a) Heirtzler, F. R. Preparation of Non-Symmetrical 2,3-Bis-(2,2'-oligopyridyl)pyrazines via 1,2-Disubstituted Ethanones. *Synlett.* **1999,** 1203-1206. (b) Norrby, T.; Roerje, A.; Zhang, L.; Aakermark, B. Regioselective Functionalization of 2,2'-Bipyridine and Transformations into Unsymmetric Ligands for Coordination Chemistry. *Acta Chem. Scand.* **1998,** *52*, 77-85.
51. (a) Sitsun'van; Borisova, E. Ya.; Golovkov, P. V.; Burdelev, O. T.; Guzeneva, N. A.; Cherkashin, M. I.; Tolstikov, G. A. *Zh Org Khim* **1995,** 31, 1169-1172. (b) Reich, S. H.; Melnick, M.; Pino, M. J.; Fuhry, M. A. M.; Trippe, A. J.; Appelt, K.; Davies, J. F. II; Wu, B.-W.; Musick, L. Structure-Based Design and Synthesis of Substituted 2-Butanols as Nonpeptidic Inhibitors of HIV Protease: Secondary Amide Series. J. *Med. Chem.* **1996,** 39, 2781-2794. (c) Salfetnikova, Yu. N.; Vasil'ev, A. V.; Rudenko, A. P. *Zh. Org. Khim.* **1998,** *34*, 888-894.
52. (a) Oki, A. R.; Morgan, R. J. An Efficient Preparation of 4,4'-Dicarboxy-2,2'-bipyridine. *Synth. Commun.* **1995,** 25, 4093-4097. (b) Garelli, N.; Vierling, P. Synthesis of New Amphiphilic Perfluoroalkylated Bipyridines. *J. Org. Chem.* **1992,** *57*, 3046-3051. (c) Koyama, J.; Ogura, T.; Tagahara, K. Diels-Alder Reaction of 1,2,3-Triazine with Aldehyde Enamine. *Heterocycles* **1994***, 38,* 1595-1600.
53. (a) Yasuda, M.; Boger, D. L. Streptonigrin and Lavendacymin Partial Structures. Preparation of 7-Amino-2-(2'-pyridyl)quinoline-5,8-quinone-6'-carboxylic Acid: A Probe for the Minium, Potent Pharmacophore of the Naturally Occurring Antitumor-Antibiotics. *J*. *Heterocyclic Chem.* **1987**, *24*, 1253-1260. (b) Levine, R.; Sneed, J. K. The Relative Reactivities of the Isomeric Methyl Pyridinecarboxylate in the Acylation of Certain Ketones. The Synthesis of β-Diketones Containing Pyridine Rings. *J*. *Am. Chem. Soc.* **1951**, *73*, 5614 -5616. (c) Hoemann, M. Z.; Melikian-Badalian, A.; Kumarave, G.; Hauske, J. R. Solid-Phase Synthesis of Substituted Quinoline and Isoquinoline Derivatives Using Heterocyclic N-oxide Chemistry. *Tetrahedron Lett.* **1998,** *39*, 4749-4752.
54. (a) Norman, M. H.; Navas, F. III; Thompson, J. B.; Rigdon, G. C. Synthesis and Evaluation of Heterocyclic Carboxamides as Potential Antipsychotic Agents. *J*. *Med. Chem.* **1996,** 39, 4692-4703. (b) Jursic, B. S.; Zdravkovski, Z. A Simple Preparation of Amides from Acids and Amines by Heating of Their Mixture. *Synth. Commun.* **1993,** 23, 2761-2770. (c) Strekowski, L.; Gulevich, Y.; Baranowski, T.C.; Parker, A. N.; Kiselyov, A. S.; Lin, S.-Y.; Tanious, F. A.; Wilson, W. D. Synthesis and Structure-DNA Binding Relationship Analysis of DNA Triple-Helix Specific Intercalators. *J*. *Med. Chem.* **1996,** *39*, 3980-3983. (d) Shi, G.; Takagishi, S.; Schlosser, M. Metalated Fluoropyridines and Fluoroquinolines as Reactive Intermediates: New Ways for Their Regioselective Generation. *Tetrahedron* **1994,** *50*, 1129-1134.
55. Chen, B.K.; Saksela, K.; Andino, R.; Baltimore, D. Distinct modes of human immunodeficiency type 1 proviral latency revealed by superinfection of nonproductively infected cell lines with recombinant luciferase-encoding viruses. *J*. *Virol,* **1994**, *68*, 654-660.
56. Clark, G. J.; Deady, L.W. "Synthetic Uses of the Sequential Ring Positional Reactivity in Pyridin-3-ol and Derivatives" *Aust. J. Chem.* **1981**, *34*, 927-932.
57. Anderson, H. J.; Loader, C. E.; Foster, A. "Pyrrole chemistry. XXII. A "one-pot" synthesis of some 4-acylpyrrole-2-carboaldehydes from pyrrole" *Can. J. Chem.* **1980**, *58*, 2527-2530.
58. Suzuki, H.; Iwata, C.; Sakurai, K.; Tokumoto, K.; Takahashi, H.; Hanada, M.; Yokoyama, Y.; Murakami, Y. " A General Synthetic Route for 1-Substituted 4-Oxygenated β-Carbolines (Synthetic Studies on Indoles and Related Compounds 41)" *Tetrahedron,* **1997,** *53(5)*, 1593-1606.
59. Marfat, A. ; and Robinson, R. P. ; "Azaoxindole Derivatives" US. *Patent* 5,811,432 1998.

### SUMMARY OF THE INVENTION

The present invention comprises compounds of Formula I, or pharmaceutically acceptable salts thereof, which are effective antiviral agents, particularly as inhibitors of HIV. 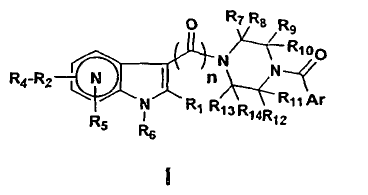 wherein: 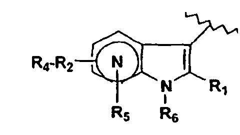 is selected from the group consisting of 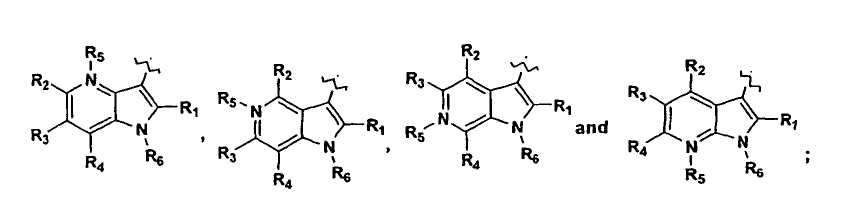 R₁, R₂, R₃, R₄ are each independently selected from the group consisting of H, C₁-C₆ alkyl, C₃-C₆ cycloalkyl, C₂-C₆ alkenyl, C₄-C₆ cycloalkenyl, C₂-C₆ alkynyl, halogen, CN, phenyl, nitro, OC(O)R₁₅, C(O)R₁₅, C(O)OR₁₆, C(O)NR₁₇R₁₈, OR₁₉, SR₂₀ and NR₂₁R₂₂;
R₁₅, is independently selected from the group consisting of H, C₁-C₆ alkyl, C₃-C₆ cycloalkyl, C₂-C₆ alkenyl and C₄-C₆ cycloalkenyl;
R₁₆, R₁₉, and R₂₀ are each independently selected from the group consisting of H, C₁-C₆ alkyl, C₁₋₆ alkyl substituted with one to three halogen atoms, C₃-C₆ cycloalkyl, C₂-C₆ alkenyl, C₄-C₆ cycloalkenyl, and C₃-C₆ alkynyl; provided the carbon atoms which comprise the carbon-carbon triple bond of said C₃-C₆ alkynyl are not the point of attachment to the oxygen or sulfur to which R₁₆, R₁₉, or R₂₀ is attached;
R₁₇ and R₁₈ are each independently selected from the group consisting of H, C₁-C₆ alkyl, C₃-C₆ cycloalkyl, C₃-C₆ alkenyl, C₄-C₆ cycloalkenyl and C₃-C₆ alkynyl; provided the carbon atoms which comprise the carbon-carbon double bond of said C₃-C₆ alkenyl or the carbon-carbon triple bond of said C₃-C₆ alkynyl are not the point of attachment to the nitrogen to which R₁₇ and R₁₈ is attached;
R₂₁ and R₂₂ are each independently selected from the group consisting of H, OH, C₁-C₆ alkyl, C₃-C₆ cycloalkyl, C₃-C₆ alkenyl, C₅-C₆ cycloalkenyl, C₃-C₆ alkynyl and C(O)R₂₃; provided the carbon atoms which comprise the carbon-carbon double bond of said C₃-C₆ alkenyl, C₄-C₆ cycloalkenyl, or the carbon-carbon triple bond of said C₃-C₆ alkynyl are not the point of attachment to the nitrogen to which R₂₁ and R₂₂ is attached;
R₂₃ is selected from the group consisting of H, C₁-C₆ alkyl, C₃-C₆ cycloalkyl, C₂-C₆ alkenyl, C₄-C₆ cycloalkenyl, and C₂-C₆ alkynyl;
R₅ is (O)ₘ, wherein m is 0 or 1;
n is 1 or 2;
R₆ is selected from the group consisting of H, C₁-C₆ alkyl, C₃-C₆ cycloalkyl, C₄-C₆ cycloalkenyl, C(O)R₂₄, C(O)OR₂₅, C(O)NR₂₆R₂₇, C₃-C₆ alkenyl and C₃-C₆ alkynyl; provided the carbon atoms which comprise the carbon-carbon double bond of said C₃-C₆ alkenyl or the carbon-carbon triple bond of said C₃-C₆ alkynyl are not the point of attachment to the nitrogen to which R₆ is attached;
R₂₄ is selected from the group consisting of H, C₁-C₆ alkyl, C₃-C₆ cycloalkyl, C₃-C₆ alkenyl, C₄-C₆ cycloalkenyl, and C₃-C₆ alkynyl;
R₂₅ is selected from the group consisting of C₁-C₆ alkyl, C₃-C₆ cycloalkyl, C₂-C₆ alkenyl, C₄-C₆ cycloalkenyl, and C₃-C₆ alkynyl; provided the carbon atoms which comprise the carbon-carbon triple bond of said C₃-C₆alkynyl are not the point of attachment to the oxygen to which R₂₅ is attached;
R₂₆ and R₂₇ are each independently selected from the group consisting of H, C₁-C₆ alkyl, C₃-C₆ cycloalkyl, C₃-C₆ alkenyl, C₅-C₆ cycloalkenyl, and C₃-C₆ alkynyl; provided the carbon atoms which comprise the carbon-carbon double bond of said C₃-C₆ alkenyl, C₅-C₆ cycloalkenyl, or the carbon-carbon triple bond of said C₃-C₆ alkynyl are not the point of attachment to the nitrogen to which R₂₆ and R₂₇ are attached;
R₇, R₈, R₉, R₁₀, R₁₁, R₁₂, R₁₃, and R₁₄ are each independently selected from the group consisting of H, C₁-C₆, alkyl, C₃-C₆ cycloalkyl, C₂-C₆ alkenyl, C₄-C₆ cycloalkenyl, C₂-C₆ alkynyl, CR₂₈R₂₉OR₃₀, C(O)R₃₁, CR₃₂(OR₃₃)OR₃₄, CR₃₅NR₃₆R₃₇, C(O)OR₃₈, C(O)NR₃₉R₄₀, CR₄₁R₄₂F, CR₄₃F₂ and CF₃;
R₂₈, R₂₉, R₃₀, R₃₁, R₃₂, R₃₅, R₄₁, R₄₂ and R₄₃ are each independently selected from the group consisting of H, C₁-C₆ alkyl, C₃-C₆ cycloalkyl, C₂-C₆ alkenyl, C₄-C₆ cycloalkenyl, C₂-C₆ alkynyl and C(O)R₄₄;
R₃₃, R₃₄ and R₃₈ are each independently selected from the group consisting of H, C₁-C₆ alkyl, C₃-C₆ cycloalkyl, C₃-C₆ alkenyl, C₁-C₆ cycloalkenyl, and C₃-C₆ alkynyl; provided the carbon atoms which comprise the carbon-carbon triple bond of said C₃-C₆ alkynyl are not the point of attachment to the oxygen to which R₃₄ and R₃₈ are attached;
R₃₆ and R₃₇ are each independently selected from the group consisting of H, C₁-C₆ alkyl, C₃-C₆ cycloalkyl, C₃-C₆ alkenyl, C₄-C₆ cycloalkenyl, and C₃-C₆ alkynyl; provided the carbon atoms which comprise the carbon-carbon triple bond of said C₃-C₆ alkynyl are not the point of attachment to the nitrogen to which R₃₆ and R₃₇ are attached;
R₃₉ and R₄₀ are each independently selected from the group consisting of H, C₁-C₆ alkyl, C₃-C₆ cycloalkyl, C₂-C₆ alkenyl, C₄-C₆ cycloalkenyl, and C₃-C₆ alkynyl; provided the carbon atoms which comprise the carbon-carbon triple bond of said C₃-C₆ alkynyl are not the point of attachment to the nitrogen to which R₃₉ and R₄₀ are attached;
R₄₄ is selected from the group consisting of H, C₁-C₆ alkyl, C₃-C₆ cycloalkyl, C₂-C₆ alkenyl, C₄-C₆ cycloalkenyl, and C₂-C₆ alkynyl;
Ar is selected from the group consisting of 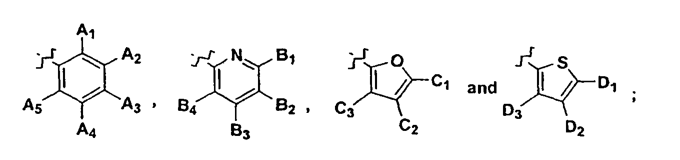 A₁, A₂, A₃, A₄, A₅, B₁, B₂, B₃, B₄, C₁, C₂, C₃, D₁, D₂, and D₃ are each independently selected from the group consisting of H, CN, halogen, NO₂, C₁-C₆ alkyl, C₃-C₆ cycloalkyl, C₂-C₆ alkenyl, C₄-C₆ cycloalkenyl, C₂-C₆ alkynyl, OR₄₅, NR₄₆R₄₇, SR₄₈, N₃ and CH(-N=N-)-CF₃;
R₄₅ is selected from the group consisting of H, C₁-C₆ alkyl, C₃-C₆ cycloalkyl, C₂-C₆ alkenyl, C₄-C₆ cycloalkenyl and C₃-C₆ alkynyl; provided the carbon atoms which comprise the carbon-carbon triple bond of said C₃-C₆ alkynyl are not the point of attachment to the oxygen to which R₄₅ is attached;
R₄₆ and R₄₇ are each independently selected from the group consisting of H, C₁-C₆ alkyl, C₃-C₆ cycloalkyl, C₃-C₆ alkenyl, C₅-C₆ cycloalkenyl, C₃-C₆ alkynyl and C(O)R₅₀; provided the carbon atoms which comprise the carbon-carbon double bond of said C₅-C₆ alkenyl, C₄-C₆ cycloalkenyl, or the carbon-carbon triple bond of said C₃-C₆ alkynyl are not the point of attachment to the nitrogen to which R₄₆ and R₄₇ are attached;
R₄₈ is selected from the group consisting of H, C₁-C₆ alkyl, C₃-C₆ cycloalkyl, C₂₋C₆ alkenyl, C₄-C₆ cycloalkenyl, C₃-C₆ alkynyl and C(O)R₄₉; provided the carbon atoms which comprise the carbon-carbon triple bond of said C₃-C₆ alkynyl are not the point of attachment to the sulfur to which R₄₈ is attached;
R₄₉ is C₁-C₆ alkyl or C₃-C₆ cycloalkyl; and
R₅₀ is selected from the group consisting of H, C₁-C₆ alkyl, and C₃-C₆ cycloalkyl.

Preferred are compounds of Formula I or pharmaceutically acceptable salts thereof wherein R₂-R₄ is independently H, -OCH₃, -OCH₂CF₃, -OiPr, -OnPr, halogen, CN, NO₂, C₁-C₆ alkyl, NHOH, NH₂, Ph, SR₂₀, or N(CH₃)₂.

Also preferred are compounds of Formula I wherein one or two of R₇-R₁₄ is independently methyl and the other substituents are hydrogen.

Also preferred are compounds of Formula I wherein one of A₁-A₅, B₁-B₄, C₁-C₃ or D₁-D₃ are either hydrogen, halogen, or amino and the remaining substituents are hydrogen.

Also preferred are compounds of the formula below: 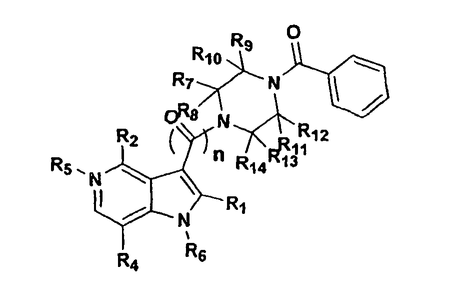 wherein:
R₂ is H, F, Cl, Br, OMe, CN, or OH;
R₄ is C₁-C₆ alkyl, C₂ C₆ alkenyl, C₃-C₆ cycloalkyl, C₅-C₆ cycloalkenyl, Cl, OMe, CN, OH, C(O)NH₂, C(O)NHMe, C(O)NHEt, Ph or -C(O)CH₃;
n is 2;
R₈, R₉, R₁₀, R₁₁, R₁₂, R₁₃ and R₁₄ are each independently H or CH3, provided up to two of these substituents may be methyl;
R₁ is hydrogen;
R₅ is unsubstituted; and
R₆ is hydrogen or methyl.

A most preferred aspect of the invention are compounds or pharmaceutically acceptable salts thereof of the Formula 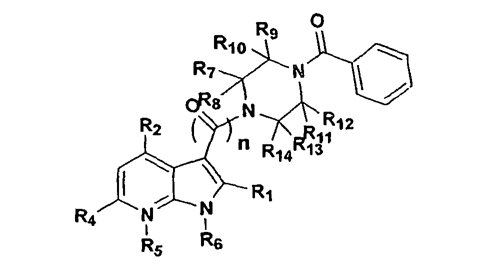 wherein:
R₂ is H, -OCH₃, -OCH₂CF₃, -OPr, halogen, CN, NO₂, or NHOH;
R₄ is H, -halogen, -CN, or hydroxy;
One or two members of R₇-R₁₄ is methyl and the remaining members are hydrogen;
n is 2;
R₁ is hydrogen;
R₅ is (O)ₘ, where m is O; and
R₆ is hydrogen, methyl, or allyl.

Another most preferred aspect of the invention are compounds of the formula below wherein: 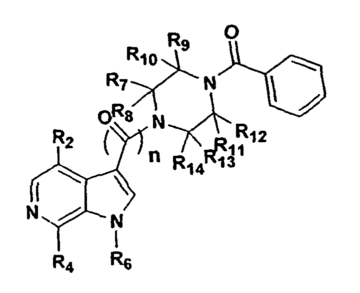 wherein:
R₂ is selected from the group consisting of H, F, Cl, Br, OMe, CN, and OH;
R₄ is selected from the group consisting of H, C₁-C₆ alkyl, C₂-C₆ alkenyl, C₃-C₆ cycloalkyl, C₅-C₆ cycloalkenyl, Cl, OMe, CN, OH, C(O)NH₂, C(O)NHMe, C(O)NHEt, phenyl and -C(O)CH_{3;}
n is 2;
R₈, R₉, R₁₀, R₁₁, R₁₂, R₁₃, and R₁₄ are each independently H or CH₃,
   provided 0-2 of the members of the group R_{8,} R₉, R₁₀, R₁₁, R₁₂, R₁₃, and R₁₄ may be CH₃ and the remaining members of the group R₈, R₉, R₁₀, R₁₁, R₁₂, R₁₃, and R₁₄ are H; and
R₆ is H or CH₃.

Another most preferred aspect of the inventions are compounds of formula: 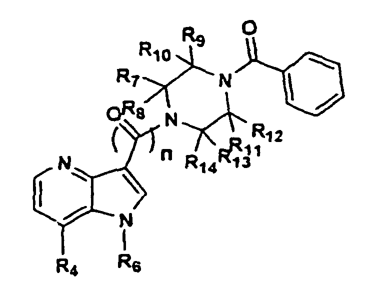 wherein:
R₄ is selected from the group consisting of H, C₁-C₆ alkyl, C₂-C₆ alkenyl, C₃-C₆ cycloalkyl, C₅-C₆ cycloalkenyl, Cl, OMe, CN, OH, C(O)NH₂, C(O)NHMe, C(O)NHEt, phenyl and -C(O)CH₃;
n is 2;
R₈, R₉, R₁₀, R₁₁, R₁₂, R₁₃, and R₁₄, are each independently H or CH₃, provided 0-2 of the members of the group R₈, R₉, R₁₀, R₁₁, R₁₂, R₁₃, and R₁₄ may be CH₃ and the remaining members of the group R₈, R₉, R₁₀, R₁₁, R₁₂, R₁₃, and R₁₄ are H; and
R₆ is H or CH₃

Since the compounds of the present invention, may possess asymmetric centers and therefore occur as mixtures of diastereomers and enantiomers, the present invention includes the individual diastereoisomeric and enantiomeric forms of the compounds of Formula I.

Another embodiment of the invention is a pharmaceutical composition which comprises an antiviral effective amount of a compound of Formula I.

Another embodiment of the present invention is the use of a compound of Formula I for manufacturing a medicament for treating mammals infected with a virus such as HIV by administration to said mammal of an antiviral effective amount of a compound of Formula I.

Another embodiment of the present invention is the use of a compound of Formula I for manufacturing a medicament for treating mammals infected with a virus such as HIV by administration to said mammal of an antiviral effective amount of a compound of Formula I in combination with an antiviral effective amount of an AIDS treatment agent selected from the group consisting of: (a) an AIDS antiviral agent; (b) an anti-infective agent; (c) an immunomodulator; and (d) HIV entry inhibitors.

### DETAILED DESCRIPTION OF THE INVENTION

The preparative procedures and anti-HIV-1 activity of the novel azaindole piperazine diamide analogs of Formula I are summarized below. The definition of various terms follow.

The term "C₁₋₆ alkyl" as used herein and in the claims (unless the context indicates otherwise) means straight or branched chain alkyl groups such as methyl, ethyl, propyl, isopropyl, butyl, isobutyl, t-butyl, amyl, hexyl and the like. Similarly, "C₁₋₆ alkenyl" or "C₁₋₆ alkynyl" includes straight or branched chain groups.

"Halogen" refers to chlorine, bromine, iodine or fluorine.

Physiologically acceptable salts and prodrugs of compounds disclosed herein are within the scope of this invention. The term "pharmaceutically acceptable salt" as used herein and in the claims is intended to include nontoxic base addition salts. Suitable salts include those derived from organic and inorganic acids such as, without limitation, hydrochloric acid, hydrobromic acid, phosphoric acid, sulfuric acid, methanesulfonic acid, acetic acid, tartaric acid, lactic acid, sulfinic acid, citric acid, maleic acid, fumaric acid, sorbic acid, aconitic acid, salicylic acid, phthalic acid, and the like. The term "pharmaceutically acceptable salt" as used herein is also intended to include salts of acidic groups, such as a carboxylate, with such counterions as ammonium, alkali metal salts, particularly sodium or potassium, alkaline earth metal salts, particularly calcium or magnesium, and salts with suitable organic bases such as lower alkylamines (methylamine, ethylamine, cyclohexylamine, and the like) or with substituted lower alkylamines (e.g. hydroxyl-substituted alkylamines such as diethanolamine, triethanolamine or tris(hydroxymethyl)- aminomethane), or with bases such as piperidine or morpholine.

In the use of the present invention, the term "antiviral effective amount" means the total amount of each active component that is sufficient to show a meaningful patient benefit, i.e., healing of acute conditions characterized by inhibition of the HIV infection. When applied to an individual active ingredient, administered alone, the term refers to that ingredient alone. When applied to a combination, the term refers to combined amounts of the active ingredients that result in the therapeutic effect, whether administered in combination, serially or simultaneously. The terms "treat, treating, treatment" as used herein and in the claims means preventing or ameliorating diseases associated with HIV infection.

The present invention is also directed to combinations of the compounds with one or more agents useful in the treatment of AIDS. For example, the compounds of this invention may be effectively administered, whether at periods of pre-exposure and/or post-exposure, in combination with effective amounts of the AIDS antivirals, immunomodulators, antiinfectives, or vaccines, such as those in the following table.

| ANTIVIRALS | | |
|---|---|---|
| Drug Name | Manufacturer | Indication |
| 097 | Hoechst/Bayer | HIV infection, AIDS, ARC (non-nucleoside reverse transcriptase (RT) inhibitor) |
| Amprenivir 141 W94 GW 141 | Glaxo Wellcome | HIV infection, AIDS, ARC (protease inhibitor) |
| Abacavir (1592U89) GW 1592 | Glaxo Wellcome | HIV infection, AIDS, ARC (RT inhibitor) |
| Acemannan | Carrington Labs (Irving, TX) | ARC |
| Acyclovir | Burroughs Wellcome | HIV infection, AIDS, ARC, in combination with AZT |
| AD-439 | Tanox Biosystems | HIV infection, AIDS, ARC |
| AD-519 | Tanox Biosystems | HIV infection, AIDS, ARC |
| Adefovir dipivoxil | Gilead Sciences | HIV infection |
| AL-721 | Ethigen (Los Angeles, CA) | ARC, PGL HIV positive, AIDS |
| Alpha Interferon | Glaxo Wellcome | Kaposi's sarcoma, HIV in combination w/Retrovir |
| Ansamycin LM 427 | Adria Laboratories (Dublin, OH) Erbamont (Stamford, CT) | ARC |
| Antibody which Neutralizes pH Labile alpha aberrant Interferon | Advanced Biotherapy Concepts (Rockville, MD) | AIDS, ARC |
| AR177 | Aronex Pharm | HIV infection, AIDS, ARC |
| Beta-fluoro-ddA | Nat'l Cancer Institute | AIDS-associated diseases |
| BMS-232623 (CGP-73547) | Bristof-Myers Squibb/ Novartis | HIV infection, AIDS, ARC (protease inhibitor) |
| BMS-234475 (CGP-61755) | Bristol-Myers Squibb/ Novartis | HIV infection, AIDS, ARC (protease inhibitor) |
| Cl-1012 | Warner-Lambert | HIV-1 infection |
| Cidofovir | Gilead Science | CMV retinitis, herpes, papillomavirus |
| Curdlan sulfate | AJI Pharma USA | HIV infection |
| Cytomegalovirus Immune globin | Medimmune | CMV retinitis |
| Cytovene Ganciclovir | Syntex | Sight threatening CMV peripheral CMV retinitis |
| Delaviridine | Pharmacia-Upjohn | HIV infection, AIDS, ARC (RT inhibitor) |
| Dextran Sulfate | Ueno Fine Chem. Ind. Ltd. (Osaka, Japan) | AIDS, ARC, HIV positive asymptomatic |
| ddC Dideoxycytidine | Hoffman-La Roche | HIV infection, AIDS, ARC |
| ddl Dideoxyinosine | Bristol-Myers Squibb | HIV infection, AIDS, ARC; combination with AZT/d4T |
| DMP-450 | AVID (Camden, NJ) | HIV infection, AIDS, ARC (protease inhibitor) |
| Efavirenz (DMP 266) (-)6-Chloro-4-(S)-cyclopropylethynyl-4(S)-trifluoromethyl-1,4-dihydro-2H-3,1-benzoxazin-2-one, STOCRINE | DuPont Merck | HIV infection, AIDS, ARC (non-nucleoside RT inhibitor) |
| EL10 | Elan Corp, PLC (Gainesville, GA) | HIV infection |
| Famciclovir | Smith Kline | herpes zoster, herpes simplex |
| FTC | Emory University | HIV infection, AIDS, ARC (reverse transcriptase inhibitor) |
| GS 840 | Gilead | HIV infection, AIDS, ARC (reverse transcriptase inhibitor) |
| HBY097 | Hoechst Marion Roussel | HIV infection, AIDS, ARC (non-nucleoside reverse transcriptase inhibitor) |
| Hypericin | VIMRx Pharm. | HIV infection, AIDS, ARC |
| Recombinant Human Interferon Beta | Triton Biosciences (Almeda, CA) | AIDS, Kaposi's sarcoma, ARC |
| Interferon alfa-n3 | Interferon Sciences | ARC, AIDS |
| Indinavir | Merck | HIV infection, AIDS, ARC, asymptomatic HIV positive, also in combination with AZT/ddI/ddC |
| ISIS 2922 | ISIS Pharmaceuticals | CMV retinitis |
| KNI-272 | Nat'l Cancer Institute | HIV-assoc. diseases |
| Lamivudine, 3TC | Glaxo Wellcome | HIV infection, AIDS, ARC (reverse transcriptase inhibitor); also with AZT |
| Lobucavir | Bristol-Myers Squibb | CMV infection |
| Nelfinavir | Agouron Pharmaceuticals | HIV infection, AIDS, ARC (protease inhibitor) |
| Nevirapine | Boeheringer Ingleheim | HIV infection, AIDS, ARC (RT inhibitor) |
| Novapren | Novaferon Labs, Inc. (Akron, OH) | HIV inhibitor |
| Peptide T Octapeptide Sequence | Peninsula Labs (Belmont, CA) | AIDS |
| Trisodium Phosphonoformate | Astra Pharm. Products, Inc. | CMV retinitis, HIV infection, other CMV infections |
| PNU-140690 | Pharmacia Upjohn | HIV infection, AIDS, ARC (protease inhibitor) |
| Probucol | Vyrex | HIV infection, AIDS |
| RBC-CD4 | Sheffield Med. Tech (Houston, TX) | HIV infection, AIDS, ARC |
| Ritonavir | Abbott | HIV infection, AIDS, ARC (protease inhibitor) |
| Saquinavir | Hoffmann-LaRoche | HIV infection, AIDS, ARC (protease inhibitor) |
| Stavudine; d4T Didehydrodeoxy-thymidine | Bristol-Myers Squibb | HIV infection, AIDS, ARC |
| Valaciclovir | Glaxo Wellcome | Genital HSV & CMV infections |
| Virazole Ribavirin | Viratek/ICN (Costa Mesa, CA) | asymptomatic HIV positive, LAS, ARC |
| VX-478 | Vertex | HIV infection, AIDS, ARC |
| Zalcitabine | Hoffmann-LaRoche | HIV infection, AIDS, ARC, with AZT |
| Zidovudine; AZT | Glaxo Wellcome | HIV infection, AIDS, ARC, Kaposi's sarcoma, in combination with other therapies |

| IMMUNOMODULATORS | | |
|---|---|---|
| Drug Name | Manufacturer | Indication |
| AS-101 | Wyeth-Ayerst | AIDS |
| Bropirimine Acemannan | Pharmacia Upjohn Carrington Labs, Inc. (Irving, TX) | Advanced AIDS AIDS, ARC |
| CL246,738 | American Cyanamid Lederle Labs | AIDS, Kaposi's sarcoma |
| EL10 | Elan Corp, PLC (Gainesville, GA) | HIV infection |
| FP-21399 | Fuki ImmunoPharm | Blocks HIV fusion with CD4+ cells |
| Gamma Interferon | Genentech | ARC, in combination w/TNF (tumor necrosis factor) |
| Granulocyte Macrophage Colony Stimulating Factor | Genetics Institute Sandoz | AIDS |
| Granulocyte Macrophage Colony Stimulating Factor | Hoechst-Roussel Immunex | AIDS |
| Granulocyte Macrophage Colony Stimulating Factor | Schering-Plough | AIDS, combination w/AZT |
| HIV Core Particle Immunostimulant | Rorer | Seropositive HIV |
| IL-2 Interleukin-2 | Cetus | AIDS, in combination w/AZT |
| IL-2 Interleukin-2 | Hoffman-LaRoche Immunex | AIDS, ARC, HIV, in combination w/AZT |
| IL-2 Interleukin-2 (aldeslukin) | Chiron | AIDS, increase in CD4 cell counts |
| Immune Globulin Intravenous (human) | Cutter Biological (Berkeley, CA) | Pediatric AIDS, in combination w/AZT |
| IMREG-1 | Imreg (New Orleans, LA) | AIDS, Kaposi's sarcoma, ARC, PGL |
| IMREG-2 | lmreg (New Orleans, LA) | AIDS, Kaposi's sarcoma, ARC, PGL |
| Imuthiol Diethyl Dithio Carbamate | Merieux Institute | AIDS, ARC |
| Alpha-2 Interferon | Schering Plough | Kaposi's sarcoma w/AZT, AIDS |
| Methionine-Enkephalin | TNI Pharmaceutical (Chicago, IL) | AIDS, ARC |
| MTP-PE Muramyl-Tripeptide | Ciba-Geigy Corp. | Kaposi's sarcoma |
| Granulocyte Colony Stimulating Factor | Amgen | AIDS, in combination w/AZT |
| Remune | Immune Response Corp. | Immunotherapeutic |
| rCD4 Recombinant Soluble Human CD4 | Genentech | AIDS, ARC |
| rCD4-lgG hybrids | | AIDS, ARC |
| Recombinant Soluble Human CD4 | Biogen | AIDS, ARC |
| Interferon Alfa 2a | Hoffman-La Roche | Kaposi's sarcoma AIDS, ARC, in combination w/AZT |
| SK&F106528 Soluble T4 | Smith Kline | HIV infection |
| Thymopentin | Immunobiology Research Institute (Annandale, NJ) | HIV infection |
| Tumor Necrosis Factor; TNF | Genentech | ARC, in combination w/gamma Interferon |

| ANTI-INFECTIVES | | |
|---|---|---|
| Drug Name | Manufacturer | Indication |
| Clindamycin with Primaquine | Pharmacia Upjohn | PCP |
| Fluconazole | Pfizer | Cryptococcal meningitis, candidiasis |
| Pastille Nystatin Pastille | Squibb Corp. | Prevention of oral candidiasis |
| Ornidyl Eflornithine | Merrell Dow | PCP |
| Pentamidine Isethionate (IM & IV) | LyphoMed (Rosemont, IL) | PCP treatment |
| Trimethoprim | | Antibacterial |
| Trimethoprim/sulfa | | Antibacterial |
| Piritrexim | Burroughs Wellcome | PCP treatment |
| Pentamidine Isethionate for Inhalation | Fisons Corporation | PCP prophylaxis |
| Spiramycin | Rhone-Poulenc diarrhea | Cryptosporidial |
| Intraconazole-R51211 | Janssen-Pharm. | Histoplasmosis; cryptococcal Meningitis |
| Trimetrexate | Warner-Lambert | PCP |
| Daunorubicin | NeXstar, Sequus | Kaposi's sarcoma |
| Recombinant Human Erythropoietin | Ortho Pharm. Corp. | Severe anemia assoc. with AZT Therapy |
| Recombinant Human Growth Hormone | Serono | AIDS-related wasting, cachexia |
| Megestrol Acetate | Bristol-Myers Squibb | Treatment of Anorexia assoc. W/AIDS |
| Testosterone | Alza, Smith Kline | AIDS-related wasting |
| Total Enteral Nutrition | Norwich Eaton Pharmaceuticals | Diarrhea and malabsorption Related to AIDS |

Additionally, the compounds of the invention herein may be used in combinations which include more than three anti HIV drugs. Combinations of four or even five HIV drugs are being investigated and the compounds of this invention would be expected to be a useful component of such combinations.

Additionally, the compounds of the invention herein may be used in combination with another class of agents for treating AIDS which are called HIV entry inhibitors. Examples of such HIV entry inhibitors are discussed in DRUGS OF THE FUTURE 1999, 24(12), pp. 1355-1362; CELL, Vol. 9, pp. 243-246, Oct. 29, 1999; and DRUG DISCOVERY TODAY, Vol. 5, No. 5, May 2000, pp. 183-194.

It will be understood that the scope of combinations of the compounds of this invention with AIDS antivirals, immunomodulators, anti-infectives, HIV entry inhibitors or vaccines is not limited to the list in the above Table, but includes in principle any combination with any pharmaceutical composition useful for the treatment of AIDS.

Preferred combinations are simultaneous or alternating treatments of with a compound of the present invention and an inhibitor of HIV protease and/or a non-nucleoside inhibitor of HIV reverse transcriptase. An optional fourth component in the combination is a nucleoside inhibitor of HIV reverse transcriptase, such as AZT, 3TC, ddC or ddl. A preferred inhibitor of HIV protease is indinavir, which is the sulfate salt of N-(2(R)-hydroxy-1-(S)-indanyl)-2(R)-phenylmethyl-4-(S)-hydroxy-5-(1-(4-(3-pyridylmethyl)-2(S)-N'-(t-butylcarboxamido)-piperazinyl))-pentaneamide ethanolate, and is synthesized according to U.S. 5,413,999. Indinavir is generally administered at a dosage of 800 mg three times a day. Other preferred protease inhibitors are nelfinavir and ritonavir. Another preferred inhibitor of HIV protease is saquinavir which is administered in a dosage of 600 or 1200 mg tid. Finally a new protease inhibitor, BMS-232632, which is currently undergoing clinical trials may become a preferred inhibitor. Preferred non-nucleoside inhibitors of HIV reverse transcriptase include efavirenz. The preparation of ddC, ddl and AZT are also described in EPO 0,484,071. These combinations may have unexpected effects on limiting the spread and degree of infection of HIV. Preferred combinations include those with the following (1) indinavir with efavirenz, and, optionally, AZT and/or 3TC and/or ddl and/or ddC; (2) indinavir, and any of AZT and/or ddl and/or ddC and/or 3TC, in particular, indinavir and AZT and 3TC; (3) stavudine and 3TC and/or zidovudine; (4) zidovudine and lamivudine and 141W94 and 1592U89; (5) zidovudine and lamivudine.

In such combinations the compound of the present invention and other active agents may be administered separately or in conjunction. In addition, the administration of one element may be prior to, concurrent to, or subsequent to the administration of other agent(s).

Parent azaindoles such as 4-azaindole, 5-azaindole, 6-azaindole, or 7-azaindole are prepared by the methods described in the literature (Mahadevan et al, Ref. 25(a)) or Hands et. al. Ref 25 (b) are available from commercial sources (7-azaindole from Aldrich Co.). This reference and similar references show some examples of substituted aza indoles. Chemist skilled in the art can recognize that the general methodology can be extended to azaindoles which have different substituents in the starting materials. Azaindoles are also prepared *via* the routes described in Scheme 1 and Scheme 2.

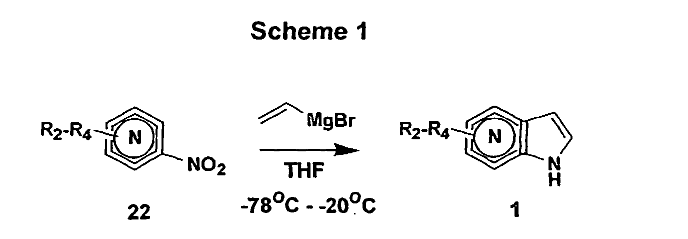

In Scheme 1, the Bartoli indole synthesis (Dobson et al, Ref. 25 (C)) is extended to prepare substituted azaindoles. Nitropyridine **22** was reacted with an excess of vinyl magnesium bromide at -78°C. After warming up to -20°C, the reaction provides the desired azaindole **1.** Generally these temperature ranges are optimal but in specific examples may be varied usually by no more than 20 °C but occasionally by more in order to optimize the yield. The vinyl magnesium bromide may be obtained commercially as a solution in tetrahydrofuran or sometimes more optimally may be prepared fresh from vinyl bromide and magnesium using literature procedures which are well known in the art. Vinyl magnesium chloride can also be used in some examples.

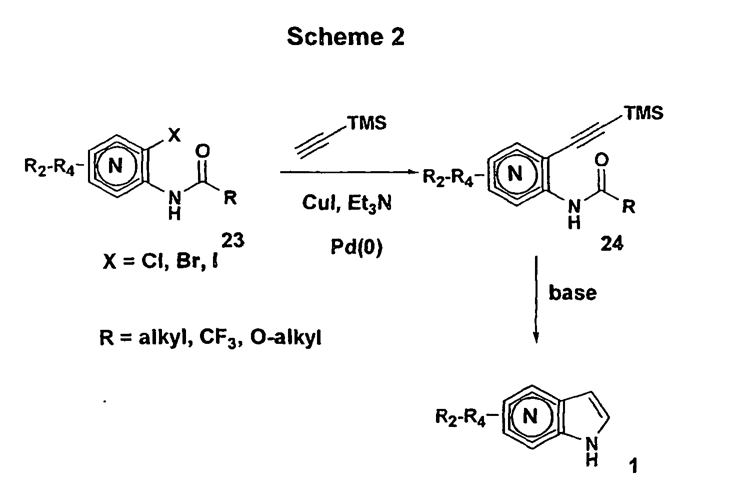

In Scheme 2, acetylene is coupled onto a halo-pyridine **23** using a Pd (0) catalyst to furnish **24.** Subsequent treatment with base effects cyclization of **24** to afford azaindole **1**(Sakamoto et al, Ref. 26). Suitable bases for the second step include sodium methoxide or other sodium, lithium, or potassium alkoxide bases.

General procedures to prepare azaindole piperazine diamide 5 of Formula I are described in Scheme 3 and Scheme 4.

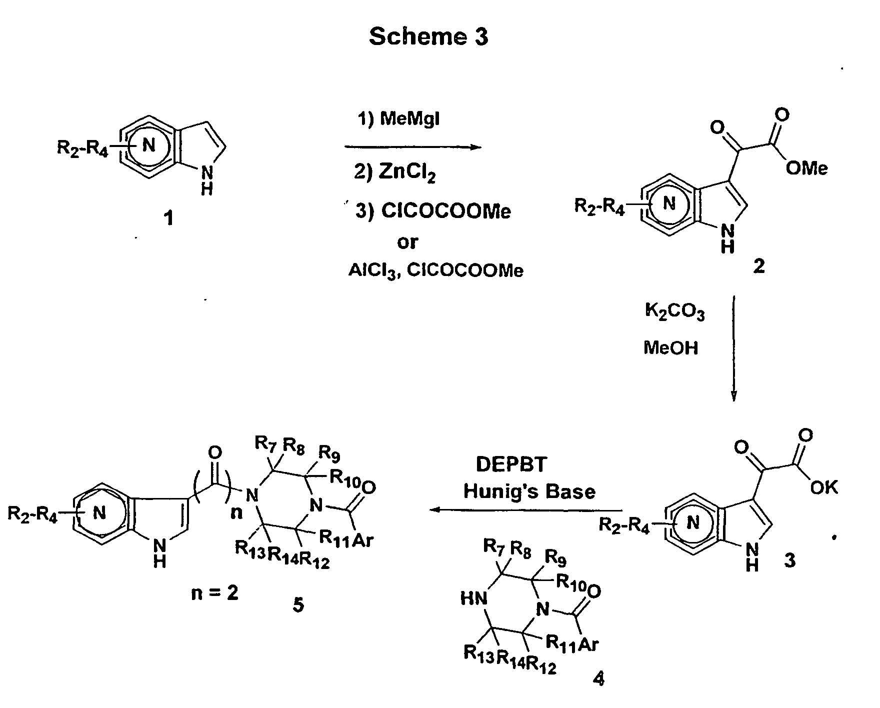

An azaindole **1,** was reacted with MeMgl (methyl magnesium iodide) and ZnCl₂ (zinc chloride), followed by the addition of CICOCOOMe (methyl chlorooxoacetate) to afford aza-indole glyoxyl methyl ester **2** (Shadrina et al, Ref. 27). Alternatively, compound **2** can be prepared by reaction of aza-indole **1** with an excess of CICOCOOMe in the presence of AlCl₃ (aluminum chloride) (Sycheva et al, Ref. 28). Hydrolysis of the methyl ester **2** affords a potassium salt 3 which is coupled with mono-benzoylated piperazine derivatives **4** in the presence of DEPBT (3-(diethoxyphosphoryloxy)-1,2,3-benzotriazin-4(3*H*)-one) and *N,N*-diisopropylethylamine, commonly known as Hunig's base, to provide azaindole piperazine diamide 5 (Li et al, Ref. 29). The mono-benzoylated piperazine derivatives **4** can be prepared according to well established procedures such as those described by Desai et al, Ref. 30(a), Adamczyk et al, Ref. 30(b), Rossen et al, Ref. 30(c), and Wang et al, 30(d) and 30(e).

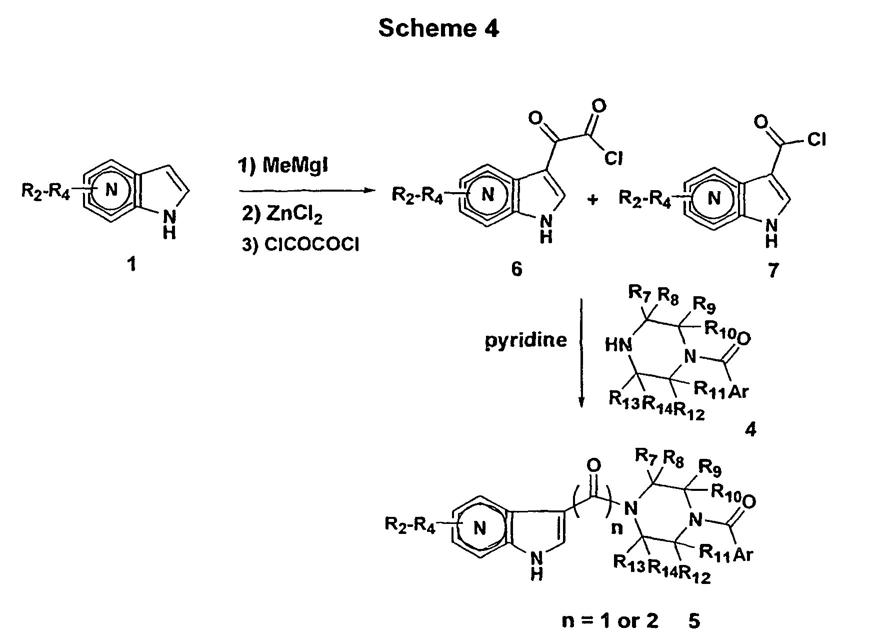

An alternative method for the preparation of **5** involves treating an azaindole **1,** obtained by procedures described in the literature or from commercial sources, with MeMgl and ZnCl₂, followed by the addition of CICOCOCI (oxalyl chloride) in either THF (tetrahydrofuran) or ether to afford a mixture of desired products, glyoxyl chloride **6** and acyl chloride **7,** Scheme 4. The resulting mixture of glyoxyl chloride **6** and acyl chloride **7** is then coupled with mono-benzoylated piperazine derivatives **4** under basic conditions to afford product **5** as a mixture of two compounds (n = 1 and 2).

General routes for further functionalizing azaindole rings are shown in Schemes 5. It should be recognized that the symbol Rx is meant to represent a general depiction of the remaining substituents from R₄-R₂ which are on the azaindole ring. As depicted in Scheme 5, the azaindole can be oxidized to the corresponding *N*-oxide derivative 8 by using mCPBA (meta-Chloroperbenzoic Acid) in acetone or DMF (Dimethylformamide) (eq. 1, Harada et al, Ref. 31 and Antonini et al, Ref. 32). The *N*-oxide **8** can be converted to a variety of substituted azaindole derivatives by using well documented reagents such as phosphorus oxychloride (POCl₃) (eq. 2, Schneller et al, Ref. 33(a)) or phosphorus tribromide (eq. 2, Wozniak et al, Ref. 33(b)), Grignard reagents RMgX (R = alkyl, X = Cl, Br or I) (eq. 4, Shiotani et al, Ref. 34), trimethylsilyl cyanide (TMSCN) (eq. 5, Minakata et al, Ref. 35), Ac₂O (eq. 6, Klemm et al, Ref. 36), thiol via a sodium thiolate or other thiolates (eq. 7, Shiotani et al, Ref. 37), alcohol via metal alkoxides as in ref 37 or (eq. 8, Hayashida et al, Ref. 38), and amine (eq. 9, using ammonia or an amine in the presence of TsCl in chloroform / water as in Miura et al, Ref. 39; or under similar conditions but with 10% aq NaOH also included as in Solekhova et al, Ref. 40). Under such conditions (respectively), a chlorine or bromine atom, nitrile group, alkyl group, hydroxyl group, thiol group, alkoxy group and amino group can be introduced to the pyridine ring. Similarly, tetramethylamonnium fluoride (Me₄NF) transforms N-oxides 8 to fluoro-azaindoles (eq. 3). Further standard modification of OH group will provide alkoxy functionality as well (eq. 6).

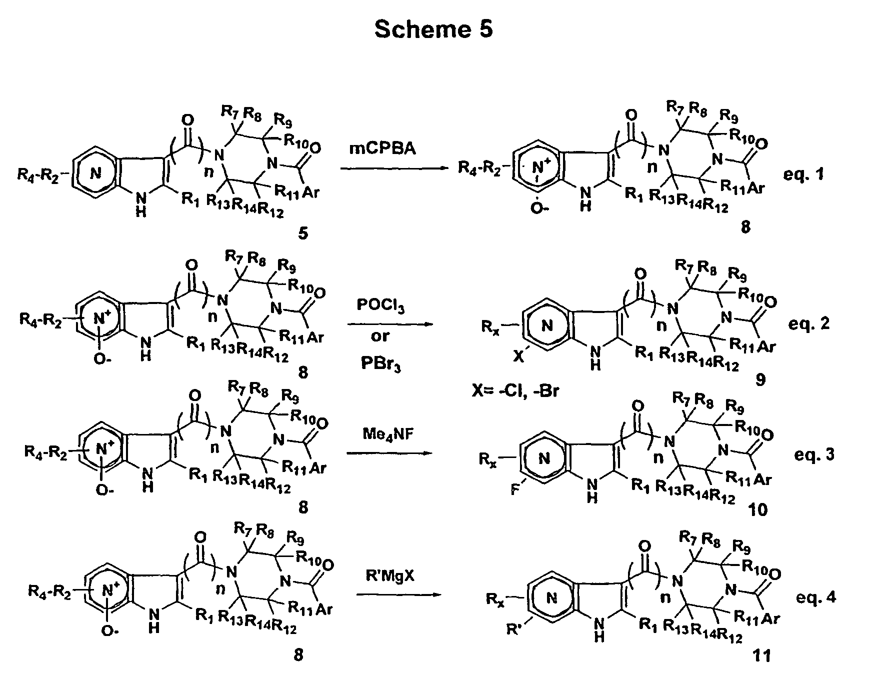

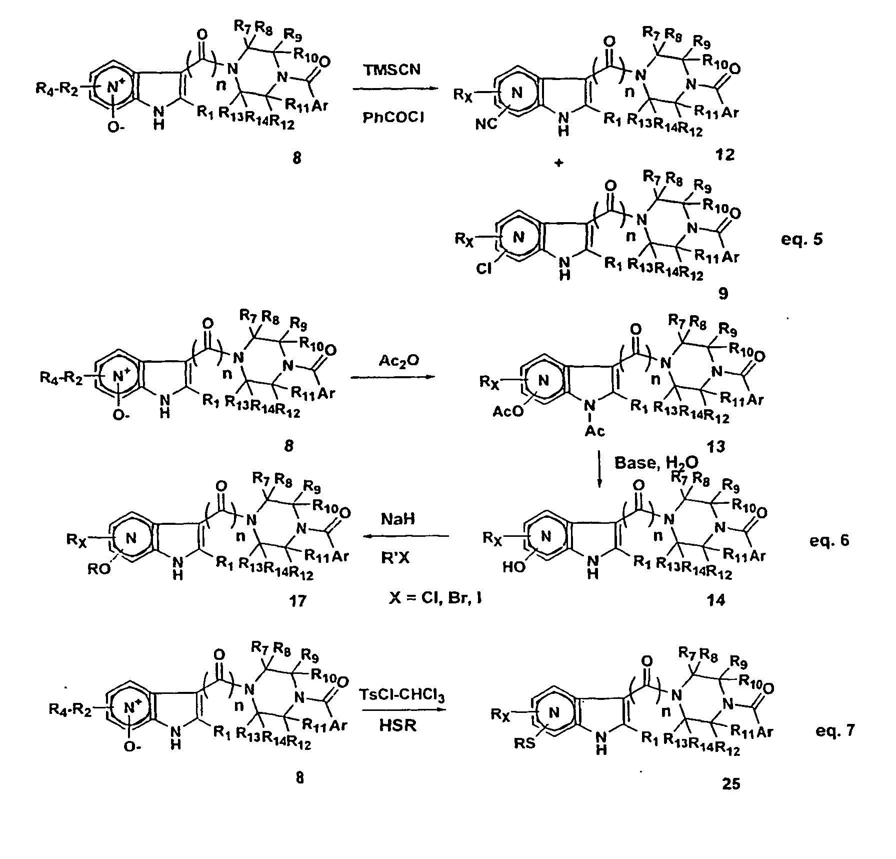

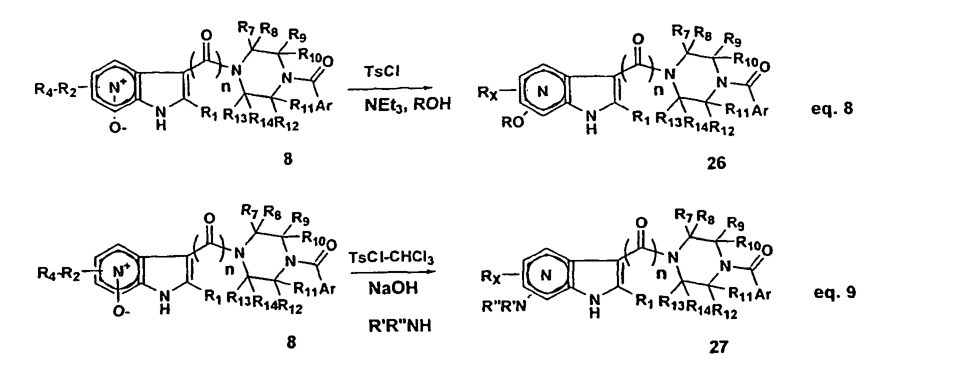

Nitration of azaindole *N*-oxides results in introduction of a nitro group to azaindole ring, as shown in Scheme 6 (eq. 10, Antonini et al, Ref. 32). The nitro group can subsequently be displaced by a variety of nucleophilic agents, such as OR, NR¹R² or SR, in a well established chemical fashion (eq. 11, Regnouf De Vains et al, Ref. 41 (a), Miura et al, Ref. 41(b), Profft et al, Ref. 41 (c)). The resulting N-oxides **16** are readily reduced to the corresponding azaindole **17** using phosphorus trichloride (PCl₃) (eq. 12, Antonini et al, Ref. 32 and Nesi et al, Ref. 42) or other reducing agents. Similarly, nitro-substituted *N*-oxide **15** can be reduced to the azaindole **18** using phosphorus trichloride (eq. 13). The nitro group of compound **18** can be reduced to either a hydroxylamine (NHOH) (eq. 14, Walser et al, Ref. 43(a) and Barker et al, Ref. 43(b)) or an amino (NH₂) group (eq. 15, Nesi et al, Ref. 42 and Ayyangar et al, Ref. 44) by carefully selecting different reducing conditions.

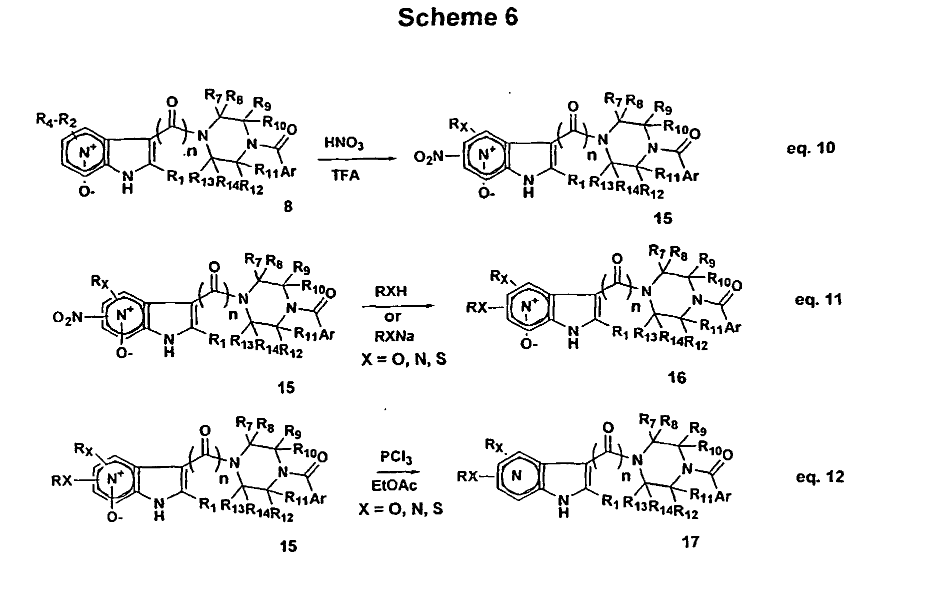

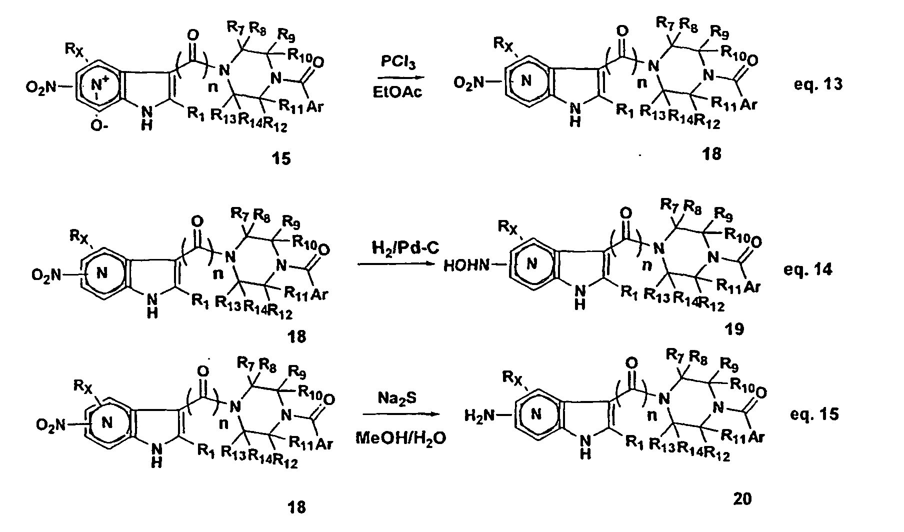

The alkylation of the nitrogen atom at position 1 of the azaindole derivatives can be achieved using NaH as the base, DMF as the solvent and an alkyl halide or sulfonate as alkylating agent, according to a procedure described in the literature (Mahadevan et al, Ref. 45) (eq. 16, Scheme 7).

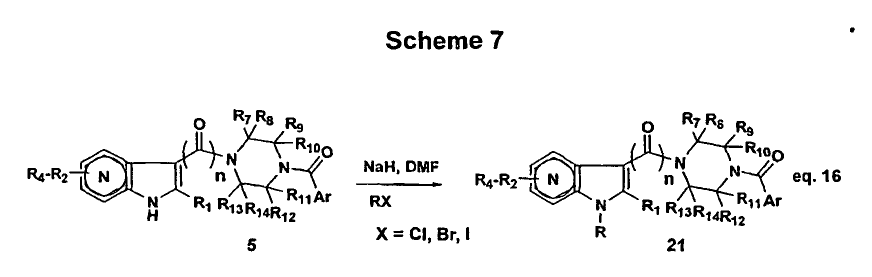

Halides can be converted to a variety of functionalities such as a nitrile (eq. 17), an amino group (eq. 18), and or an alkoxy group (eq. 19) (Scheme 8) using well established procedures. Examples of these types of transformations as depicted in eq.17 are shown in Sakamoto et al (Ref. 46 (a) in which a copper cyanide is used to form a nitrile from a halide, Halley et al (Ref. 46 (b)) which provides nitriles via copper I cyanide in DMF, Yamaguchi et al (Ref. 46 (c)), Funhoff et al (Ref. 46 (d)) uses CuCN in NMP, Shiotani et al (Ref. 37). Typically the reaction of CuCN to displace a halide requires heating. Temperatures such as 145°C for 18h have been found to be preferred but these conditions may be varied. The temperature may be raised or lowered by up to 100°C and reaction times may vary from as little 30 minutes to as long as 80h depending on reaction temperature and substrate. As an alternative to Eq. 17, Klimesova et al uses a primary amide precursor (which can come from the carboxylic acid as described elsewhere) and phosphorus oxy chloride to generate a nitrile (Ref. 47) and Katritzky et al (Ref.48). As shown in eq 18 halides can be displaced with amines or ammonia. Some example conditions are contained in Shiotani et. al. reference 37 and in Katritzky et.al. reference 48. For example heating the halide **9** in an excess of a primary or secondary amine as solvent at a temperature of reflux (or between 20°C and 200°C) will result in displacement of the halide to provide amines **27.** In the instance of ammonia or volatile amines, a pressure reactor as described in in Katritzky et.al. reference 48 can be utilized to carry out the reaction without losing the volatile amine during heating. The reactions may be monitored by TLC or or liquid chromatography and the reaction temperature increased until reaction is observed. Cosolvents such as dioxane or pyridine may be utilized when the amine is costly. An alternative method would employ the modified palldium catalysis methods of Hartwig (Yale) or Buchwald (MIT) to effect displacement under milder conditions. As shown in eq. 19 of Scheme 8, alkoxides may be used to displace halogens in **9** and provide ethers **26.** Typically this transformation is best carried out by adding sodium to a solution of the parent alcohol to generate an alkanoate. Alternatively a strong base such as NaH, or NaN(SiMe₃)₂ may be employed. The corresponding lithium or potassium bases or metals may also be utilized. Usually, an excess of base with respect to the halide to be displaced is employed. Between two and twenty equivalents of alkanoate are usually used with ten being preferred. The reaction is carried out at reflux or a temperature of between 30°C and 200°C. Typically approximately 80°C is useful. The reaction may take from four to eighty hours to reach completion with times between 12 and 48 hours being typical. As described above for eq.18, the reaction progress may be monitored. Typical conditions for displacement with sodium methoxide in methanol are provided in Shiotani et.al. reference 37 in the general procedure used for the preparation of examples 5a,5c, and 6 of the reference.

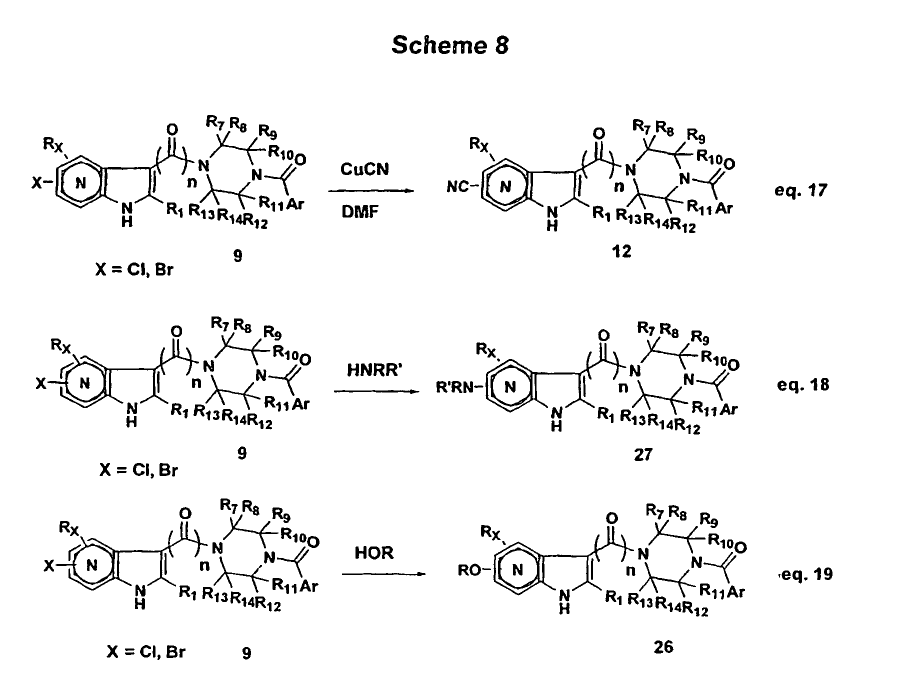

The nitrile group can be converted to a carboxylic acid **28** (eq. 20, using aqueous sodium hydroxide in ethanol as in Miletin et al, Ref. 49 (a); or using KOH in aqueous ethanol as in Shiotani et al, Ref. 49 (b); or using 6N HCl as in EI Hadri et al, Ref 49 (c)). The nitrile group can be converted to an ester **29** (eq. 21, using sodium methoxide in methanol as in Heirtzler et al, Ref. 50 (a); or using HCl in methanol as in Norrby et al, Ref. 50 (b)). The nitrile group can be converted to an amide **30** (eq. 22, using sulfuric acid as in Sitsun'Van et al, Ref. 51 (a); or using acetic acid, tertbutanol, sulfuric acid, and acetonitrile as in Reich et al, 51 (b); or using MeOS(O)₂F as in Salfetnikova et al, 51 (c)).

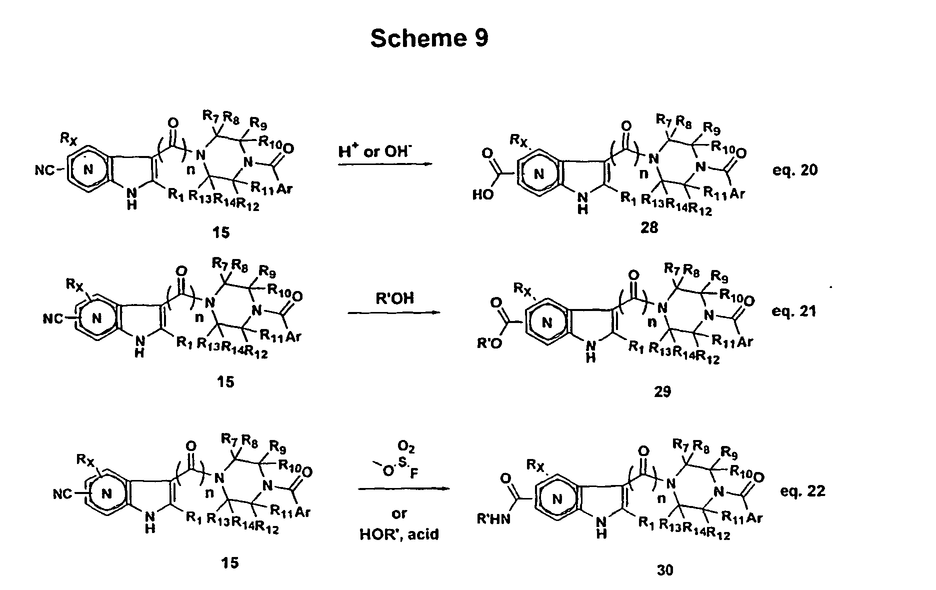

In Scheme10, the methyl group on the pyridine ring can be also oxidized to a carboxylic acid **28** using K₂Cr₂O₇ in 98% sulfuric acid as in (eq. 23, Oki et al, Ref. 52 (a); or using Chromium trioxide in conc sulfuric acid as in Garelli et al, Ref. 52 (b); or using selenium dioxide in pyridine as in Koyama et al, Ref. 52 (c)). The carboxylic acid may be transformed to an ester **29** using HCl in 10% methanol as in (eq. 24, Yasuda et al, Ref. 53 (a); or using thionyl chloride followed by a sodium alkyl alkoxide as in Levine et al, 53 (b); or using an alcohol and PyBOP in NMM, DMAP, and DMF as in Hoemann, 53 (c)).)). The carboxylic acid may be transformed to an amide **30** using aqueous KOH followed by oxalyl chloride in benzene followed by triethylamine in dichloromethane as in (eq. 25, Norman et al, Ref. 54 (a); or by heating an amine with the acid as in Jursic et al, 54 (b); or by coupling an amine to the acid with N,N-carbonyldiimidazole Strekowski et al, 54 (c); or by using oxalyl chloride in diethylether and an amine as in Shi et al, 54 (d)).

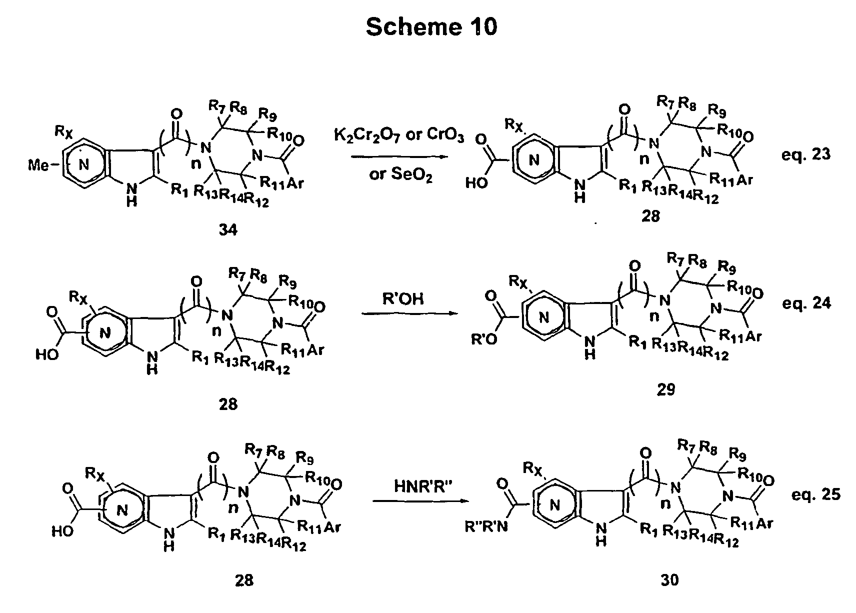

An alternative strategy for the synthesis of compounds containing varied substituents Ar is shown in Scheme 11. The benzamide moiety of the diamide **5** can be selectively hydrolyzed using to give intermediate **31.** Coupling of amine **31** with with other carboxylic acids under DEBPT and base using conditions described above for earlier couplings, provides other novel diamides **5.**

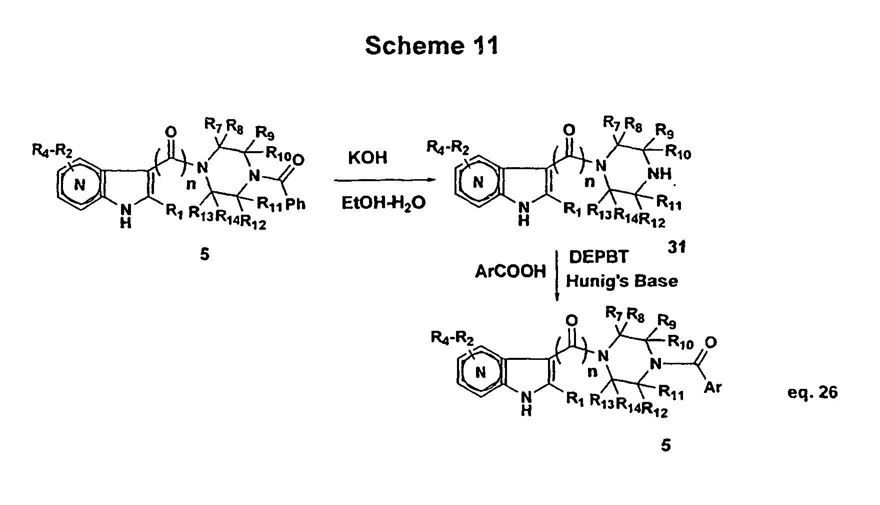

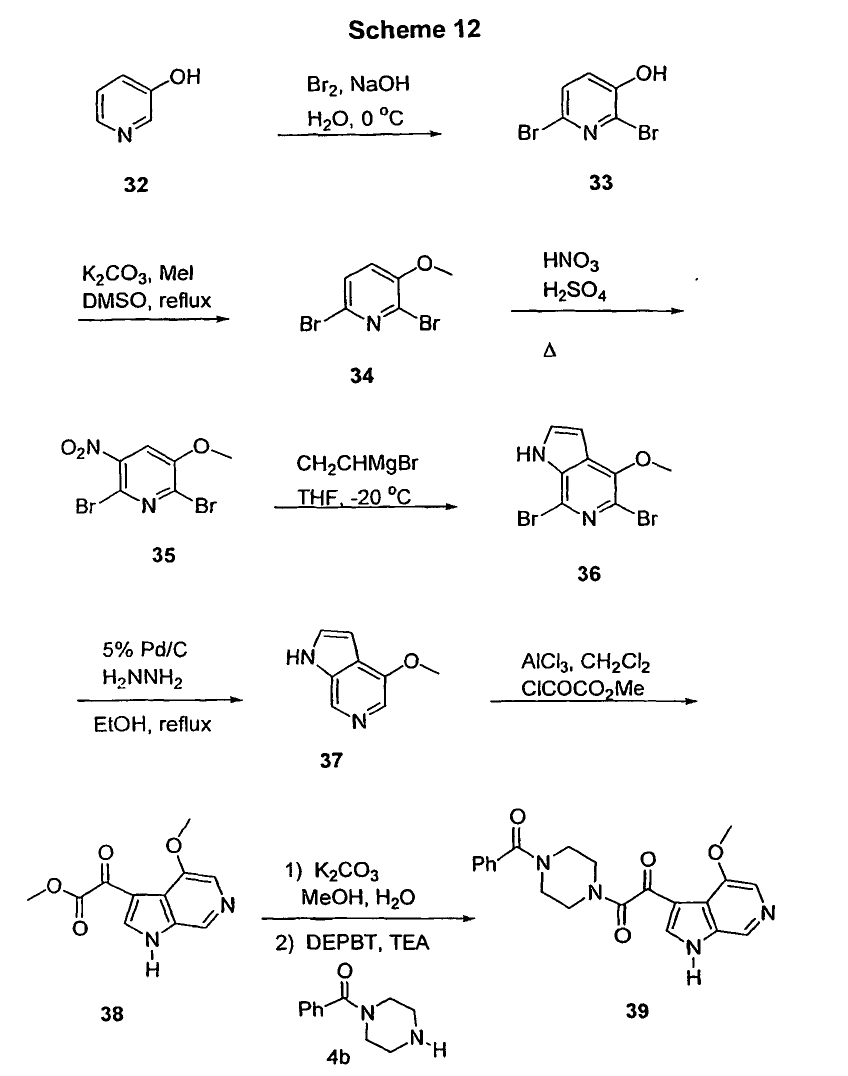

The preparation of compound **35** shown in Scheme 12 was carried out from commercially available **32** as described in Clark,G. J. ,Reference 56. The Bartoli methodology described in Scheme 1 was used to prepare 4-methoxy-6-azaindole **36.** Reduction of the bromides using transfer hydrogenation provided the desired 4-methoxy indole **37.** Compound **36** could be converted into a separable mixture of monobromides via selective lithium bromine exchange using t-Buli at cold temperatures of between -100 to -78° followed by a quench with ammonium chloride. The alternate methodology described in Scheme 3 for acylation with chloro methyl oxalate at the 3-position was applied to **37** as shown and provided intermediate **38.** The methodology of Scheme 3 could then be followed to provide compound **39.** While the methodology in Scheme 12 is the preferred route for preparing compound **39** and other compounds of formula I, an alternative route which is depicted in Scheme 13 was developed for preparing such compounds. Pyrrole **40** was prepared via the method described in Anderson, H. J., reference 57; Hydrolysis of ester **40** using standard conditions such as potassium hydroxide in ethanol at ambient temperature for ~2h or until completion provided potassium 2-pyrrolecarboxaldehyde-4-oxoacetate. A solution of this carboxylate salt, N-benzoylpiperazine hydrochloride, 3-(diethoxyphosphoryloxy)-1,2,3-benzotriazin-4(3*H*)-one and triethylamine in DMF was stirred for approximately one day or until completion to provide after workup and crystallization amide **41.** Amide/ aldehyde **41** was stirred as a slurry in EtOH for a short time of from 1 to 60 min., cooled to 0 °C (or between -15 and 20°) and then was stirred with glycine methyl ester hydrochloride, triethylamine (or alternatively Hunig's base, 2,6-Lutidine, or no base), and sodium cyanoborohydride to provide amine **42.** This transformation could also be carried out using aldehyde **41,** glycine methyl ester hydrochloride, and sodium triacetoxy borohydride in either dichloromethane, tetrahydrofuran, or C₁-C₄ alcohol solvents. Alternatively, the free base of glycine methyl ester could be substituted in either procedure and a dehydrating agent such as molecular sieves could be employed in the reaction prior to addition of the borohydride reducing agent. Alternatively this transformation could be carried out by first protecting the pyrrole nitrogen with a benzoyl (from benzoyl chloride and tertiary amine) or benzyl moiety (benzyl bromide, NaH or DBU in THF). The protecting groups can be removed when desired using hydrolysis with aqueous base or hydrogenation respectively. The methyl ester **42** was hydrolyzed using potassium carbonate in methanol to provide after acidification with HCl the corresponding carboxylic acid. The acid was placed in anhydrous methanesulfonic acid containing phosphorus pentoxide which had been preheated for between 15 and 40 minutes and heated at approximately 110° (usually between 90 and 150°) for a short time of approximately 15 minutes but usually less than an hour and then poured over ice. Acylation or benzoylation of the product using for example modified Schotten-Bauman conditions (dichloromethane, potassium carbonate, and benzoyl chloride) provided ketone **43.** Reaction with dimethoxy propane and anhydrous p-toluenesulfonic acid generates an intermediate enol ether which upon reaction with chloranil provided compound **39.** The enol ether can alteratively be prepared using trimethyl ortho acetate and a sulfonic acid catalyst. Azaindoles such as **39** can be functionalized into nitriles which are versatile intermediates by oxidation to the N-oxide followed by reaction with DEPC and TEA or phosphorus oxychloride followed by CuCN in DMF. Details for reactions which convert **41** into **43-45** using these conditions on a similar substrate are described in reference 58 which is Suzuki, H.; Iwata, C.; Sakurai, K.; Tokumoto, K.; Takahashi, H.; Hanada, M.; Yokoyama, Y.; Murakami, Y., Tetrahedron, 1997, 53(5), 1593-1606. It should be apparent that in Schemes 12 and 13, **4b** may be replaced with any of the substrates represented by formula **4** in Scheme 4. It should also be apparent that indole **37,39, 44,** and **45** may be elaborated using appropriate chemistry described in the Schemes 5-11 herein which describe general methodology for functionalization of the azaindoles.

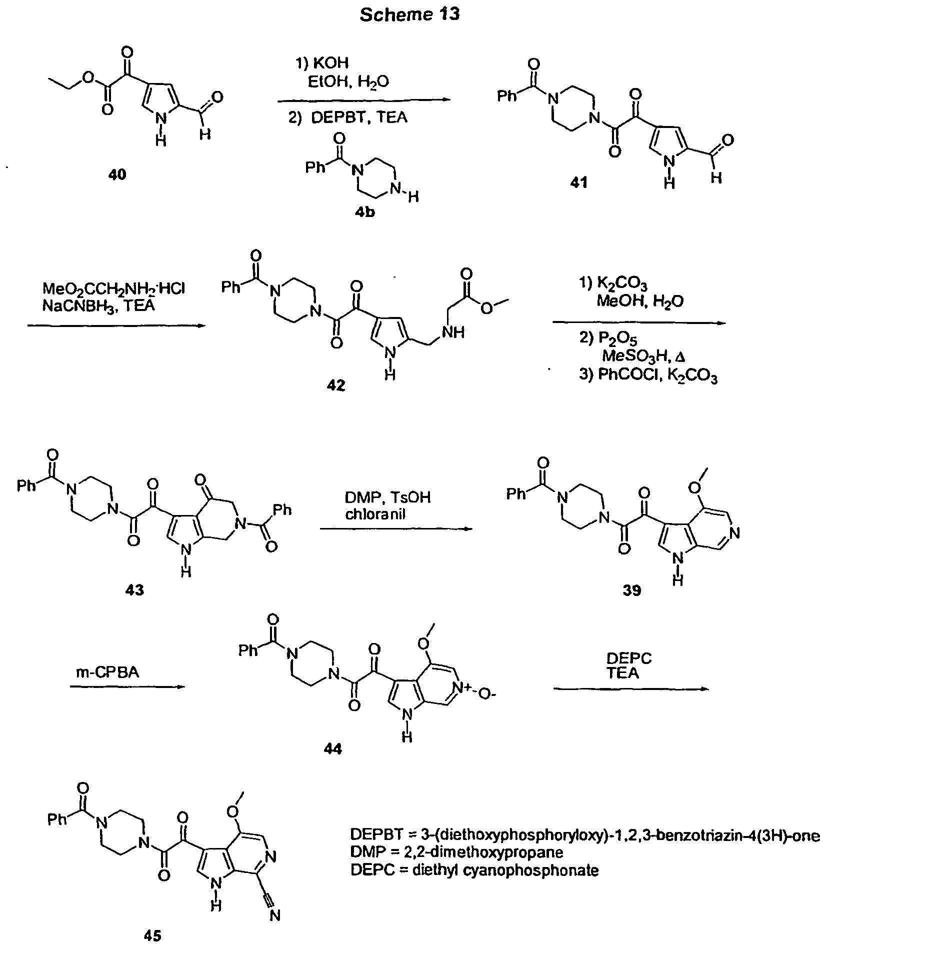

It should be noted that 2-chloro-5-fluoro-3-nitro pyridine may be prepared by the method in example 5B of reference 59 Marfat et.al. The chemistry in Schemes 1 and 3 to provide the derivative which corresponds to general formula 5 and has a 6-aza ring and R₂=F and R₄ = Cl. In particular, reaction of 2-chloro-5-fluoro-3-nitro pyridine with 3 equivalents of vinyl Magnesium bromide using the typical conditions described herein will provide 4-fluoro-7-chloro-6-azaindole in high yield. Addition of this compound to a solution of aluminum trichloride in dichlorometane stirring at ambident temperature followed 30 minutes later with chloromethyl or chloroethyl oxalate provides an ester. Hydrolysis with KOH as in the standard procedures herein provides an acid salt which reacts with piperazines **4** (for example 1-benzoyl piperazine) in the presence of DEPBT under the standard conditions described herein to provide the compound **5** described just above. The compound with the benzoyl piperazine is *N-(benzoyl)-N'-[(4-fluoro-7-chloro-6-azaindol-3 yl)-oxoacetyl]-piperazine* and is compound **5av.** The 7-chloro moiety in **5av** can be utilized by the methods of this invention to provide the desired derivatives where R₄ is substituted according to the general claim. For example, exposure of **5av** to sodium methoxide in refluxing methanol will provide the compound **5ay** in which the 6-azaindole ring contains a 4-fluoro-and 7-methoxy substituent. Alternatively, the 4-fluoro-7-chloro-6-azaindole may be reacted with sodium methoxide and then carried through the sequence as above to provide *N-(benzoyl)-N'[-(4-fluoro-7-methoxy-6-azaindol-3 yl)-oxoacetyl]-piperazine,* **5ay.** 4-fluoro-7-chloro-6-azaindole can also be reacted with CuCN/DMF as described in eq.17 to provide a 7-cyano intermediate which can be hydrolyzed to an acid as described in eq.21 Scheme 9 using HCl in MeOH at RT for 12h followed by reflux to complete the reaction. The acid can be smoothly converted to to a methly ester by adding diazomethane in ether to a stitting solution of the acid in diazometane at ambient temperature or lower. These are the standard conditions for using diazomethane which is conveniently generated as a solution in diethyl ether from Diazald® based on instructions which come with a kit from Aldrich Chemical Co. The methyl ester may be carried through the acylation using oxalyl chloride as shown in Scheme 4, followed by coupling with a piperazine (benzoyl piperazine for example) to generate the corresponding 4-fluoro-7-carbomethoxy-6-azaindole which upon addition to a solution of methylamine in water would provide **5az** which is *N-(benzoyl)-N'-[(4-fluoro-7-(N-methyl-carboxamido)-6-azaindol-3-yl)-oxoacetyl]-piperazine.* The same sequences of chemistry described above for 4-fluoro-7-chloroindole may be carried out using 7-chloro-4aza-indole and (R)-3-methyl-N-benzoylpiperazine **4a** to provide **5abc** which is *(R)-N-(benzoyl)-3-methyl-N-[(7-methoxy-4-azaindol-3-yl)-oxoacetyl]-piperazine* or **5abd** which is *(R)-N-(benzoyl)-3-methyl-N'-[(7-(N-methyl-carboxamido)-4-azaindol-3-yl)-oxoacetyl]-piperazine.* The starting 7-chloro-4-aza-indole is compound **1l** and its prepartion is described as in example in the experimental section.

It should be clear that in addition to compounds **5a-5abd** compounds **8, 11-30, 39, 44,** and **45** are all compounds of formula I and are within the scope of the invention.

Detailed descriptions of many of the preparations of piperazine analogs of compounds of this invention and conditions for carrying out the general reactions described herein are described in PCT WO 00/76521 published December 21, 2000.

In the general routes for substituting the azaindole ring described above, each process can be applied repeatedly and combinations of these processes are permissible in order to provide azaindoles incorporating multiple substituents. The application of such processes provides additional compounds of Formula I.

### Antiviral Activity

The antiviral activity of compounds was determined in HeLa CD4 CCR5 cells infected by single-round infectious HIV-1 reporter virus in the presence of compound at concentrations ≤ 10 µM. The virus infection was quantified 3 days after infection by measuring luciferase expression from integrated viral DNA in the infected cells (Chen et al, Ref. 55). The percent inhibition for each compound was calculated by quantifying the level of luciferase expression in cells infected in the presence of each compound as a percentage of that observed for cells infected in the absence of compound and subtracting such a determined value from 100. Compounds exhibiting anti-viral activity without appreciable toxicity at concentrations ≤ 10 µM are presented in Table 1.

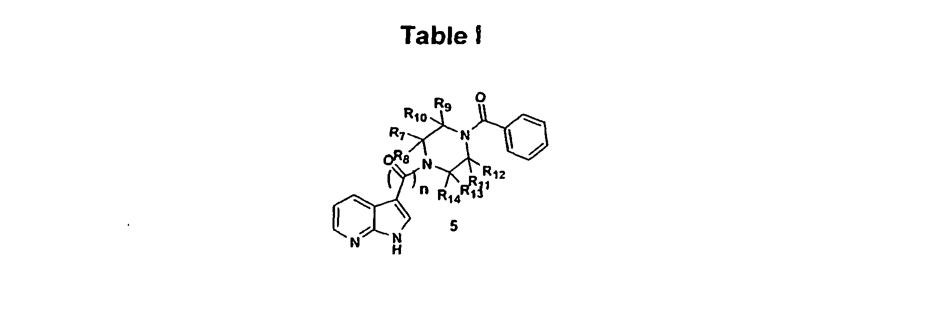

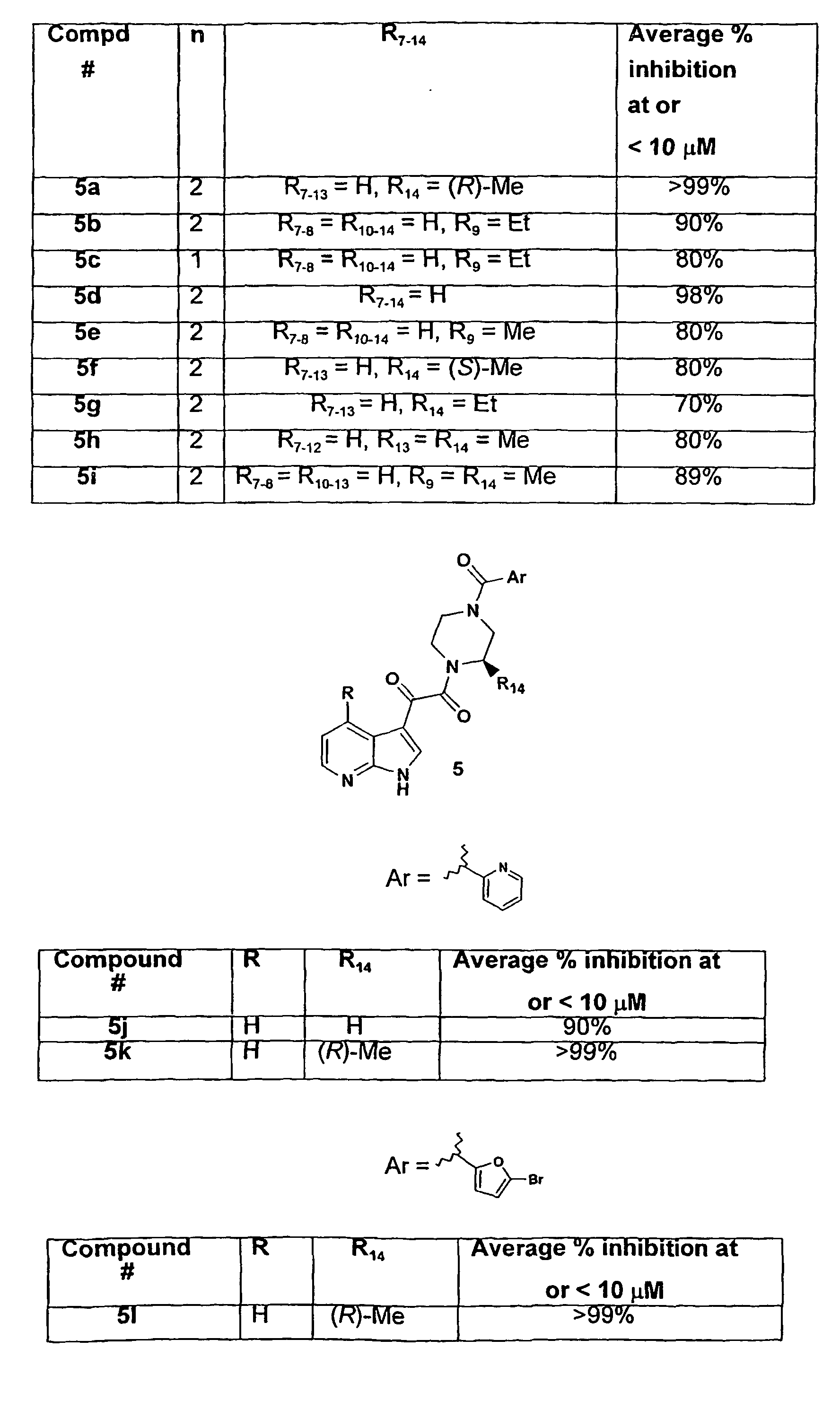

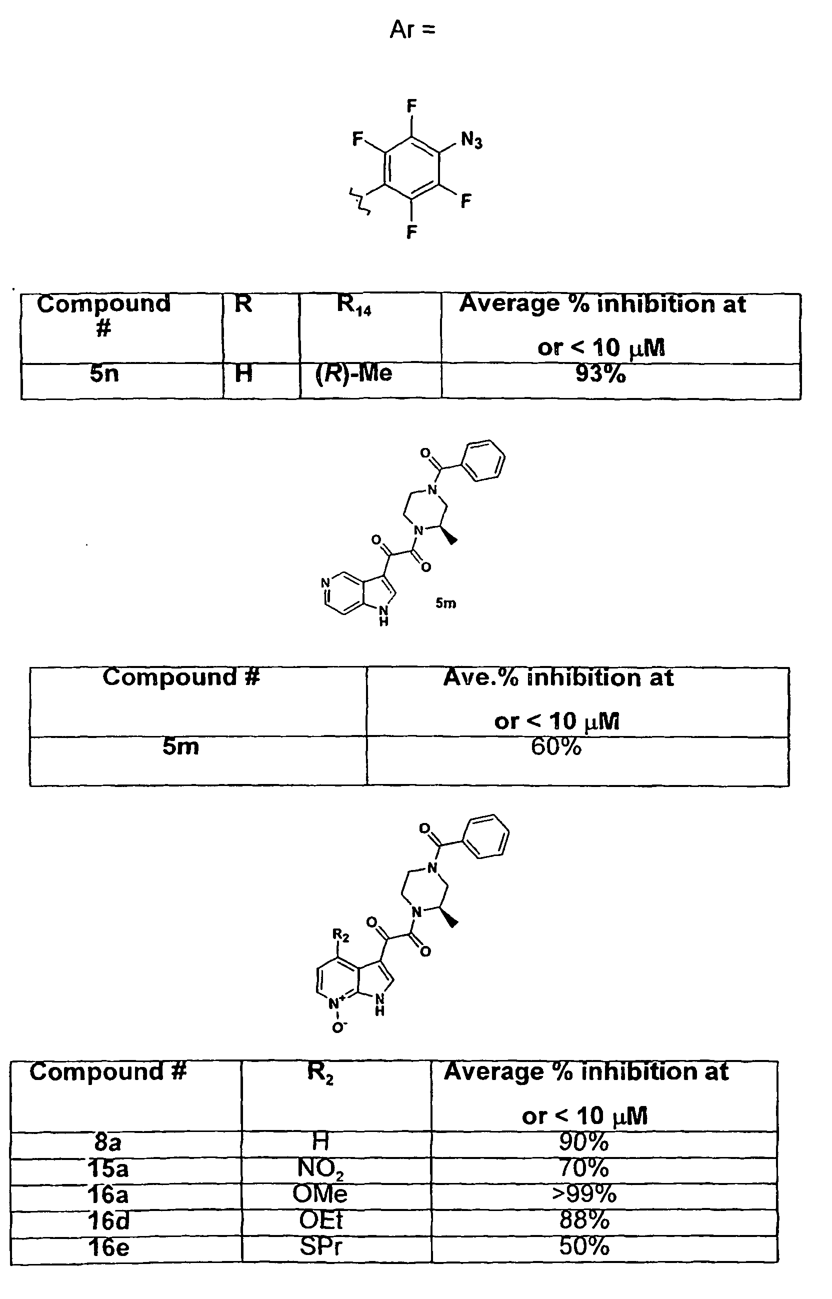

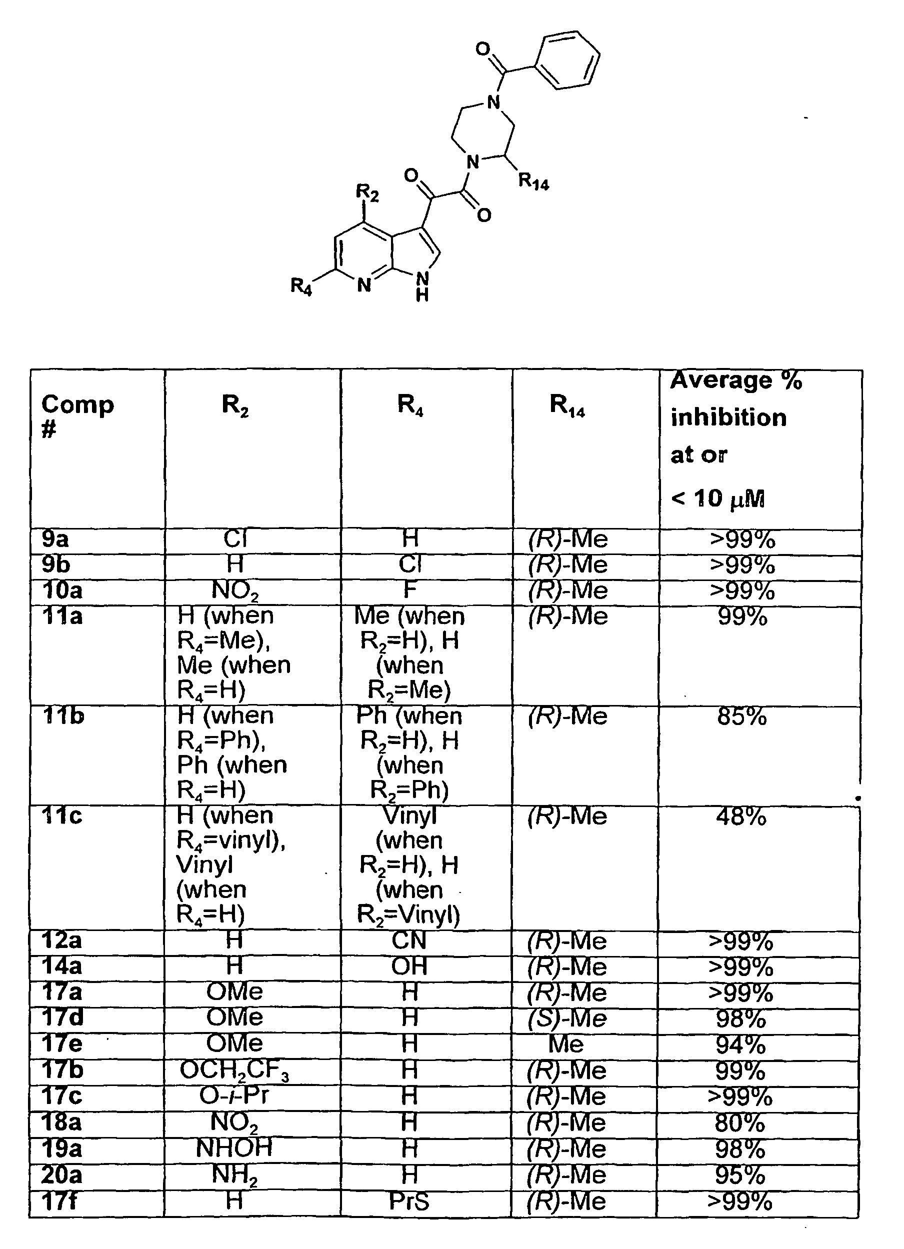

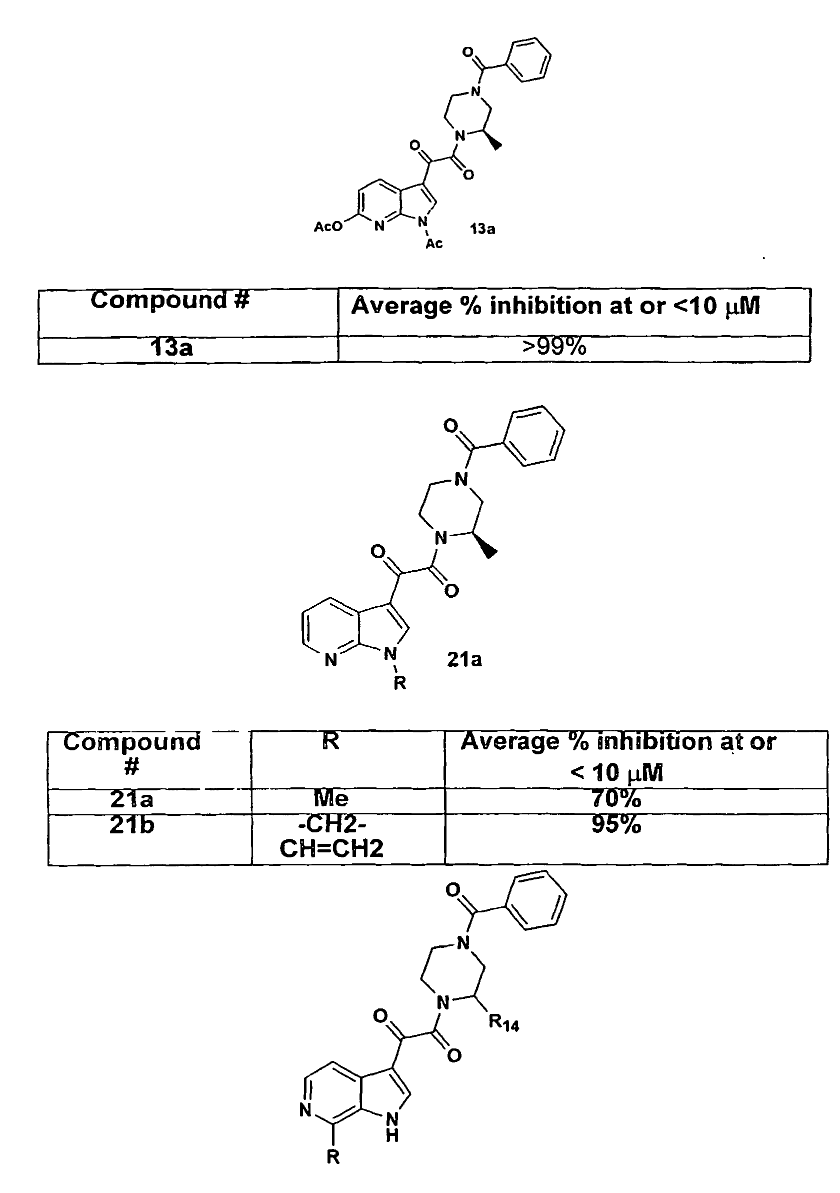

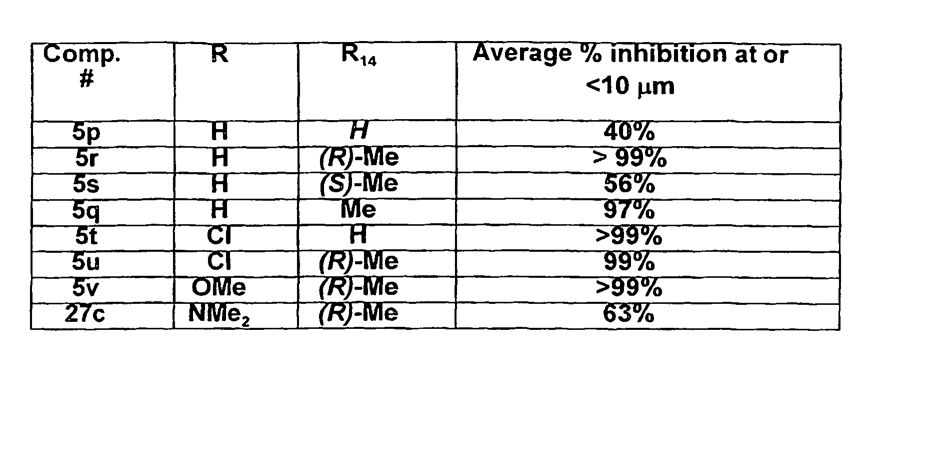

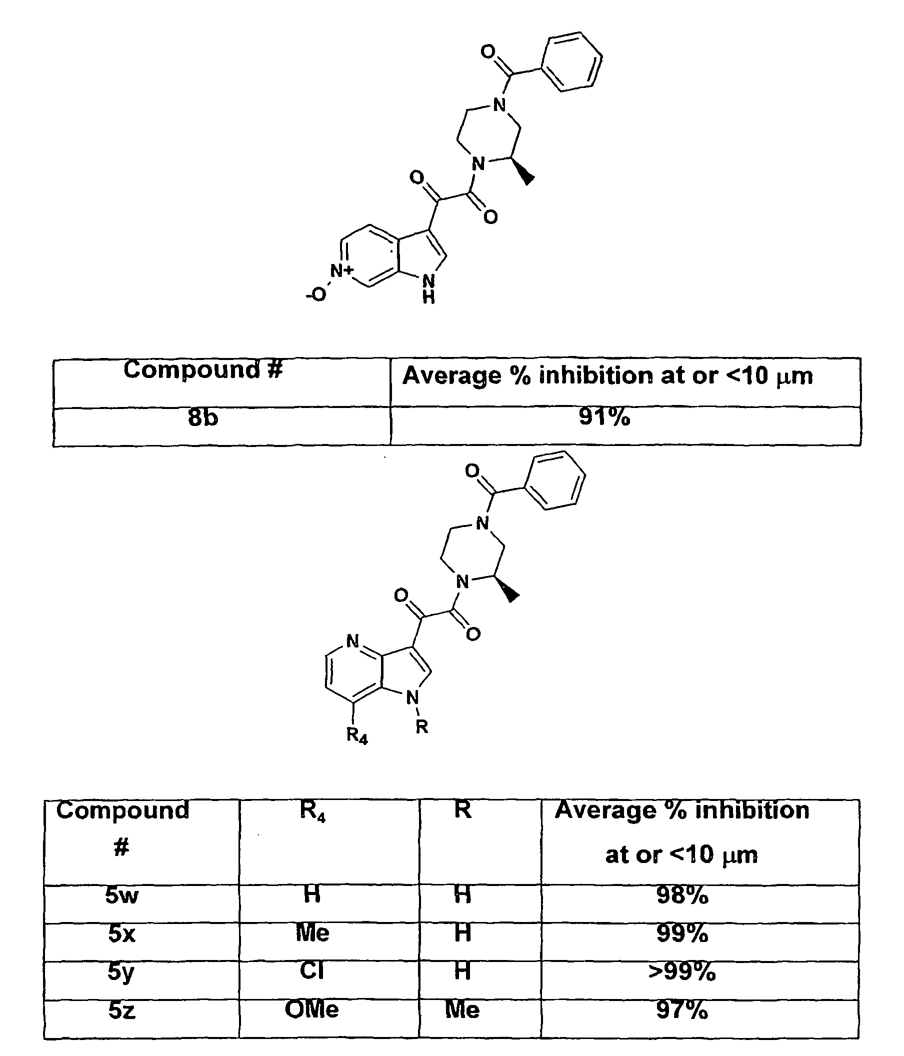

### Experimental Procedures

### Biology

In Table I and hereafter, the following definitions apply.
"µM" means micromolar;
- "ml" or "mL" means milliliter;
- "µl" means microliter;
- "mg" means milligram;
- "nM" means nanomolar
- "a" refers to percent inhibition data as representing the mean values of at least two experiments with duplicate determinations in each experiment.

The materials and experimental procedures used to obtain the results reported in Table I are described below.

### Cells:

- Virus production-Human embryonic Kidney cell line, 293, propagated in Dulbecco's Modified Eagle Medium (Life Technologies, Gaithersburg, MD) containing 10% fetal Bovine serum (FBS, Sigma, St. Louis , MO).
- Virus infection- Human epithelial cell line, HeLa, expressing the HIV-1 receptors CD4 and CCR5 was propagated in Dulbecco's Modified Eagle Medium (Life Technologies, Gaithersburg, MD) containing 10% fetal Bovine serum (FBS, Sigma, St. Louis , MO) and supplemented with 0.2 mg/ml Geneticin (Life Technologies, Gaithersburg, MD) and 0.4 mg/ml Zeocin (Invitrogen, Carlsbad, CA).

**Virus**-Single-round infectious reporter virus was produced by co-transfecting human embryonic Kidney 293 cells with an HIV-1 envelope DNA expression vector and a proviral cDNA containing an envelope deletion mutation and the luciferase reporter gene inserted in place of HIV-1 net sequences (Chen et al, Ref. 55). Transfections were performed using lipofectAMINE PLUS reagent as described by the manufacturer (Life Technologies, Gaithersburg, MD).

### Experiment

1. Compound was added to HeLa CD4 CCR5 cells plated in 96 well plates at a cell density of 5 X 10⁴ cells per well in 100 µl Dulbecco's Modified Eagle Medium containing 10 % fetal Bovine serum at a concentration of <20 µM.
2. 100 µl of single-round infectious reporter virus in Dulbecco's Modified Eagle Medium was then added to the plated cells and compound at an approximate multiplicity of infection (MOl) of 0.01, resulting in a final volume of 200 µl per well and a final compound concentration of <10 µM.
3. Samples were harvested 72 hours after infection.
4. Viral infection was monitored by measuring luciferase expression from viral DNA in the infected cells using a luciferase reporter gene assay kit (Roche Molecular Biochemicals, Indianapolis, IN). Infected cell supernatants were removed and 50 µl of Dulbecco's Modified Eagle Medium (without phenol red) and 50 µl of luciferase assay reagent reconstituted as described by the manufacturer (Roche Molecular Biochemicals, Indianapolis, IN) was added per well. Luciferase activity was then quantified by measuring luminescence using a Wallac microbeta scintillation counter.
5. The percent inhibition for each compound was calculated by quantifying the level of luciferase expression in cells infected in the presence of each compound as a percentage of that observed for cells infected in the absence of compound and subtracting such a determined value from 100.

### Method for extrapolating % inhibition at 10µM

The data in Table 1 was obtained using the general procedures above and by the following methods. Data is not reported for all compounds since data for all the compounds is reported by the alternate method in Table 2. The percent inhibition for each compound was calculated by quantifying the level of luciferase expression in cells infected in the presence of compound as a percentage of that observed for cells infected in the absence of compound and subtracting such a determined value from 100. For compounds tested at concentrations less than 10 µM, the percent inhibition at 10 µM was determined by extrapolation using the XLfit curve fitting feature of the Microsoft Excel spreadsheet software. Curves were obtained from 10 data points (% inhibition determined at 10 concentrations of compound) by using a four parameter logistic model (XLfit model 205: y = A + ((B-A)/(1+((C/x)^{D}))), where, A = minimum y, B = maximum y, C = logEC₅₀, D = slope factor, and x and y are known data values. Extrapolations were performed with the A and B parameters unlocked.

### Biological Data Expressed as an EC₅₀

Table 2 presents the data for the compounds grouped based on their EC₅₀ which provides an additional method for comparing the antiviral potency of the compounds of this invention. These values were calculated by the following method. The effective concentration for fifty percent inhibition (EC50) was calculated with the Microsoft Excel XLfit curve fitting software. For each compound, curves were generated from percent inhibition calculated at 10 different concentrations by using a four paramenter logistic model (model 205).

**Table 2.**

| Biological Data Expressed as EC₅₀s | | |
|---|---|---|
| **Compounds* with EC**_{**50**}**s** | **Compounds with EC**_{**50**}**s >1 µM but <5µM** | **Compounds with EC50 < 1 µM** |
| **> 0.4 µM: 5ac. >0.5 µM: 5m, 5p, 5s, 5ab, 5ad, 5ae, 5ab, 5ad, 5ae, 16b, 16c, 16h,** | **5h, 11b, 18a,** | **5a, 5b, 5c, 5d, 5e, 5f, 5g, 5i, 5j, 5k, 5l, 5n, 5q, 5r, 5t, 5u, 5v, 5r, 5t, 5u, 5v, 5w, 5x, 5y, 5z, 5ai, 5ak, 8a, 8b,** |
| **17f, 17g, 17h. >5 µM: 5af, 5ag, 5ah, 8e, 11c, 16e, 17g,** | | **9a, 9b, 10a, 11a, 12a, 13a, 15a, 16a, 16d, 17a, 17b, 17c, 17d, 17e, 19a, 20a, 21a, 21b, 27c,39** |

| | | |
|---|---|---|
| *Some of these compounds were tested at a concentration lower than their EC₅₀ but showed some ability to cause inhibition and thus should be evaluated at a higher concentration to determine the exact EC₅₀. An approximate attempt to exclude compounds which did not show some potential for inhibition (those which might have an EC50 > 100uM) was made. | | |

### Chemistry

All Liquid Chromatography (LC) data were recorded on a Shimadzu LC-10AS liquid chromatograph using a SPD-10AV UV-Vis detector with Mass Spectrometry (MS) data determined using a Micromass Platform for LC in electrospray mode.

### LC/MS Method (i.e., compound identification)

| | |
|---|---|
| Column A | YMC ODS-A S7 3.0×50 mm column |
| Column B | PHX-LUNA C18 4.6x30 mm Column |
| Gradient | 100% Solvent A / 0% Solvent B to 0% Solvent A / 100% Solvent B |
| Gradient time | 2 minutes |
| Hold time | 1 minute |
| Flow rate | 5 ml/min |
| Detector Wavelength | 220 nm |
| Solvent A | 10% MeOH / 90% H₂O / 0.1 % Trifluoroacetic Acid |
| Solvent B | 10% H₂O / 90% MeOH / 0.1 % Trifluoroacetic Acid |

Compounds purified by preparative HPLC were diluted in methanol (1.2 ml) and purified using the following methods on a Shimadzu LC-10A automated preparative HPLC system.

### Preparative HPLC Method (i.e., compound purification)

Purification Method: Initial gradient (30% B, 70% A) ramp to final gradient (100% B, 0% A) over 20 minutes, hold for 3 minutes (100% B, 0% A)

| | |
|---|---|
| Solvent A | 10% MeOH / 90% H₂O/ 0.1 % Trifluoroacetic Acid |
| Solvent B | 10% H₂O / 90% MeOH / 0.1% Trifluoroacetic Acid |
| Column | YMC C18 S5 20x100 mm column |
| Detector Wavelength | 220 nm |

### Typical Procedure and Characterization of Selected Examples

### Typical Procedure for the Preparation of Compounds in Scheme 1

### 1) Preparation of Azaindole 1

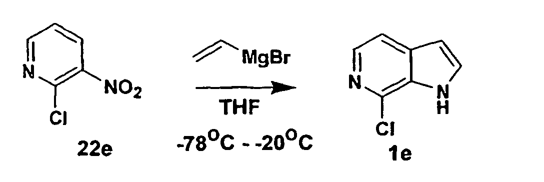

Preparation of azaindole, Method A: Preparation of 7-Chloro-6-azaindole **1e:** 2-Chloro-3-nitropyridine **22e** (5.0 g) was dissolved in dry THF (200 ml). After the solution was cooled down to -78°C, an excess of vinyl magnesium bromide (1.0 M in THF, 100 ml) was added. Then, the reaction was left at -20°C for eight hours before quenched with 20% NH₄Cl (150 ml). The aqueous phase was extracted with EtOAc (3 x 150 ml). The combined organic layer was dried over MgSO₄. After filtration and concentration, the crude product was purified by silica gel column chromatography to afford 1.5 g of 7-chloro-6-azaindole **1e** in 31% yield.

Summarized below is the characterization of compounds **1** with the following structures:

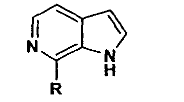

Compound 1e, R = Cl, 7-Chloro-6-azaindole: ¹H NMR (500 MHz, CD₃OD) δ 7.84 (d, 1H, *J* = 7.95 Hz), 7.76 (m, 2H), 6.61 (d, 1H, *J* = 5.45 Hz). MS *m*/*z*: (M+H)⁺ calcd for C₇H₆ClN₂: 153.02; found 152.93. HPLC retention time: 0.51 minutes (column A).

Compound 1f, R = OMe, 7-Methoxy-6-azaindole: MS *m*/*z*: (M+H)⁺ calcd for C₈H₉N₂O: 149.07; found 149.00. HPLC retention time: 0.42 minutes (column A).

Characterization of compounds 1 with the following substructure prepared by the method above:

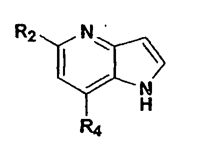

Compound **1g**, R₂ = H, R₄ = Me, 7-Methyl-4-azaindole: MS *m*/*z*: (M+H)⁺ calcd for C₈H₉N₂: 133.08; found 133.01. HPLC retention time: 0.34 minutes (column A).

Compound **1ak**, R₂ = Cl, R₄ = Me, 5-Chloro-7-methyl-4-azaindole: MS *m*/*z*: (M+H)⁺ calcd for C₈H₈ClN₂: 167.04; found 166.99. HPLC retention time: 1.22 minutes (column B).

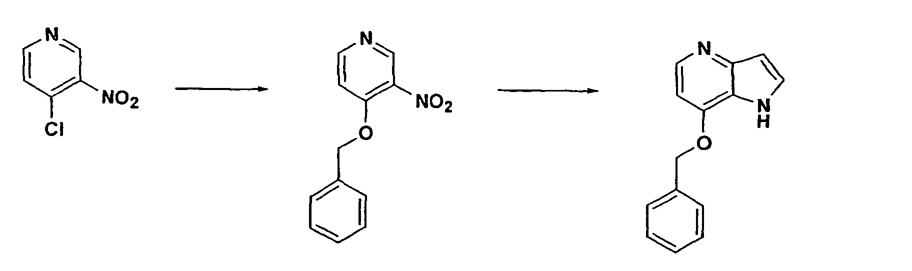

Preparation of azaindole, Method A: Preparation of 7-Benzyloxy-4-azaindole **1j**: To a solution of benzyl alcohol (16.6 g) in 200 ml of DMF was added NaH (4.8 g) slowly. The mixture was stirring at room temperature for 2 hours to afford sodium benzoxide, which was transferred into a solution of 4-chloro-3-nitropyridine hydrochloride 22j (20 g) in DMF (100 ml). The resulting mixture was kept stirring for 10 hours before quenched with water. After DMF was removed under vaccum, the crude product was suspended in water and extracted with EtOAc (3 x 250ml). The organic phase was dried over MgSO₄ and concentrated to give a residue, which was purified via recrystallization to afford 6.1 g of 4-benzoxy-3-nitropyridine **22j.**

### Characterization of compound 22j:

4-benzyloxy-3-nitropyridine: MS *m*/*z:* (M+H)⁺ calcd for C₁₂H₁₁N₂O₃: 231.08; found 231.06. HPLC retention time: 1.46 minutes (column A).

Preparation of compound **1j,** 7-benzoxy-4-azaindole: The general procedure and conditions described for the Bartoli-type reaction used to prepare **1e** were followed.

### Characterization of compound 1j:

Compound **1j,** 7-benzyloxy-4-azaindole: ¹H NMR (500 MHz, CDCl₃) δ 8.64 (b, 1H), 8.34 (d, 1H, *J* = 5.35 Hz), 7.40 (m, 6H), 6.72 (d, 1H, *J* = 3.25 Hz), 6.67 (d, 1H, J = 5.45 Hz), 5.35 (s, 2H); ¹³C NMR (125 MHz, CDCl₃) δ 151.1, 147.9, 145.2, 135.8, 128.8, 128.6, 127.9, 126.3, 119.6, 103.9, 99.6, 70.2. MS *m*/*z*: (M+H)⁺ calcd for C₁₄H₁₃N₂O: 225.10; found 225.03. HPLC retention time: 1.11 minutes (column A).

Preparation of azaindole, Typical example for Method B: Preparation of 7-chloro-4-azaindole **1i:**

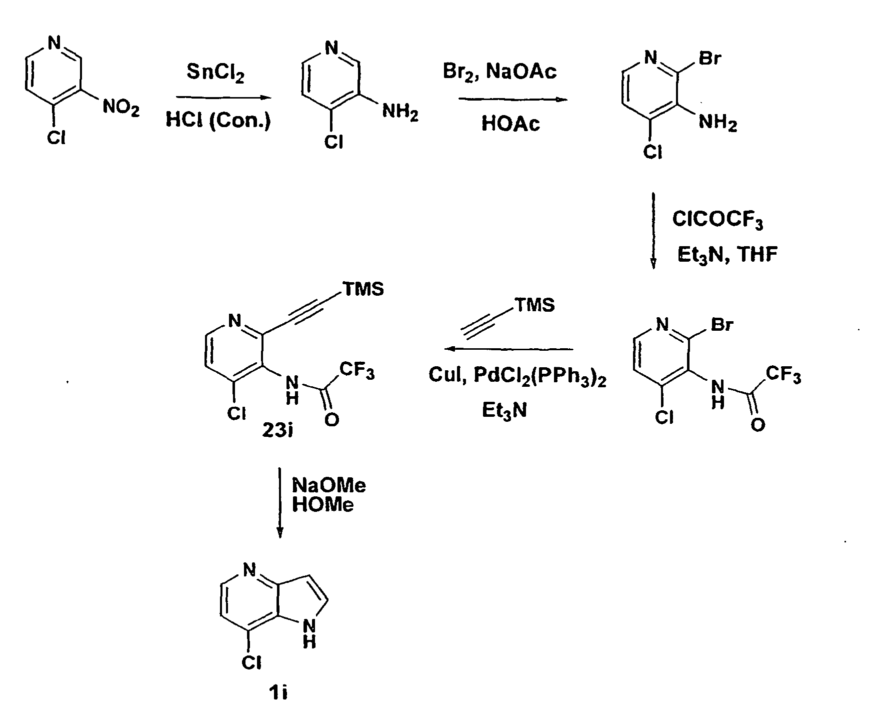

An excess of SnCl₂ (25 g) was cautiously added into a solution of 4-chloro-3-nitropyridine hydrochloride (5 g) in concentrated HCl and the reaction was stirred for 12 hours. Concentration under pressure provided a mixture, which was neutralized with 2N NaOH to pH 6-7. The aqueous phase was extracted with EtOAc (5 x 100 ml). The organic layers were then combined, dried over anhydrous MgSO₄ and concentrated *in vacuo* to give a crude product (2.2 g), which was 4-chloro-3-nitropyridine which was pure enough for direct use in further reactions.

7g of the crude product from the previous step was dissolved in 200 ml of TFA. Then, 10.7 g of NBS was added into the mixed solution cautiously. After 8 hours, solvent was removed under vacuum. The residue was dissolved in 2N NaOH (200 ml) and aqueous layer was extracted with EtOAc (3 x 200 ml). The combined organic layer was dried over MgSO₄ and concentrated to provide a crude product with was purified via recrystallization in hexane to afford 5 g of 3-amino-2-bromo-4-chloropyridine.

Characterization of 3-amino-2-bromo-4-chloropyridine:
MS *m*/*z*: (M+H)⁺ calcd for C₅H₅BrClN₂: 206.93; found 206.86. HPLC retention time: 1.32 minutes (column B).

To a solution of 3-amino-2-bromo-4-chloropyridine in 250 ml of ether was added 8.4 g of trifluoroacetic anhydride at 0°C. 5.3 g of Na₂CO₃ was added 10 minutes later, and the reaction mixture was stirred at room temperature for 10 hours before the reaction was quenched with water (100 ml). The aqueous phase was extracted with EtOAc (3 x 150 ml). The combined organic layer was dried over MgSO₄ and concentrated to give a residue, which was purified by silica gel column chromatography to afford 3.7 g of compound **23i.**

### Characterization of compound 23i:

2-Bromo-4-chloro-3-trifluoroacetaminopyridine: MS *m*/*z*: (M+H)⁺ calcd for C₇H₄BrClF₃N₂O: 302.90; found 302.91. HPLC retention time: 1.48 minutes (column B).

A mixture of compound **23i** (0.9 g), trimethylsilylacetylene (0.49 g), Pd Cl₂(PPh₃)₂ (0.1 g) and Cul (0.05g ) in Et₃N (1.5 ml) was heated to 100°C in sealed tube for 10 hours. Then, solvent was removed under vaccum. The residue was partitioned between water (10 ml) and EtOAc (10 ml). Aqueous phase was extracted with EtOAc (2 x 10 ml). The combined organic layer was dried over MaSO₄ and concentrated under vaccum to provide a crude product **24i** which was used in the further reaction without purification.

### Characterization of compound 24i:

Compound **24i,** 4-Chloro-3-trifluoroacetamido-2-(trimethylsilylethynyl)pyridine: MS *m*/*z*: (M+H)⁺ calcd for C₇H₄BrClF₃N₂O: 321.04; found 320.99. HPLC retention time: 1.79 minutes (column B).

A mixture of compound **24i** (0.28 g) and sodium ethoxide (0.30 ml) in 20 ml of ethanol was heated to reflux for 10 hours under nitrogen atmosphere. After solvent removed under vaccum, the residue was purified using Shimadzu automated preparative HPLC System to give compound **1i** i (0.1 g).

### Characterization of compound 1i:

Compound 1i, 7-Chloro-4-azaindole: ¹H NMR (500 MHz, CD₃OD) δ 8.50 (d, 1H, *J* = 6.20 Hz), 8.10 (d, 1H, *J* = 3.20 Hz), 7.71 (d, 1H, *J* = 6.30 Hz), 6.91 (d, 1H, J = 3.25 Hz). MS *m*/*z*: (M+H)⁺ calcd for C₇H₆CIN₂: 153.02; found 152.90. HPLC retention time: 0.45 minutes (column A).

### 1) Preparation of azaindole 3-glyoxylmethyl ester 2

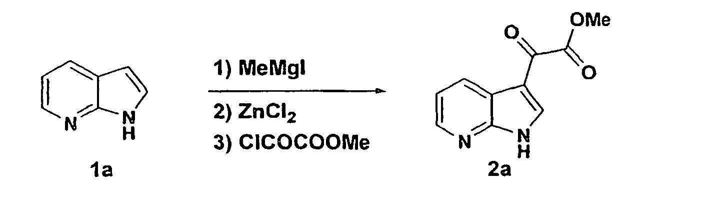

*Acylation* of *azaindole, method* A: *Preparation* of *Methyl (7-azaindol-3-yl*)*-oxoacetate* **2a:** To a solution of 7-azaindole **1a** (20.0 g, 0.169 mol) in dry CH₂Cl₂ (1000 ml), 62.1 ml of MeMgl (3.0M in Et₂O, 0.186 mol) was added at room temperature. The resulting mixture was stirred at room temperature for 1 hour before ZnCl₂ (27.7 g, 0.203 mol) was added. One hour later, methyl chlorooxoacetate (24.9 g, 0.203 mol) was injected into the solution dropwise. Then the reaction was stirred for 8 hours before being quenched with methanol.

After all solvents were evaporated, the residue was partitioned between ethyl acetate (500 ml) and H₂O (300 ml). The aqueous phase was neutralized with saturated Na₂CO₃ to pH 6-6.5, and extracted with EtOAc (3 x 500 ml). The organic layers were then combined, washed with 0.1N HCl (3 x 200 ml), dried over anhydrous MgSO₄ and concentrated *in vacuo* to give a crude product **2a** (14.3 g, 41.5%), which was pure enough for the further reactions.

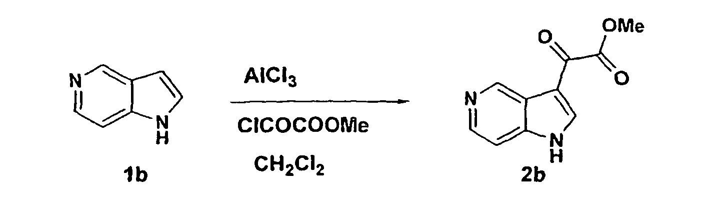

*Acylation* of *azaindole, method B: Preparation of Methyl (5-azaindol-3-yl)-oxoacetate **2b:*** 5-Azaindole **1b** (0.5 g, 4.2 mmol) was added to a suspension of AlCl₃ (2.8 g, 21.0 mmol) in CH₂Cl₂ (100 ml). Stirring was continued at room temperature for 1 hour before methyl chlorooxoacetate (2.5 g, 21.0 mmol) was added dropwise. The reaction was stirred for 8 hours. After 20 ml of MeOH was added cautiously to quench the reaction, solvents were removed under vaccum. The solid residue was purified by silica gel column chromatography (EtOAc/MeOH = 10 : 1) to afford 0.6 g (70%) of the acylated product **2b.**

### Characterization of compounds 2:

Compound **2a,** *Methyl (7-azaindol-3-yl)-oxoacetate:* ¹H NMR (300 MHz, DMSO-d₆) δ 8.60 (s, 1H), 8.47 (d, 1H, *J* = 7.86 Hz), 8.40 (d, 1H, *J* = 4.71 Hz), 7.34 (dd, 1H, *J* = 7.86, 4.77 Hz), 3.99 (s, 3H); ¹³C NMR (75 MHz. DMSO-d₆) δ 178.7, 163.3, 149.0, 145.1, 138.8, 129.7, 119.0, 118.0, 111.2, 52.7. MS *m*/*z*: (M+H)⁺ calcd for C₁₀H₉N₂O₃: 205.06; found 205.04. HPLC retention time: 0.94 minutes (column A).

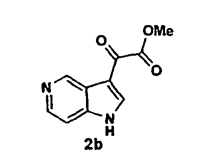

Compound **2b,** *Methyl (5-azaindol-3-yl)-oxoacetate:* ¹H NMR (500 MHz, CD₃OD) δ 9.61 (s, 1H), 9.02 (s, 1H), 8.59 (d, 1H, *J* = 6.63 Hz), 8.15 (d, 1H, *J* = 6.60 Hz), 4.00 (s, 3H); ¹³C NMR (125 MHz, CD₃OD) δ 178.9, 163.0, 145.6, 144.2, 138.3, 135.0, 124.7, 116.3, 112.1, 53.8. MS *m*/*z*: (M+H)⁺ calcd for C₁₀H₉N₂O₃: 205.06; found 205.04. HPLC retention time: 0.32 minutes (column A).

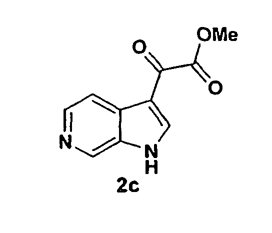

Compound **2c,** *Methyl (6-azaindol3-yl)-oxoacetate:* MS *m*/*z:* (M+H)⁺ calcd for C₁₀H₉N₂O₃: 205.06; found 205.14. HPLC retention time: 0.61 minutes (column A).

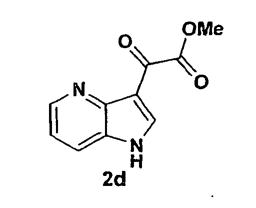

Compound **2d,** *Methyl (4-azaindol-3-yl)-oxoacetate:* MS *m*/*z*: (M+N)⁺ calcd for C₁₀H₉N₂O₃: 205.06; found 204.99. HPLC retention time: 0.34 minutes (column A).

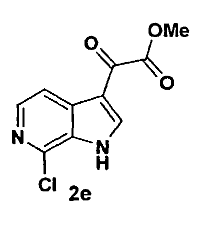

Compound **2e,** *Methyl (7-chloro-6-azaindol-3-yl)-oxoacetate:* ¹H NMR (500 MHz, DMSO-d₆) δ 8.66 (s, 1H), 8.17 (d, 1H, *J* = 5.35 Hz), 8.05 (d, 1H, *J* = 5.30 Hz), 3.91 (s, 3H); ¹³C NMR (125 MHz, DMSO-d₆) δ 178.4, 162.7, 141.3, 140.9, 134.6, 133.0, 130.1, 115.4, 113.0, 52.8. MS *m*/*z*: (M+H)⁺ calcd for C₁₀H₈ClN₂O₃: 239.02; found 238.97. HPLC retention time: 1.18 minutes (column A).

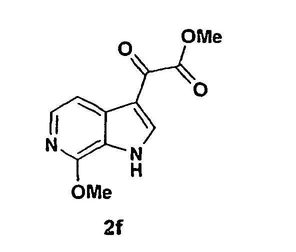

Compound **2f,** *Methyl (7-methoxy-6-azaindol-3-yl)-oxoacetate:* MS *m*/*z:* (M+H)⁺ calcd for C₁₁H₁₁N₂O₄: 235.07; found 234.95. HPLC retention time: 0.95 minutes (column A).

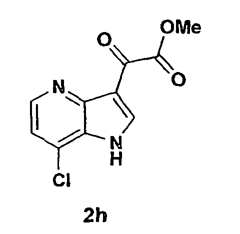

Compound **2h,** *Methyl (7-chloro-4-azaindol-3-yl)-oxoacetate:* MS *m*/*z:* (M+H)⁺ calcd for C₁₀H₈CIN₂O₃: 239.02; found 238.97. HPLC retention time: 0.60 minutes (column A).

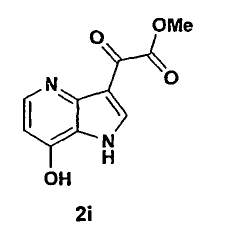

Compound **2i**, *Methyl (7-hydroxyl-4-azaindol-3-yl)-oxoacetate:* MS *m*/*z*: (M+H)⁺ calcd for C₁₀H₉N₂O₄: 221.06; found 220.96. HPLC retention time: 0.76 minutes (column A).

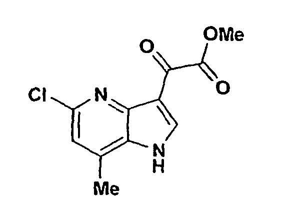

Compound **2ak,** *Methyl (5-chloro-7-methyl-4-azaindol-3-yl)-oxoacetate:* MS *m*/*z:* (M+H)⁺ calcd for C₁₁H₁₀ClN₂O₃: 253.04; found 252.97. HPLC retention time: 1.48 minutes (column B).

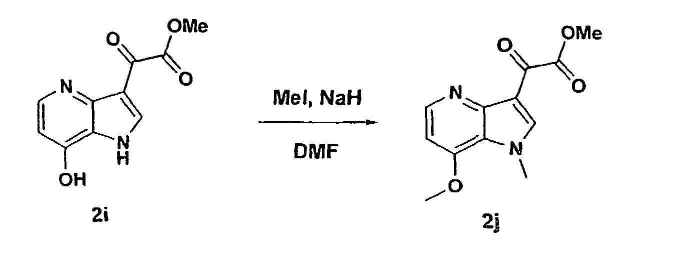

Preparation of compound 2j, Methyl (7-methoxyl-1-methyl-4-azaindol-3-yl)-oxoacetate: To a solution of compound **2i** (27 mg) in 10 ml of dry DMF was added 4.4 mg of NaH. After 1 hour, 26 mg of Mel was added and the mixture was stirred at room temperature for 10 hours. DMF was then removed under vaccum to provide a crude product **2j** which was used in the further reaction without purification.

### Characterization of compounds 2j:

Compound **2j,** *Methyl (7-methoxy-1-methyl-4-azaindol-3-yl)-oxoacetate:* MS *m*/*z*: (M+H)⁺ calcd for C₁₂H₁₃N₂O₄: 249.09; found 249.33. HPLC retention time: 0.91 minutes (column A).

### 2) Preparation of potassium azaindole 3-glyoxylate 3

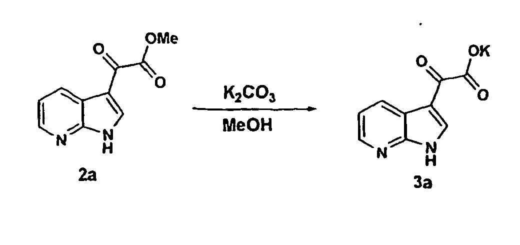

*Preparation of Potassium* (7*-azaindol-3-yl)-oxoacetate **3a:*** Compound **2a** (43 g, 0.21 mol) and K₂CO₃ (56.9g, 0.41 mol) were dissolved in MeOH (200 ml) and H₂O (200 ml). After 8 hours, product **3a** precipitated out from the solution. Filtration afforded 43 g of compound **3a** as a white solid in 90.4% yield.

### Characterization of compounds 3:

Compound **3a,** *Potassium (7-azaindol-3-yl)-oxoacetate:* ¹H NMR (300 MHz, DMSO-d₆) δ 8.42 (d, 1H, *J* = 7.86 Hz), 8.26 (d, 1H, *J* = 4.71 Hz), 8.14 (s, 1H), 7.18 (dd, 1H, *J* = 7.86, 4.71Hz); ¹³C NMR (75 MHz, DMSO-d₆) δ 169.4, 148.9, 143.6, 135.1, 129.3, 118.2, 117.5, 112.9. MS *m*/*z*: (M+H)⁺ of the corresponding acid of compound **3a** (**3a**-K+H) calcd for C₉H₇N₂O₃: 191.05; found 190.97. HPLC retention time: 0.48 minutes (column A).

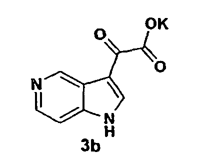

Compound **3b,** *Potassium (5-azaindol-3-yl)-oxoacetate:* MS *m*/*z:* (M+H)⁺ of the corresponding acid of compound **3b** (**3b**-K+H) calcd for C₉H₇N₂O₃: 191.05; found 191.02. HPLC retention time: 0.13 minutes (column A).

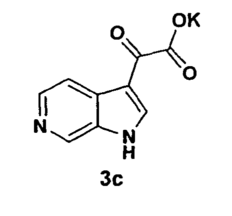

Compound **3c,** *Potassium (6-azaindol-3-yl)-oxoacetate:* MS *m*/*z:* (M+H)⁺ of the corresponding acid of compound **3c** (**3c**-K+H) calcd for C₉H₇N₂O₃: 191.05; found 190.99. HPLC retention time: 0.23 minutes (column A).

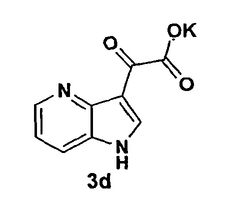

Compound **3d,** *Potassium (4-azaindol-3-yl)-oxoacetate:* **MS** *m*/*z:* (M+H)⁺ of the corresponding acid of compound **3d** (**3d**-K+H) calcd for C₉H₇N₂O₃: 191.05; found 190.87. HPLC retention time: 0.19 minutes (column A).

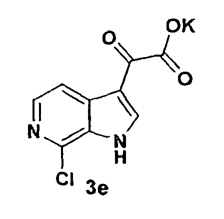

Compound **3e,** *Potassium (7-chloro-6-azaindol-3-yl)-oxoacetate:* MS *m*/*z:* (M+H)⁺ of the corresponding acid of compound **3e** (3e-K+H)⁺ calcd for C₉H₆ClN₂O₃: 225.01; found 224.99. HPLC retention time: 0.93 minutes (column A).

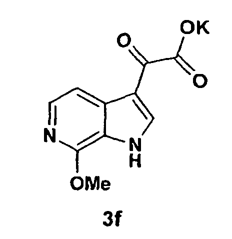

Compound **3f**, *Potassium (7-methoxy-6-azaindol-3-yl)-oxoacetate:* MS *m*/*z:* (M+H)⁺ of the corresponding acid of compound **3f (3f**-K+H)⁺ calcd for C₁₀H₉N₂O₄: 221.06; found 220.97. HPLC retention time: 0.45 minutes (column A).

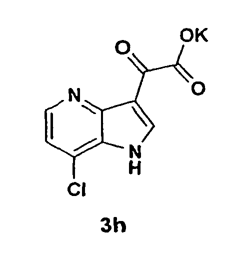

Compound **3h,** *Potassium (7-chloro-4-azaindol-3-yl)-oxoacetate:* MS *m*/*z:* (M+H)⁺ of the corresponding acid of compound **3h** (3h-K+H)⁺ calcd for C₉H₆ClN₂O₃: 225.01; found 225.27. HPLC retention time: 0.33 minutes (column A).

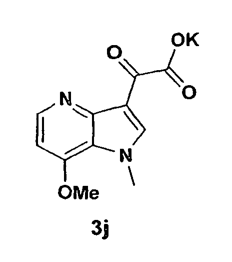

Compound **3j,** Potassium (7-methoxyl-1-methyl-4-azaindol-3-yl)-oxoacetate: MS *m*/*z*: (M+H)⁺ of the corresponding acid of compound **3j** (3j-K+H)⁺ calcd for C₁₁H₁₁N₂O₄: 235.07; found 235.01. HPLC retention time: 0.36 minutes (column A).

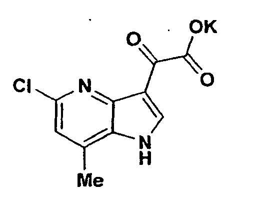

Compound **3ak,** *Potassium (5-chloro-7-methyl-4-azaindol-3-yl)-oxoacetate:* MS *m*/*z:* (M⁺H)⁺ of the corresponding acid of compound **3ak** (**3ak**-K+H)⁺ calcd for C₁₀H₈ClN₂O₃: 239.02; found 238.94. HPLC retention time: 1.24 minutes (column B).

### 1) Preparation of azaindole piperazine diamide 5

### Typical Procedure for the Preparation of Compounds in Scheme 3

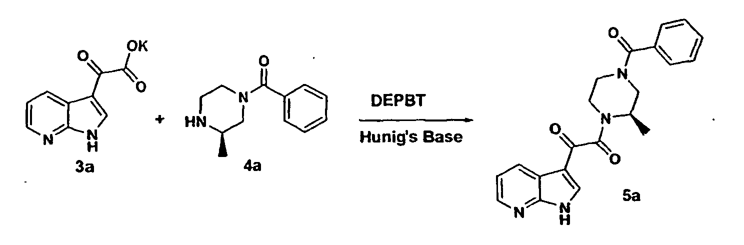

*Preparation* of *(R)-N-(benzoyl)-3-methyl-N'-[(7-azaindol-3-yl)-oxoacetyl]-piperazine* **5a:** Potassium 7-azaindole 3-glyoxylate **3a** (25.4 g, 0.111 mol), (R)-3-methyl-N-benzoylpiperazine **4a** (22.7 g, 0.111 mol), 3-(diethoxyphosphoryloxy)-1,2,3-benzotriazin-4(3*H*)-one (DEPBT) (33.3 g, 0.111 mol) and Hunig's Base (28.6 g, 0.222 mol) were combined in 500 ml of DMF. The mixture was stirred at room temperature for 8 hours.

DMF was removed via evaporation at reduced pressure and the residue was partitioned between ethyl acetate (2000 ml) and 5% Na₂CO₃ aqueous solution (2 x 400 ml). The aqueous layer was extracted with ethyl acetate (3 x 300 ml). The organic phase combined and dried over anhydrous MgSO₄. Concentration *in vacuo* provided a crude product, which was purified by silica gel column chromatography with EtOAc/MeOH (50:1) to give 33 g of product **5a** in 81% yield.

### Typical Procedure for the Preparation of Compounds in Scheme 4

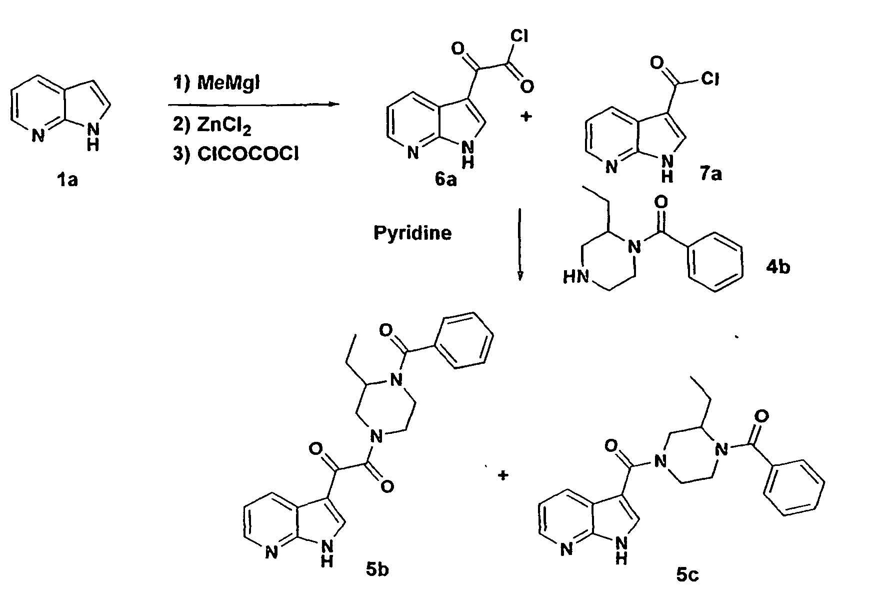

*Preparation of N-(benzoyl)-2-ethyl-N'-[(7-azaindol-3-yl)-oxoacetyl]-piperazine **5b** and N-(benzoyl)-2-ethyl-N'-[(7-azaindol-3-yl)-carbonyl]-piperazine **5c:*** To a solution of 7-azaindole **1a** (1.0 g, 8.5 mmol) in dry diethyl ether (20 ml), 3.1 ml of MeMgl (3.0M in Et₂O, 9.3 mmol) was added at room temperature. The resulting mixture was stirred at room temperature for 1 hour before ZnCl₂ (1M in ether, 10.2 ml, 10.2 mmol) was added. One hour later, oxalyl chloride (10.7 g, 85 mmol) was injected into the solution cautiously. After the reaction was stirred for 8 hours, solvent and excess oxayl chloride were removed under vaccum to give a residue containing a mixture of **6a** and **7a.**

After the residue was dissolved in dry CH₃CN (8 ml), mono-benzoylated piperazine **4b** (0.25 g, 1.15 mmol) and pyridine (1 g, 12.7 mmol) were added into the solution subsequently. 1 hour later, solvents were removed and residue was purified using Shimadzu automated preparative HPLC System to give compound **5b** (20 mg, 0.6%) and compound **5c** (16 mg, 0.5%).

Characterization of compounds **5** with the following sub-structure:

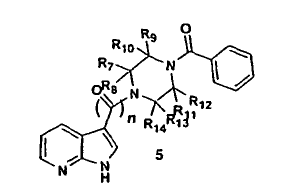

Compound **5a,** n = 2, R₇₋₁₃ = H, R₁₄ = (R)-Me, *(R)-N-(benzoyl)-3-methyl-N'-[(7-azaindol-3-yl)-oxoacetyl]-piperazine*: ¹H NMR (300 MHz, CD₃OD) δ 8.57 (d, 1H, J = 5.97 Hz), 8.38 (d, 1H, J = 4.20 Hz), 8.27 (m, 1H), 7.47 (s, 5H), 7.35 (t, 1H, J = 5.13 Hz), 4.75-2.87 (m, 7H), 1.31 (b, 3H); ¹³C NMR (75 MHz, CD₃OD) δ 185.6, 172.0, 166.3, 148.9, 144.6, 137.0, 134.8, 130.2, 129.9, 128.4, 126.6, 118.6, 118.0, 112.2, 61.3, 50.3, 45.1, 35.5, 14.9, 13.7. MS *m*/*z*: (M+H)⁺ calcd for C₂₁H₂₁N₄O₃: 377.16; found 377.18. HPLC retention time: 1.21 minutes (column A).

Compound **5ai,** n = 2, R₇₋₁₃ = H, R₁₄ = Me, *N-(benzoyl)-3-methyl-N'-[(7-azaindol-3-yl)-oxoacetyl]-piperazine:* MS *m*/*z*: (M+H)⁺ calcd for C₂₁H₂₁N₄O₃: 377.16; found 377.05.

Compound **5b,** n = 2, R₇₋₈ = R₁₀₋₁₄ = H, R₉ = Et, *N-(benzoyl)-2-ethyl-N'-[(7-azaindol-3-yl)-oxoacetyl]-piperazine:* ¹H NMR (500 MHz, CD₃OD) δ 8.63 (s, 1H), 8.40 (s, 1H), 8.25 (m, 1H), 7.42 (m, 6H), 4.70-2.90 (m, 7H), 1.80-0.60 (m, 5H); ¹³C NMR (125 MHz, CD₃OD) δ 186.8, 174.2, 168.3, 149.6, 145.4, 138.8, 136.9, 132.6, 131.3, 130.0, 128.0, 120.2, 117.7, 114.1, 58.4, 52.2, 47.5, 44.8, 23.0, 10.9, 10.7. MS *m*/*z:* (M+H)⁺ calcd for C₂₂H₂₃N₄O₃: 391.18; found 391.22. HPLC retention time: 1.35 minutes (column A).

Compound **5c**, n = 1, R₇₋₈ = R₁₀₋₁₄ = H, R₉ = Et, *N-(benzoyl)-2-ethyl-N'-[(7-azaindol-3-yl)-carbonyl]-piperazine:* ¹H NMR (500 MHz, CD₃OD) δ 8.33(m, 2H), 7.87 (s, 1H), 7.47 (m, 5H), 7.33 (m, 1H), 4.74-2.90 (m, 7H), 1.78-0.75 (m, 5H); ¹³C NMR (125 MHz, CD₃OD) δ 168.0, 164.2, 162.8, 147.0, 142.8, 136.9, 133.1, 132.8, 131.3, 130.4, 130.0, 128.0, 118.4, 110.3, 57.0, 53.4, 46.7, 24.0, 10.7. MS *m*/*z*: (M+H)⁺ calcd for C₂₁H₂₃N₄O₂: 363.18; found 363.22. HPLC retention time: 1.14 minutes (column A).

Compound **5d,** n = 2, R₇₋₁₄ = H, *N-(benzoyl)-N'-[(7-azaindol-3-yl)-oxoacetyl]-piperazine:* ¹H NMR (500 MHz, CD₃OD) δ 8.62 (s, 1H), 8.44 (s, 1H), 8.26 (s, 1H), 7.46 (s, 5H), 7.29 (m, 1 H), 3.97-3.31 (m, 8H). MS *m*/*z:* (M+H)⁺ calcd for C₂₀H₁₉N₄O₃: 363.15; found 363.24. HPLC retention time: 1.18 minutes (column A).

Compound **5e,** n = 2, R₇₋₈ = R₁₀₋₁₄ = H, R₉ = Me, *N-(benzoyl)-2-methyl-N'-[(7-azaindol-3-yl)-oxoacetyl]-piperazine:* ¹H NMR (500 MHz, CD₃OD) δ 8.64 (s, 1H), 8.51 (s, 1H), 8.28 (m, 1H), 7.42 (m, 6H), 4.48-2.90 (m, 7H), 1.26 (m, 3H); ¹³C NMR (125 MHz, CD₃OD) δ 185.3, 171.4, 166.8, 164.0, 147.9, 143.6, 137.3, 135.3, 131.2, 129.8, 128.4, 126.2, 118.6, 112.4, 49.4, 45.9, 45.6, 45.1, 40.8, 40.4, 14.1. MS *m*/*z:* (M+H)⁺ calcd for C₂₁H₂₁N₄O₃: 377.16; found 377.21. HPLC retention time: 1.26 minutes (column A).

Compound **5f,** n = 2, R₇₋₁₃ = H, R₁₄ = (*S*)-Me, *(S)-N-(benzoyl)-3-methyl-N'-[(7-azaindol-3-yl)-oxoacetyl]-piperazine:* ¹H NMR (500 MHz, CD₃OD) δ 8.64 (s, 1H), 8.39 (s, 1 H), 8.26 (m, 1H), 7.44 (m, 6H), 4.71-3.79 (m, 7H), 1.26 (m, 3H); ¹³C NMR (125 MHz, CD₃OD) δ 185.5, 171.9, 166.0, 158.4, 147.6, 143.5, 137.2, 134.8, 131.3, 129.8, 128.3, 126.6, 118.6, 112.4, 50.3, 45.1, 41.2, 40.3, 14.9, 13.7. MS *m*/*z*: (M+H)⁺ calcd for C₂₁H₂₁N₄O₃: 377.16; found 377.21. HPLC retention time: 1.25 minutes (column A).

Compound **5g**, n = 2, R₇₋₁₃= H, R₁₄ = Et, *N-(benzoyl)-3-ethyl-N'-[(7-azaindol-3-yl)-oxoacetyl]-piperazine:* ¹H NMR (500 MHz, CD₃OD) δ 8.65 (b, 1H), 8.40 (s, 1H), 8.27 (m, 1H), 7.46 (m, 6H), 4.73-3.00 (m, 7H), 1.80-0.58 (m, 5H); ¹³C NMR (125 MHz, CD₃OD) δ 187.1, 173.0, 168.0, 149.2, 145.0, 138.8, 136.4, 133.0, 131.4, 129.9, 128.2, 120.2, 114.1, 57.5, 46.0, 43.0, 37.5, 23.0, 10.7. MS *m*/*z:* (M+H)⁺ calcd for C₂₂H₂₃N₄O₃: 391.18; found 391.20. HPLC retention time: 1.33 minutes (column A).

Compound **5h,** n = 2, R₇₋₁₂ = H, R₁₃ = R₁₄ = Me, *N-(benzoyl)-3,3-dimethyl-N'-[(7-azaindol-3-yl)-oxoacetyl]-piperazine:* MS *m*/*z:* (M+H)⁺ calcd for C₂₂H₂₃N₄O₃: 391.18; found 390.98. HPLC retention time: 1.22 minutes (column A).

Compound **5i,** n = 2, R₇₋₈ = R₁₀₋₁₃ = H, R₉ = R₁₄ = Me, *trans-N-(benzoyl)-2,5-dimethyl-N'-[(7-azaindol-3-yl)-oxoacetyl]-piperazine:* ¹H NMR (500 MHz, CD₃OD) δ 8.58 (m, 1H), 8.37 (d, 1H, J = 15.7 Hz), 8.25 (m, 1H), 7.77 (m, 1H), 7.46 (m, 5H), 5.09-3.16 (m, 6H), 1.30 (m, 6H). MS *m*/*z*: (M+H)⁺ calcd for C₂₂H₂₃N₄O₃: 391.18; found 391.11. HPLC retention time: 1.22 minutes (column A).

Compound **5ab,** n = 2, R₇₋₉ = R₁₀₋₁₃ = H, R₁₄ = i-Pr, *N-(benzoyl)-3-iso-Propyl-N'-[(7-azaindol-3-yl)-oxoacetyl]-piperazine:* MS *m*/*z:* (M+H)⁺ calcd for C₂₃H₂₅N₄O₃: 405.19; found 405.22. HPLC retention time: 1.52 minutes (column A).

Compound **5ac,** n = 2, R₇₋₈= R₁₀₋₁₄ = H, R₉ = i-Pr, *N-(benzoyl)-2-isoPropyl-N'-[(7-azaindol-3-yl)-oxoacetyl]-piperazine:* MS *m*/*z*: (M+H)⁺ calcd for C₂₃H₂₅N₄O₃: 405.19; found 405.25. HPLC retention time: 1.53 minutes (column A).

Compound **5ad,** n = 1, R₇₋₈= R₁₀₋₁₄= H, R₉ = i-Pr, *N-(benzoyl)-2-isoPropyl-N'-[(7-azaindol-3-yl)-carbonyl]-piperazine:* MS *m*/*z:* (M+H)⁺ calcd for C₂₂H₂₅N₄O₂: 377.20; found 377.23. HPLC retention time: 1.34 minutes (column A).

Compound **5ae**, n = 2, R₇₋₈ = R₁₀₋₁₄ = H, R₉ = Pentyl, *trans-N-(benzoyl)-2-Pentyl-N'-[(7-azaindol-3-yi)-oxoacetyl]-piperazine:* MS *mlz:* (M+H)⁺ calcd for C₂₅H₂₉N₄O₃: 433.22; found 433.42. HPLC retention time: 1.74 minutes (column A).

Characterization of compounds **5** with the following sub-structure:

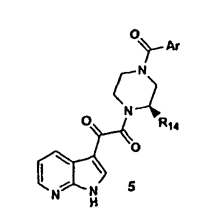

When

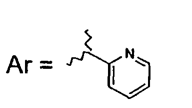

Compound **5j,** R₁₄ = H, *N-*(*pyridin-2-yl)-N-[(7-azaindol-3-yl)-oxoacetyl]-piperazine:* ¹H NMR (500 MHz, CD₃OD) δ 8.65-7.30 (m, 8H), 4.00-3.33 (m, 8H). MS *m*/*z*: (M+H)⁺ calcd for C₁₉H₁₈N₅O₃: 364.14; found 364.08. HPLC retention time: 0.97 minutes (column A).

Compound **5k,** R₁₄ = (*R*)-Me, (*R*)*-N-(pyridin-2-yl)-3-methyl-N'-*[(*7-azaindol-3-yl)-oxoacetyl]-piperazine:* ¹H NMR (300 MHz, CD₃OD) δ 8.67-7.38 (m, 8H), 4.76-3.00 (m, 7H), 1.35 (m, 3H); ¹³C NMR (75 MHz, CD₃OD) δ 186.0, 168.9, 166.6, 152.9, 148.5, 144.0, 138.7, 137.8, 131.8, 125.6, 124.0, 119.0, 112.9, 51.3, 50.9, 50.7, 46.7, 46.2, 45.7, 42.6, 42.0, 41.8, 40.8, 36.6, 35.7, 15.5, 14.2. MS *m*/*z*: (M⁺H)⁺ calcd for C₂₀H₂₀N₅O₃: 378.16; found 378.14. HPLC retention time: 1.02 minutes (column A).

When

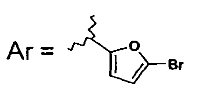

Compound **5l**, R₁₄ = (*R*)-Me, *(R)-N-(5-bromo-furan-2-yl)-3-methyl-N'-[(7-azaindol-3-yl)-oxoacetyl]-piperazine:* ¹H NMR (500 MHz, CD₃OD) δ 8.59 (d, 1H, *J* = 9.4 Hz), 8.37 (s, 1H), 8.26 (m, 1H), 7.34 (d, 1H, *J* = 10.1Hz), 7.06 (s, 1H), 6.59 (s, 1H), 4.56-3.16 (m, 7H), 1.30 (m, 3H); ¹³C NMR (125 MHz, CD₃OD) δ 187.2, 167.8, 161.0, 150.1, 149.8, 145.8, 138.7, 132.1, 127.0, 120.5, 120.2, 119.8, 114.8, 113.9, 51.8, 47.0, 42.0, 37.0, 16.6, 15.4. MS *m*/*z:* (M±H)⁺ calcd for C₁₉H₁₈BrN₄O₄: 445.05; found 445.18. HPLC retention time: 1.35 minutes (column A).

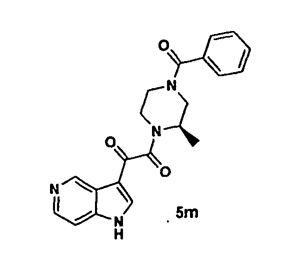

### Characterization of compound 5m:

Compound **5m,** *(R)-N-(benzoyl)-3-methyl-N'-(5-azaindol-3-yl)-oxoacetyl]-piperazine:* ¹H NMR (500 MHz, CD₃OD) δ 9.62 (b, 1 H), 8.72 (m, 1H), 8.61 (d, 1H, *J* = 4.5 Hz), 8.16 (d, 1H, *J* = 5.8 Hz), 7.51 (b, 6H), 4.90-3.10 (m, 7H), 1.35 (b, 3H). MS *m*/*z:* (M+H)⁺ calcd for C₂₁H₂₁N₄O₃ 377.16, found 377.15. HPLC retention time: 0.89 minutes (column A).

Characterization of compounds **5** with the following sub-structure:

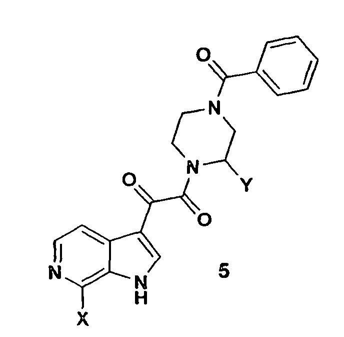

Compound **5p**, X = H, Y = H, *N-(benzoyl)-N'-f(6-azaindol-3-yl)-oxoacetyl]-piperazine:* MS *m*/*z:* (M+H)⁺ calcd for C₂₀H₁₉N₄O₃ 363.15, found 363.09. HPLC retention time: 0.96 minutes (column A).

Compound **5q**, X = H, Y = Me, *N-(benzoyl)-3-Methyl-N'-[(6-azaindol-3-yl)-oxoacetyl]-piperazine:* MS *m*/*z*: (M+H)⁺ calcd for C₂₁H₂₁N₄O₃ 377.16, found 377.11. HPLC retention time: 0.99 minutes (column A).

Compound **5r**, X = H, Y = (*R*)-Me, (*R*)*-N-(benzoyl)-3-Methyl-N'-[(6-azaindol-3-yl)-oxoacetyl]-piperazine:* MS *m*/*z:* (M+H)⁺ calcd for C₂₁H₂₁N₄O₃ 377.16, found 377.10. HPLC retention time: 0.99 minutes (column A).

Compound **5s**, X = H, Y = (*S*)-Me, *(S)-N-(benzoyl)-3-Methyl-N'-[(6-azaindol-3-yl)-oxoacetyl]-piperazine:* MS *m*/z: (M+H)⁺ calcd for C₂₁H₂₁N₄O₃ 377.16, found 377.10. HPLC retention time: 1.00 minutes (column A).

Compound **5t**, X = Cl, Y = H, *N-(benzoyl)-N'-[(7-Chloro-6-azaindol-3-yl)-oxoacetyl]-piperazine:* MS *m*/*z:* (M+H)⁺ calcd for C₂₀H₁₈ClN₄O₃ 397.11, found 397.26. HPLC retention time: 1.60 minutes (column B).

Compound **5u,** X = Cl, Y = (*R*)-Me, *(R)-N-(benzoyl)-3-Methyl-N'-[(7-Chloro-6-azaindol-3-yl)-oxoacetyl]-piperazine:* MS *m*/*z*: (M+H)⁺ calcd for C₂₁H₂₀ClN₄O₃ 411.12, found 411.16. HPLC retention time: 1.43 minutes (column A).

Compound **5v**, X = OMe, Y = *(R)*-Me*, (R)-N-(benzoyl)-3-Methyl-N'-[(7-Methoxy-6-azaindol-3-yl)-oxoacetyl]-piperazine:* MS *m*/*z*: (M+H)⁺ calcd for C₂₁H₂₀ClN₄O₃ 407.17, found 407.13. HPLC retention time: 1.31 minutes (column A).

Characterization of compounds **5** with the following sub-structure:

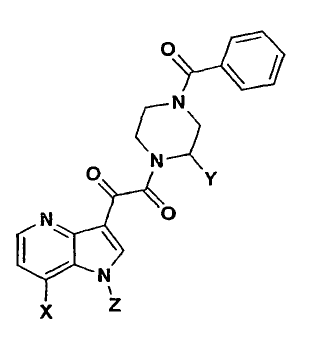

Compound **5w**, X = H, Y = (*R*)-Me, Z = H, *(R)-N-(benzoyl)-3-Methyl-N'-[(4-azaindol-3-yl)-oxoacetyl]-piperazine:* MS *m*/*z:* (M+H)⁺ calcd for C₂₁H₂₁N₄O₃ 377.16, found 377.14. HPLC retention time: 0.96 minutes (column A).

Compound **5x**, X = CH₃, Y = (*R*)-Me, Z = H, *(R)-N-(benzoyl)-3-Methyl-N'-[(7-Methyl-4-azaindol-3-yl)-oxoacetyl]-piperazine:* MS *m*/*z:* (M+H)⁺ calcd for C₂₁H₂₁N₄O₃ 391.18, found 391.15. HPLC retention time: 1.15 minutes (column A).

Compound **5y**, X = Cl, Y = (*R*)-Me, Z = H, *(R)-N-(benzoyl)-3-Methyl-N'-[(7-Chloro-4-azaindol-3-yl)-oxoacetyl]-piperazine:* MS *m*/*z:* (M+H)⁺ calcd for C₂₁H₂₀ClN₄O₃ 411.12, found 411.04. HPLC retention time: 1.10 minutes (column A).

Compound **5z,** X = OMe, Y = (*R*)-Me, Z = Me, *(R)-N-(benzoyl)-3-Methyl-N'-[(7-Methoxy-1-methyl-4-azaindol-3-yl)-oxoacetyl]-piperazine:* MS *m*/*z*: (M+H)⁺ calcd for C₂₃H₂₅N₄O₄: 421.19, found 421.05. HPLC retention time: 1.06 minutes (column A).

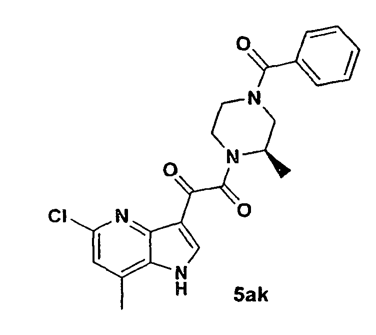

Compound **5ak,** *(R)-N-(benzoyl)-3-Methyl-N'-[(5-Chloro-7-methyl-4-azaindol-3-yl)-oxoacetyl]-piperazine:* MS *m*/*z:* (M+H)⁺ calcd for C₂₂H₂₂ClN₄O₃ 425.24, found 425.04. HPLC retention time: 1.72 minutes (column B).

### Typical Procedure for Preparation of Compounds in Scheme 5, 6 and 7

### 1) N-Oxide formation (equation 1, Scheme 5)

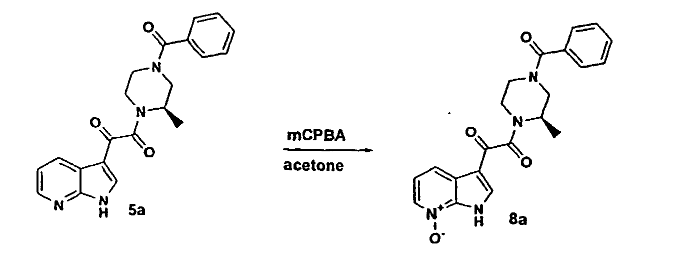

*Preparation of (R)-N-(benzoyl)-3-methyl-N'-[(7-oxide-7-azaindol-3-yl)-oxoacetyl]-piperazine **8a**:* 10 g of 7-azaindole piperazine diamide **5a** (26.6 mmol) was dissolved in 250 ml acetone. 9.17 g of mCPBA (53.1 mmol) was then added into the solution. Product **8a** precipitated out from the solution as a white solid after 8 hours and was collected via filtration. After drying under vacuum, 9.5 g of compound **8a** was obtained in 91% yield. No further purification was needed.

Characterization of compound **8** with he following sub-structure:

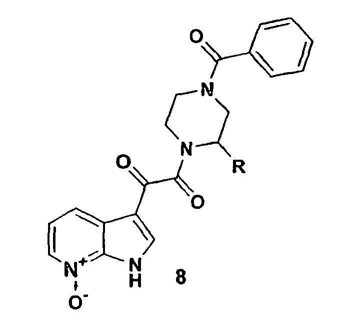

Compound **8a,** R = (*R*)-Me, *(R)-N-(benzoyl)-3-methyl-N'-[(7-oxide-7-azaindol-3-yl)-oxoacetyl]-piperazine:* ¹H NMR (300 MHz, DMSO-d₆) δ 8.30 (d, 1H, *J* = 12.2 Hz), 8.26 (d, 1H, *J* = 10.1 Hz), 8.00 (d, 1H, *J* = 7.41 Hz), 7.41 (s, 5H), 7.29 (m, 1H), 4.57-2.80 (m, 7H), 1.19 (b, 3H); ¹³C NMR (75 MHz, DMSO-d₆) δ 186.2, 170.0, 165.0, 139.5, 136.9, 136.7, 135.5, 133.5, 129.7, 128.5, 126.9, 121.6, 119.9, 113.6, 49.4, 44.3, 15.9, 14.8. MS *m*/*z*: (M+H)⁺ calcd for C₂₁H₂₁N₄O₄: 393.16; found 393.16. HPLC retention time: 1.05 minutes (column A).

Compound **8e**, R = H, *N-(benzoyl)-N'-[(7-oxide-7-azaindol-3-yl)-oxoacetyl]-piperazine:* MS *m*/*z*: (M+H)⁺ calcd for C₂₀H₁₉N₄O₄: 379.14; found 379.02. HPLC retention time: 1.15 minutes (column A).

Compound **8c**, R = (*S*)-Me, *(S)-N-(benzoyl)-3-methyl-N'-[(7-oxide-7-azaindol-3-yl)-oxoacetyl]-piperazine:* MS *m*/*z*: (M+H)⁺ calcd for C₂₁H₂₁N₄O₄: 393.16; found 393.05.

Compound **8d,** R = Me, *N-(benzoyl)-3-methyl-N-[(7-oxide-7-azaindol-3-yl)-oxoacetyl]-piperazine:* MS *m*/*z:* (M+H)⁺ calcd for C₂₁H₂₁N₄O₄: 393.16; found 393.05.

Characterization of compound **8b**:

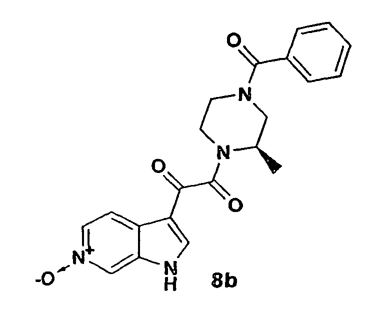

**Compound 8b,** *(R)-N-(benzoyl)-3-methyl-N'-[(6-oxide-6-azaindol-3-yl)-oxoacetyl]-piperazine:* MS *m*/*z:* (M+H)⁺ calcd for C₂₁H₂₁N₄O₄: 393.16; found 393.08. HPLC retention time: 1.06 minutes (column A).

### 2) Chlorination (equation 2, Scheme 5)

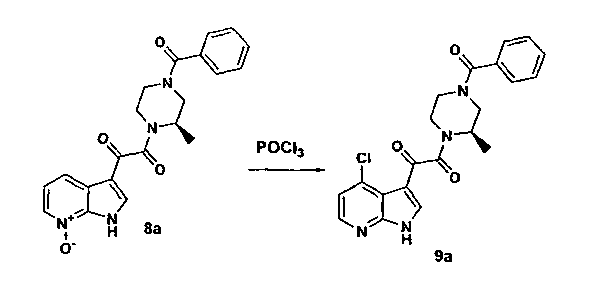

*Preparation of* (*R*)*-N-*(*benzoyl*)*-3-methyl N'-*[(*4-chloro-7-azaindol-3-yl)-oxoacetyl*]*-piperazine **9a:*** 55 mg of 7-azaindole piperazine diamide *N*-Oxide (0.14 mmol) **8a** was dissolved in 5 ml of POCl₃. The reaction mixture was heated at 60°C for 4 hours. After cooling, the mixture was poured into ice cooled saturated NaHCO₃ solution and the aqueous phase was extracted with EtOAc (3 x 50 ml). The combined organic layer was dried over MgSO₄ and concentrated under vacuum. The crude product was purified using a Shimadzu automated preparative HPLC System to give compound **9a** (15 mg, 26%).

### Characterization of compound 9a:

Compound **9a**, *(R)-N-(benzoyl)-3-methyl-N'-((4-chloro-7-azaindol-3-yl)-oxoacetyl]-piperazine:* ¹H NMR (500 MHz, DMSO-d₆) δ 13.27 (b, 1 H), 8.46 (m, 2H), 7.43 (m, 6H), 5.00-2.80 (m, 7H), 1.23 (b, 3H). MS m/z: (M+H)⁺ calcd for C₂₁H₂ClN₄O₃: 411.12; found 411.09. HPLC retention time: 1.32 minutes (column A).

### 3) Nitration of N-Oxide (equation 10, Scheme 6)

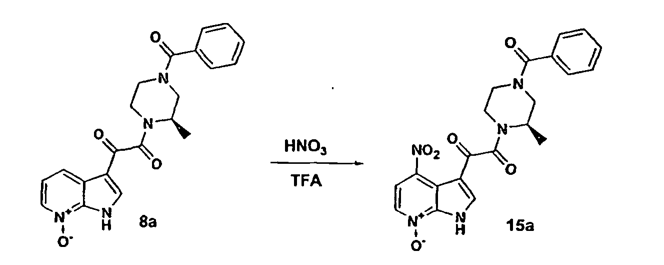

*Preparation of (R)-N-(benzoyl)-3-methyl-N'-(4-nitro-7-oxide-7-azaindol-3-yl)-oxoacetyl]-piperazine **15a**: N*-oxide **8a** (10.8 g, 27.6 mmol) was dissolved in 200 ml of trifluoroacetic acid and 20 ml of fuming nitric acid. The reaction mixture was stirred for 8 hours and quenched with methanol. After filtration, the filtrate was concentrated under vacuum to give crude product **15a** as a brown solid, which was carried to the next step without further purification. A small amount of crude product was purified using a Shimadzu automated preparative HPLC System to give compound 3 mg of compound **15a**.

Characterization of compound 15 with the following sub-structure:

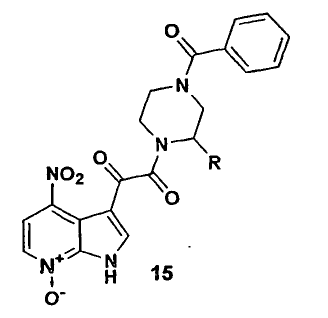

Compound **15a**, R = *(R)-Me, (R)-N-(benzoyl)-3-methyl-N-[(4-nitro-7-oxide-7-azaindol-3-yl)-oxoacetyl]-piperazine*: MS *m*/*z*: (M+H)⁺ calcd for C₂₁H₂₀N₅O₆: 438.14; found 438.07. HPLC retention time: 1.18 minutes (column A).

Compound **15b**, R = *(S)*-Me, *(S)-N-(benzoyl)-3-methyl-N-[(4-nitro-7-oxide-7-azaindol-3-yl)-oxoacetyl]-piperazine*: MS *m*/*z*: (M+H)⁺ calcd for C₂₁H₂₀N₅O₆: 438.14; found 438.02. HPLC retention time: 1.18 minutes (column A).

Compound **15c**, R = Me, *N-(benzoyl)-3-methyl-N-[(4-nitro-7-oxide-7-azaindol-3-yl)-oxoacetyl]-piperazine*: MS *m*/*z:* (M+H)⁺ calcd for C₂₁H₂₀N₅O₆: 438.14; found 438.02. HPLC retention time: 1.18 minutes (column A).

### 4) Fluorination (equation 5, Scheme 3)

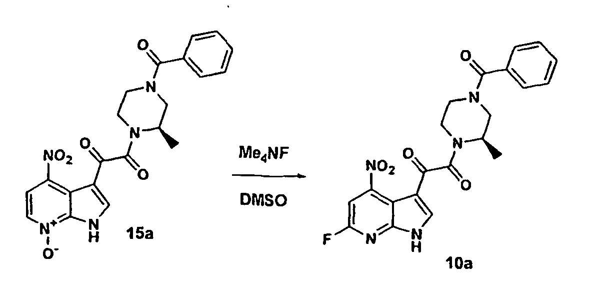

*Preparation of (R)-N-(benzoyl)-3-methyl N'[(4-nitro-6-fluoro-7-azaindol-3-yl)-oxoacetyl]-piperazine **10a:*** 20 mg of crude 4-nitro-7-azaindole piperazine diamide *N*-oxide **15a** and an excess of Me₄NF (300 mg) were dissolved in 5 ml of DMSO-d₆. The reaction mixture was heated at 100°C for 8 hours. After cooling, DMSO-d₆ was removed by blowing nitrogen. The residue was partitioned between ethyl acetate (10 ml) and 2N NaOH solution (10 ml). The aqueous phase was extracted with EtOAc (2 x 10 ml). The organic layers were combined and concentrated under vacuum to give a residue, which was further purified using a Shimadzu automated preparative HPLC System to give compound of **10a** (8.3 mg).

### Characterization of compound 10a:

Compound **10a:** (*R*)*-N-(benzoyl)-3-methyl-N'-[(4-nitro-6-fluoro-7-azaindol-3-yl)-oxoacetyl]-piperazine:* ¹H NMR (300 MHz, acetone-d₆) δ 8.44 (d, 1H, *J* = 8.24 Hz), 7.47 (s, 6H), 4.80-3.00 (m, 7H), 1.29 (b, 3H). MS *m*/*z*: (M+H)⁺ calcd for C₂₁H₁₉FN₅O₅: 440.14; found 440.14. HPLC retention time: 1.40 minutes (column B).

### 5) Alkylation and Arylation (equation 4, Scheme 5)

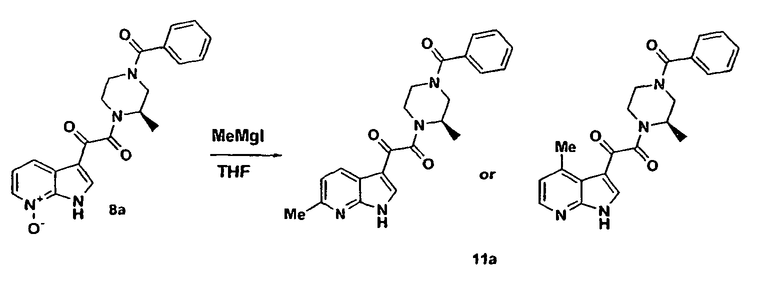

*Preparation of (R)-N-(benzoyl)-3-methyl-N'-[(4 or 6)-methyl-7-azaindol-3-yl)-oxoacetyl]-piperazine **11a:*** An excess of MeMgl (3M in THF, 0.21 ml, 0.63 mmol) was added into a solution of 7-azaindole piperazine diamide *N*-oxide **8a** (25 mg, 0.064 mmol). The reaction mixture was stirred at room temperature and then quenched with methanol. The solvents were removed under vacuum, the residue was diluted with methanol and purified using a Shimadzu automated preparative HPLC System to give compound **11a** (6.7 mg, 27%).

Characterization of compounds **11** with the following sub-structure:

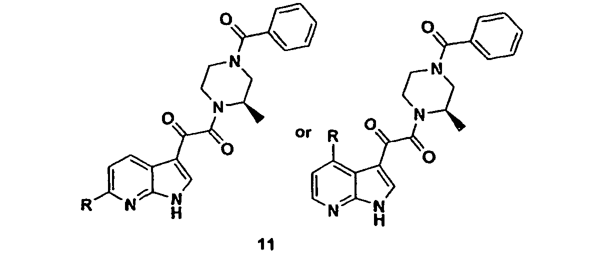

Compound **11a**: R = Me, *(R)-N-(benzoyl)-3-methyl-N'-[(4 or 6)-methyl-7-azaindol-3-yl)-oxoacetyl]-piperazine*: MS m/z: (M+H)⁺ calcd for C₂₂H₂₃N₄O₃: 391.18; found 391.17. HPLC retention time: 1.35 minutes (column B).

Compound **11b**: R = Ph, *(R)-N-(benzoyl)-3-methyl-N'-[(4 or 6)-phenyl-7-azaindol-3-yl)-oxoacetyl]-piperazine*: MS *m*/*z*: (M+H)⁺ calcd for C₂₇H₂₅N₄O₃: 453.19; found 454.20. HPLC retention time: 1.46 minutes (column B).

Compound **11c**, R = CH=CH2, *(R)-N-(benzoyl)-3-methyl-N'-[(4 or 6)-vinyl-7-azaindol-3-yl)-oxoacetyl]-piperazine*: MS *m*/*z*: (M+Na)⁺ calcd for C₂₃H₂₂N₄NaO₃: 425.16; found 425.23. HPLC retention time: 1.12 minutes (column A).

### 6) Nitrile Substitution and Chlorination (equation 5, Scheme 5)

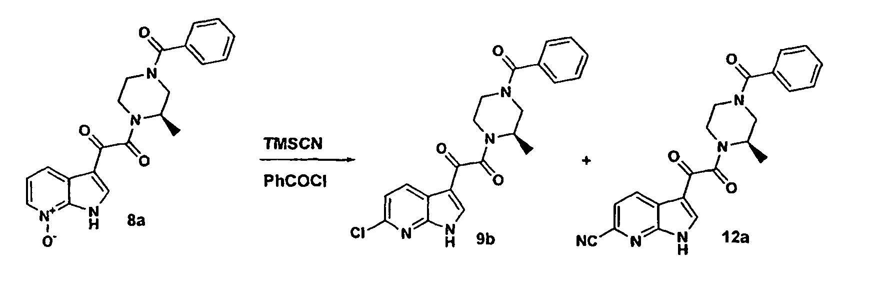

*Preparation of (R)-N-(benzoyl)-3-methyl-N'-[(6-chloro-7-azaindol-3-yl)-oxoacetyl]-piperazine* **9b** *and (R)-N-(benzoyl)-3-methyl-N'-[(6-cyano-7-azaindol-3-yl)-oxoacetyl]-piperazine 12a: N*-oxide **8a** (0.20 g, 0.51 mmol) was suspended in 20 ml of dry THF, to which TMSCN (0.3 g, 3.0 mmol) and BzCl (0.28 g, 2.0 mmol) were added. The reaction mixture was stirred at room temperature for 2 hours, and then heated at reflux for 5 hours. After cooling, the mixture was poured into 100 ml of saturated NaHCO₃ and the aqueous phase extracted with EtOAc (3 x 50 ml). The organic phase was combined and concentrated under vacuum to give a residue, which was diluted with methanol and purified using a Shimadzu automated preparative HPLC System to give compound **12a** (42 mg, 20%) and compound **9b** (23 mg, 11%).

### Characterization of compounds 9b and 12a:

Compound **9b,** *(R)-N-(benzoyl)-3-methyl-N'-[(6-chloro-7-azaindol-3-yl)-oxoacetyl]-piperazine:* ¹H NMR (500 MHz, DMSO-d₆) δ 8.39 (m, 2H), 7.42 (m, 6H), 5.00-2.80 (m, 7H), 1.19 (b, 3H); ¹³C NMR (125 MHz, DMSO-d₆) δ 185.8, 170.0, 165.1, 147.9, 145.1, 137.4, 135.4, 132.2, 129.5, 128.3, 126.8, 118.6, 116.1, 111.8, 49.3, 47.2, 44.2, 15.6, 14.5. MS *m*/*z*: (M+H)⁺ calcd for C₂₁H₂₀ClN₄O₃: 411.12; found 411.09. HPLC retention time: 1.43 minutes (column A).

Compound **12a,** *(R)-N-(benzoyl)-3-methyl-N'-[(6-cyano-7-azaindol-3-yl)-oxoacetyl]-piperazine:* ¹H NMR (500 MHz, DMSO-d₆) δ 8.67 (m, 2H), 7.86 (s, 1H), 7.42 (m, 5H), 4.80-2.80 (m, 7H), 1.22 (b, 3H); ¹³C NMR (125 MHz, DMSO-d₆) δ 185.7, 170.0, 164.8, 148.5, 140.9, 135.3, 130.3, 129.5, 128.3, 126.8, 126.2, 123.0, 120.4, 118.0, 111.8, 49.4, 47.3, 44.2, 15.6, 14.5. MS *m*/*z*: (M+H)⁺ calcd for C₂₂H₂₀N₅O₃: 402.16; found 402.13. HPLC retention time: 1.29 minutes (column A).

### 7) Hydroxylation (equation 6, Scheme 5)

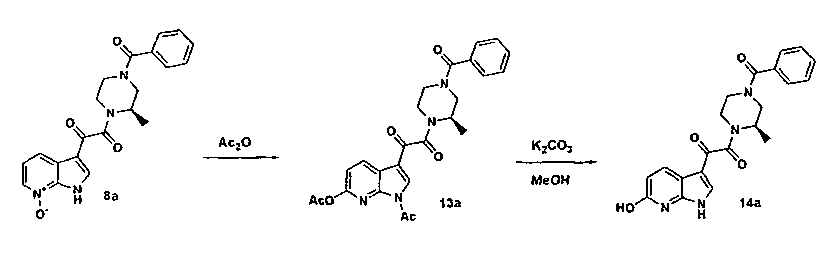

*Preparation of (R)-N-(benzoyl)-3-methyl-N'-[(1-acetyl-6-acetoxy-7-azaindol-3-yl)-oxoacetyl]-piperazine **13a***: 20 mg of 7-azaindole piperazine diamide N-oxide **8a** was dissolved in 5 ml of acetic anhydride (Ac₂O). The reaction mixture was heated at reflux for 8 hours. After cooling, the solvents were removed under vacuum to give product **13a**, which was pure enough for further reactions.

### Characterization of compound 13a:

Compound **13a,** *(R)-N-(benzoyl)-3-methyl-N-[(1-acetyl-6-acetoxy-7-azaindol-3-yl)-oxoacetyl)-piperazine*: ¹H NMR (300 MHz, acetone-d₆) δ 8.67 (m, 2H), 7.47 (s, 5H), 7.27 (d, 1H, *J* = 8.34 Hz), 4.90-2.80 (m, 7H), 2.09 (s, 6H), 1.30 (b, 3H); ¹³C NMR (75 MHz, acetone-d₆) δ 187.0, 170.8, 169.0, 168.6, 164.9, 155.3, 136.5, 134.7, 134.2, 133.2, 130.0, 129.8, 127.5, 118.9, 115.4, 113.8, 50.3, 45.4, 41.3, 36.3, 25.5, 20.5, 16.0, 14.8. MS *m*/*z:* (M+Na)⁺ calcd for C₂₅H₂₄N₄O₆Na: 499.16; found 499.15. HPLC retention time: 1.46 minutes (column B).

*Preparation of (R)-N-(benzoyl)-3-methyl-N'-[(6-hydroxyl-7-azaindol-3-yl)-oxoacetyl]-piperazine 14a:* The crude compound **13a** and an excess of K₂CO₃ (100 mg) were mixed in MeOH and H₂O (1:1). The reaction mixture was stirred for 8 hours. The MeOH was removed under vacuum, the aqueous phase extracted with EtOAc (3 x 10ml) and the organic layers combined and concentrated. The crude product was purified using a Shimadzu automated preparative HPLC System to give compound 1 mg of **14a** (5% from compound **8a**).

### Characterization of compound 14a:

Compound **14a,** *(R)-N-(benzoyl)-3-methyl-N'-[(6-hydroxyl-7-azaindol-3-yl)-oxoacetyl]-piperazine:* MS *mlz:* (M+H)⁺ calcd for C₂₁H₂₁N₄O₄: 393.16; found 393.12. HPLC retention time: 1.13 minutes (column A).

### 8) Thiol formation (equation 7, Scheme 5)

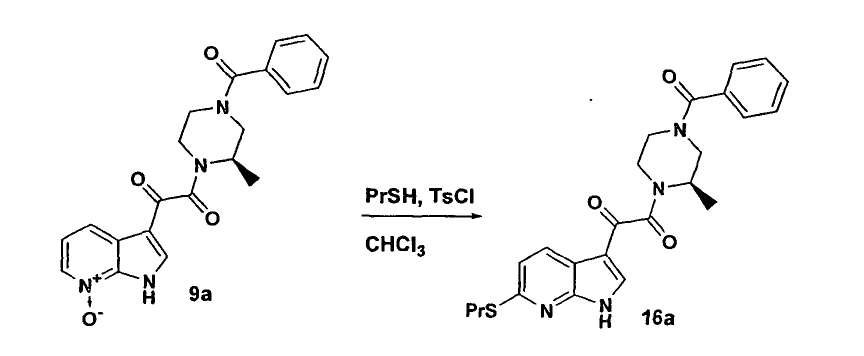

*Preparation of (R)-N-(benzoyl)-3-methyl-N'-[(6-propylthio-7-azaindol-3-yl)-oxoacetyl]-piperazine **17f***. To an solution of 100 mg of compound **9a** in 10 ml of CHCl₃ was added TsCl (63 mg), and the solution was stirred for 5 minutes. Then, 2 ml of propylthiol was added and the reaction mixture was stirred for 8 hours. After concentration, the crude product was purified using a Shimadzu automated preparative HPLC System to give compound 1.4 mg of **17f**.

### Characterization of compound 17f:

Compound **17f,** *(R)-N-(benzoyl)-3-methyl-N'[(6-propylthiol-7-azaindol-3-yl)-oxoacetyl]-piperazine:* MS *m*/*z:* (M+H)⁺ calcd for C₂₄H₂₇N₄O₃S: 451.18; found 451.09. HPLC retention time: 1.45 minutes (column A).

### 9) Displacement of Nitro Group (equation 11, Scheme 6)

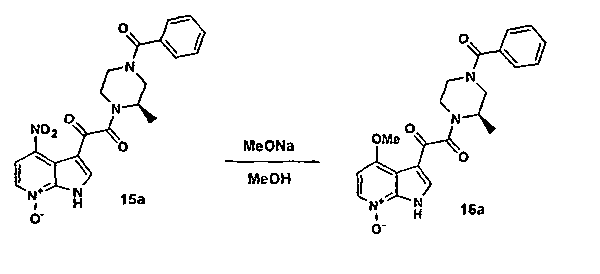

*Preparation of (R)-N-(benzoyi)-3-methyl-N'-[(4-methoxy-7-oxide-7-azaindol-3-yl)-oxoacetyl]-piperazine **16a:*** 100 mg of crude compound **15a** from the previous step was dissolved in 6 ml of 0.5M MeONa in MeOH. The reaction mixture was refluxed for 8 hours, and the solvent removed under vacuum to afford a mixture including product **16a** and other inorganic salts. This mixture was used in the next step without further purification. A small portion of the crude mixture was purified using a Shimadzu automated preparative HPLC System to give 5 mg of compound **16a.**

Characterization of compounds **16** with the following sub-structure:

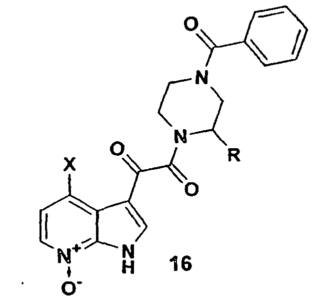

Compound **16a,** X = OMe, R = (*R*)-Me, *(R)-N-(benzoyl)-3-methyl-N'-[(4-methoxy-7-oxide-7-azaindol-3 yl)-oxoacetyl]-piperazine:* MS *m*/*z:* (M+H)⁺ calcd for C₂₂H₂₃N₄O₅ 423.17, found 423.04. HPLC retention time: 0.97 minutes (column A).

Compound **16f,** X = OMe, R = (*S*)-Me, *(S)-N-(benzoyl)-3-methyl-N'-[(4-methoxy-7-oxide-7-azaindol-3-yl)-oxoacetyl]-piperazine:* MS *m*/*z:* (M+H)⁺ calcd for C₂₂H₂₃N₄O₅ 423.17, found 423.02.

Compound **16g,** X = OMe, R = Me, *N-(benzoyl)-3-methyl-N'-[(4-methoxy-7-oxide-7-azaindol-3-yl)-oxoacetyl]-piperazine:* MS *m*/*z:* (M+H)⁺ calcd for C₂₂H₂₃N₄O₅ 423.17, found 423.03.

Compound **16b,** X = OCH₂CF₃, R = (*R*)-Me, *(R)-N-(benzoyl)-3-methyl-N'-[(4-(2,2,2-trifluoroethoxy)-7-oxide-7-azaindol-3-yl)-oxoacethyl]-piperazine:* ¹H NMR (500 MHz, CD₃OD) δ 8.44 (b, 1H), 8.30 (m, 1H), 7.50 (b, 5H), 7.14 (b, 1H), 4.90-3.10 (m, 9H), 1.30 (m, 3H). MS *m*/*z:* (M+H)⁺ calcd for C₁₃H₂₂F₃N₄O₅: 491.15; found 491.16. HPLC retention time: 1.17 minutes (column A).

Compound **16c**, X = OCH(CH₃)₂, R = (*R*)-Me, *(R)-N-(benzoyl)-3-methyl-N'-[(4-(1-methylethoxy)-7-oxide-7-azaindol-3-yl)-oxoacetyl]-piperazine:* ¹H NMR (500 MHz, CD₃OD) δ 8.48 (s, 1H), 8.24 (m, 1H), 7.46 (m, 5H), 7.13 (s, 1H), 5.03-3.00 (m, 8H), 1.49-1.15 (m, 9H). MS *m*/*z:* (M+H)⁺ calcd for C₂₄H₂₇N₄O₅: 451.20; found 451.21. HPLC retention time: 1.14 minutes (column A).

Compound **16d,** X = OCH₂CH₃, R = *(R)*-Me, *(R)-N-(benzoyl)-3-methyl-N'-[(4-ethoxy-7-oxide-7-azaindol-3 yl)-oxoacetyl]-piperazine:* MS *m*/*z:* (M+H)⁺ calcd for C₂₃H₂₅N₄O₅: 437.18; found 437.13. HPLC retention time: 1.08 minutes (column A).

Compound **16e** X = SCH₂CH₂CH₃, R = (*R*)-Me, *(R)-N-(benzoyl)-3-methyl-N'-[(4-propylthio-7-oxide-7-azaindol3-yl)-oxoacetyl]-piperazine:* ¹H NMR (500 MHz, CD₃OD) δ 8.24 (m, 2H), 7.45 (m, 5H), 7.25 (s, 1H), 4.90-3.00 (m, 9H), 1.81 (b, 2H), 1.30 (m, 6H). MS *m*/*z:* (M+H)⁺ calcd for C₂₄H₂₇N₄O₄S: 467.18; found 467.14. HPLC retention time: 1.30 minutes (column A).

Compound **16h,** X = NHMe, R = *(R)*-Me, *(R)-N-(benzoyl)-3-methyl-N'-[(4-methylamino-7-oxide-7-azaindol-3-yl)-oxoacetyl]-piperazine:* MS *m*/*z*: (M+H)⁺ calcd for C₂₂H₂₄N₅O₄: 422.18; found 422.09. HPLC retention time: 1.19 minutes (column A).

### 10) Reduction of N-Oxide (equation 12, Scheme 6)

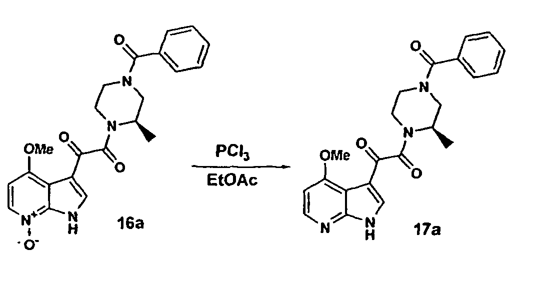

*Preparation* of *(R)-N-(benzoyl)-3-methyl-N'-[(4-methoxy-7-azaindol-3-yl)-oxoacetyl]-piperazine **17a:*** 48 mg of crude **16a** was suspended in 30 ml of ethyl acetate at room temperature. 1 ml of PCl₃ was added and the reaction was mixture stirred for 8 hours. The reaction mixture was poured into ice cooled 2N NaOH solution with caution. After separating the organic layer, the aqueous phase was extracted with EtOAc (6 x 80 ml). The organic layers were combined, and concentrated *in vacuo* to give a residue which was purified using a Shimadzu automated preparative HPLC System to give 38 mg of compound **17a.**

Characterization of compounds **17** with the following sub-structure:

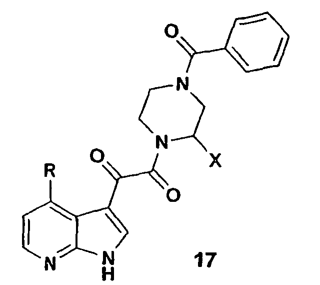

Compound **17a,** R = Ome, X = (R)-Me, (R)*-N-(benzoyl)-3-methyl-N'-[(4-methoxy-7-azaindol-3-yl)-oxoacetyl]-piperazine:* ¹H NMR (300 MHz, CD₃OD) δ 8.24 (d, 1H, *J =* 5.7 Hz), 8.21 (m, 1H), 7.47 (s, 5H), 6.90 (d, 1H, *J* = 5.7 Hz), 4.71-3.13 (m, 10H), 1.26 (b, 3H); ¹³C NMR (75 MHz, CD₃OD) δ 185.3, 172.0, 167.2, 161.2, 150.7, 146.6, 135.5, 134.8, 129.9, 128.3, 126.7, 112.8, 106.9, 100.6, 54.9, 50.2, 48.1, 45.1, 14.5, 13.8. MS *m*/*z:* (M⁺H)⁺ calcd for C₂₂H₂₃N₄O₄: 407.17; found 407.19. HPLC retention time: 1.00 minutes (column A).

Compound **17d,** R = Ome, X = (*S*)-Me, *(S)-N-(benzoyl)-3-methyl-N'-[(4-methoxy-7-azaindol-3-yl)-oxoacetyl]-piperazine:* MS *m*/*z*: (M+H)⁺ calcd for C₂₂H₂₃N₄O₄: 407.17; found 407.03.

Compound **17e,** R = Ome, X = Me, *N-(benzoyl)-3-methyl-N'-[(4-methoxy-7-azaindol-3-yl)-oxoacetyl]-piperazine:* MS *m*/*z*: (M+H)⁺ calcd for C₂₂H₂₃N₄O₄: 407.17; found 407.03.

Compound **17b,** R = OCH₂CF₃, X = (R)-Me, *(R)-N-(benzoyl)-3-methyl-N'-[(4-(2,2,2-trifluoroethoxy)-7-azaindol-3-yl)-oxoacetyl]-piperazine:* ¹H NMR (500 MHz, CD₃OD) δ 8.33 (s, 1H), 8.19 (m, 1H), 7.45 (m, 5H), 7.05 (s, 1H), 4.90-3.00 (m, 9H), 1.29 (b, 3H); ¹³C NMR (125 MHz, CD₃OD) δ 185.7, 174.0, 168.3, 162.0, 151.0, 146.1, 138.5, 136.4, 131.4, 130.0, 128.2, 114.8, 109.5, 103.6, 67.2, 66.9, 52.0, 47.0, 16.4, 15.3. MS *m*/*z*: (M+H)⁺ calcd for C₂₃H₂₂F₃N₄O₄: 475.16; found 475.23. HPLC retention time: 1.22 minutes (column A).

Compound **17c,** R = OCH(CH₃)₂, X = (R)-Me, *(R)-N-(benzoyl)-3-methyl-N'-[(4-(1-methylethoxy)-7-azaindol-3-yl)-oxoacetyl]-piperazine:* ¹H NMR (500 MHz, CD₃OD) δ 8.42 (s, 1H), 8.24 (m, 1H), 7.47 (m, 5H), 7.21 (s, 1H), 5.20-3.00 (m, 8H), 1.51 (b, 6H), 1.22 (b, 3H); ¹³C NMR (125 MHz, CD₃OD) δ 185.4, 173.6, 167.9, 166.1, 145.3, 141.4, 138.2, 136.4, 131.5, 129.7, 128.2, 113.9, 111.4, 104.0, 75.5. 54.4, 53.7, 51.8, 46.9, 22.1, 16.4, 15.3. MS *m*/*z*: (M+H)⁺ calcd for C₂₄H₂₇N₄O₄: 435.20; found 435.20. HPLC retention time: 1.15 minutes (column A).

Compound **17m,** R = OCH₂CH₃, X = (R)-Me, *(R)-N-(benzoyl)-3-methyl-N'-[(4-ethoxy-7-azaindol-3-yl)-oxoacetyl]-piperazine:* MS *m*/*z*: (M+H)⁺ calcd for C₂₃H₂₅N₄O₄: 421.19; found 421.13. HPLC retention time: 1.13 minutes (column A).

Compound **17g**, R = SCH₂CH₂CH₃, X = (R)-Me, *(R)-N-(benzoyl)-3-methyl-N'-[(4-propylthio-7-azaindol-3-yl)-oxoacetyl]-piperazine:* MS *m*/*z:* (M+H)⁺ calcd for C₂₄H₂₇N₄O₄S: 451.18; found 451.13. HPLC retention time: 1.50 minutes (column A).

Compound **17h**, R = NHMe, X = (R)-Me, *(R)-N-(benzoyl)-3-methyl-N'-[(4-methylamino-7-azaindol-3-yl)-oxoacetyl]-piperazine:* MS *m*/*z*: (M+H)⁺ calcd for C₂₂H₂₄N₅O₃: 406.19; found 406.03. HPLC retention time: 1.19 minutes (column A).

Characterization of compound **18a**

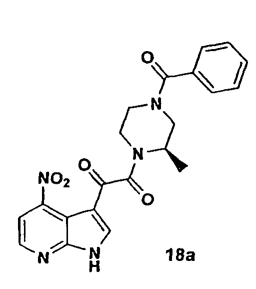

Compound **18a**, *(R)-N-(benzoyl)-3-methyl-N'-[(4-nitro-7-azaindol-3-yl)-oxoacetyl]-piperazine:* ¹H NMR (300 MHz, CD₃OD) δ 8.58 (s, 1H), 8.53 (m, 1H), 7.64 (s, 1H), 7.47 (s, 5H), 4.90-3.00 (m, 7H), 1.30 (b, 3H); ¹³C NMR (75 MHz, CD₃OD) δ 184.1, 172.1, 165.6, 151.9, 149.6, 145.5, 139.4, 134.8, 129.7, 128.4, 126.7, 111.6, 111.2, 107.4, 53.7, 48.4, 45.9, 15.0, 13.7. MS *m*/*z*: (M+H)⁺ calcd for C₂₁H₂₀N₅O₅: 422.15; found 422.09. HPLC retention time: 1.49 minutes (column B).

### 11) Reduction of Nitro to Hydoxylamine Group (equation 14, Scheme 6)

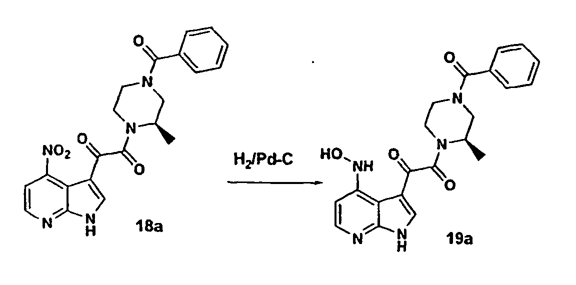

*Preparation of* (*R*)*-N-*(*benzoyl*)*-3-methyl-N'-*[(*4-hydroxylamino-7-azaindol-3-yl*)*-oxoacetyl*]*-piperazine **19a:*** 10 mg of Pd (10% on activated carbon) was added to a solution of compound **18a** (48 mg, 0.11 mmol) in methanol (10 ml) under an atmosphere of hydrogen. The reaction mixture was stirred for 8 hours at room temperature. After filtration, the filtrate was concentrated *in vacuo* to give a residue which was purified using a Shimadzu automated preparative HPLC System to give compound **19a** (7.9 mg, 17%).

### Characterization of compound 19a:

Compound **19a,** *(R)-N-(benzoyl)-3-methyl-N'-[(4-hydroxylamino-7-azaindol-3-yl)-oxoacetyl]-piperazine: MS m*/*z*: (M+H)⁺ calcd for C₂₁H₂₂N₅O₄: 408.17; found 408.21. HPLC retention time: 1.03 minutes (column A).

### 12) Reduction of Nitro to Amine Group (equation 15, Scheme 6)

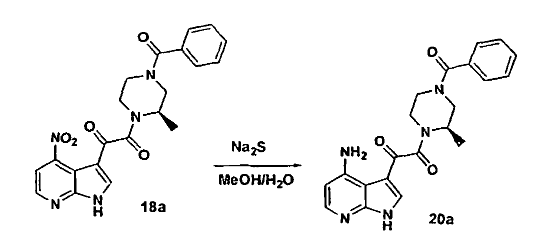

*Preparation of (R)-N-(benzoyl)-3-methyl-N'-((4-amino-7-azaindol-3-yl)-oxoacetyl]-piperazine **20a***: 114 mg of Na₂S.2H₂O (1 mmol) was added to a solution of compound **18a** (20 mg, 0.048mmol) in MeOH (5 ml) and H₂O (5 ml). The reaction mixture was heated at reflux for 8 hours. After cooling, the reaction mixture was concentrated *in vacuo* to give a residue which was purified using a Shimadzu automated preparative HPLC System to give 4 mg of compound **20a** (21.3%).

### Characterization of compound 20a:

Compound **20a,** *(R)-N-(benzoyl)-3-methyl-N'-[(4-amino-7-azaindol-3-yl)-oxoacetyl]-piperazine:* ¹H NMR (500 MHz, CD₃OD) δ 8.16 (m, 1H), 8.01 (d, 1H, *J* = 8.1 Hz), 7.47 (m, 5H), 6.66 (s, 1H), 4.90-3.00 (m, 7H), 1.30 (b, 3H). MS *m*/*z*: (M+H)⁺ calcd for C₂₁H₂₂N₅O₃: 392.17; found 392.14. HPLC retention time: 0.96 minutes (column A).

### 13) Alkylation of the nitrogen atom at position 1 (equation 16, Scheme 7)

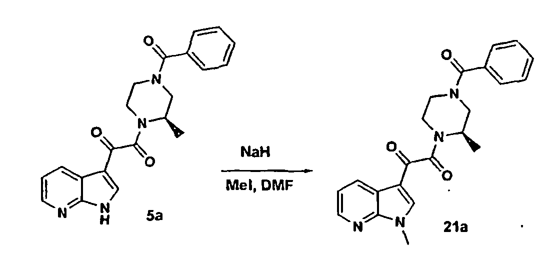

*Preparation of (R)-N-(benzoyl)-3-methyl-N-[(1-methyl-7-azaindol-3-yl)-oxoacetyl]-piperazine **21a***: NaH (2 mg, 60% pure, 0.05 mmol) was added to a solution of compound **5a** (10 mg, 0.027 mmol) in DMF. After 30 minutes, Mel (5 mg, 0.035 mmol) was injected into the mixture via syringe. The reaction mixture was stirred for 8 hours at room temperature and quenched with methanol. The mixture was partitioned between ethyl acetate (2 ml) and H₂O (2 ml). The aqueous phase was extracted with EtOAc (3 x 2 ml). The organic layers were combined, dried over anhydrous MgSO₄ and concentrated *in vacuo* to give a crude product which was purified using a Shimadzu automated preparative HPLC System to give compound **21a** (2.5 mg, 24%).

Characterization of compound **21** with the following sub-structure:

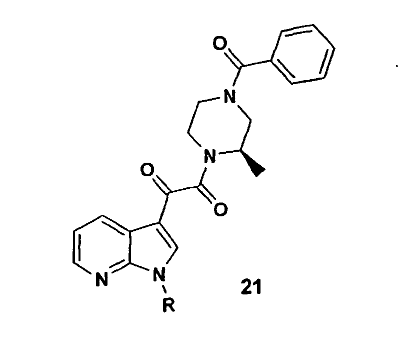

Compound **21a**, R = Me, *(R)-N-(benzoyl)-3-methyl-N'-[(9-methyl-7-azaindol-3-yl)-oxoacetyl]-piperazine:* ¹H NMR (500 MHz, CD₃OD) δ 8.56 (b, 1H), 8.42 (s, 1H), 8.30 (m, 1H), 7.47 (m, 6H), 4.90-3.00 (m, 7H), 3.96 (s, 3H), 1.28 (b, 3H). MS *m*/*z:* (M+Na)⁺ calcd for C₂₂H₂₂N₄O₃Na: 413.16; found 413.15. HPLC retention time: 1.47 minutes (column B).

Compound **21b**, R = CH₂-CH=CH₂, *(R)-N-(benzoyl)-3-methyl-N'-((1-allyl-7-azaindol-3-yl)-oxoacetyl]-piperazine:* ¹H NMR (500 MHz, CD₃OD) δ 8.37 (m, 3H), 7.44 (m, 6H), 6.08 (m, 1H), 5.22 - 3.06 (m, 11H), 1.27 (m, 3H); ¹³C NMR (75 MHz, CD₃OD) δ 184.2, 184.1, 170.8, 165.0, 146.7, 143.5, 137.9, 133.8, 131.4, 129.2, 128.8, 127.3, 125.6, 117.9, 117.4, 116.3, 110.3, 50.4, 49.7, 49.1, 45.7, 44.0, 41.0, 39.6, 34.8, 14.0, 12.8, MS *m*/*z:* (M+H)⁺ calcd for C₂₄H₂₅N₄O₃: 417.19; found 417.11. HPLC retention time: 1.43 minutes (column A).

### 14) Group transfer reactions from halide (equation 18, Scheme 8)

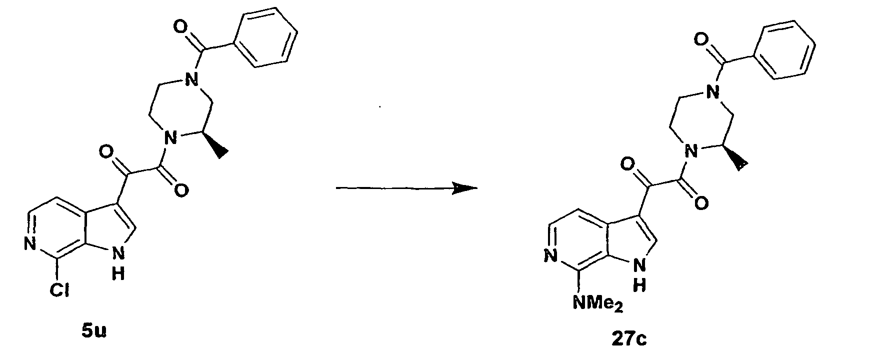

Preparation of (*R*)*-N-*(*benzoyl*)*-3-methyl-N'-*[(*7-dimethylamino-6-azaindol-3-yl*)*-oxoacetyl*]*-piperazine* **27c:** A mixture of compound **5u** (50 mg) and 4 ml of dimethylamine (40% in water) was heated to 150°C in sealed tube for 18 hours. The solvents were then removed under vaccum and the residue was purified using Shimadzu automated preparative HPLC System to give 10 mg of compound **27c**.

### Characterization of compound 27c:

Compound **27c,** *(R)-N-(benzoyl)-3-methyl-N'-[(7-dimethylamino-6-azaindol-3-yl)-oxoacetyl]-piperazine:* MS *m*/*z*: (M+H)⁺ calcd for C₂₃H₂₆N₅O₃ 420.20, found 420.16. HPLC retention time: 1.13 minutes (column A).

### 15) Modification of benzoyl moiety(equation 26, Scheme 11)

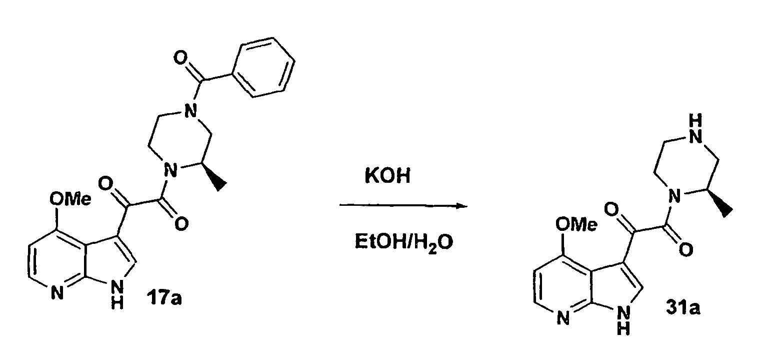

Hydrolysis of benzoyl amide, preparation of *(R)-2-methyl-N-[(4-methoxy-7-azaindol-3-yl)-oxoacetyl]-piperazine* **31a**: Compound **17a** (0.9 g) and KOH (2.0 g) were mixed in a solution of EtOH (15 ml) and water (15 ml). The reaction was refluxed for 48 hours. Solvents were removed under vaccum and the resulting residue was purified by silica gel column chromatography (EtOAc / .Et₃N = 100 : 1 to 3 :1) to afford 0.6 g of compound **31a.**

### Characterization of compound 31a:

Compound **31a,** *(R)-2-methyl-N-[(4-methoxy-7-azaindol-3-yl)-oxoacetyl]-piperazine:* MS m/z: (M+H)⁺ calcd for C₁₅H₁₉N₄O₃ 303.15, found 303.09. HPLC retention time: 0.29 minutes (column A).

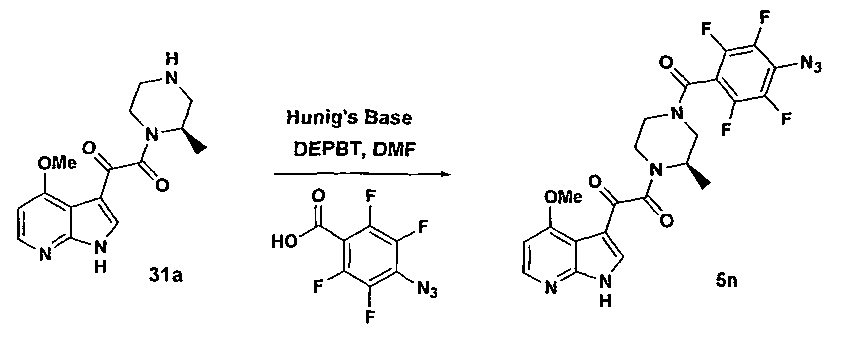

Diamide formation: *Preparation* of *(R)-N-(4-azido-2,3,5,6-tetra-fluorobenzoyl)-3-methyl-N'-[(4-methoxy-7-azaindol 3-yl)-oxoacetyl] piperazine 5n:* Amine **31a** (0.15 g), 4-azido-2,3,5,6-teirafluorobenzoic acid (0.12 g), 3-(diethoxyphosphoryloxy)-1,2,3-benzotriazin-4(3*H*)-one (DEPBT) (0.15 g) and Hunig's Base (0.5 ml) were combined in 5 ml of DMF. The mixture was stirred at room temperature for 8 hours. Solvents were then removed under vaccum and the residue was purified using Shimadzu automated preparative HPLC System to give 10 mg of compound **5n.**

### Characterization of compound 5n:

Compound **5n**, *(R)-N-(4-azido-2,3,5,6-tetra-fluorobenzoyl)-3-methyl N'-[(4-methoxy-7 azaindol-3-yl)-oxoacetyl]-piperazine:* MS *m*/*z*: (M+H)⁺ calcd for C₂₂H₁₈F₄N₇O₄ 520.14, found 520.05. HPLC retention time: 1.42 minutes (column A).

Compound **5af**, Ar = 4, 5-dibromophenyl, *(R)-N-(3, 5-dibromobenzyl)-3-methyl-N'-[(4-methoxy-7-azaindol-3-yl)-oxoacetyl]-piperazine:* MS *m*/*z:* (M+H)⁺ calcd for C₂₂H₂₁Br₂N₄O₄ 562.99, found 562.99. HPLC retention time: 1.54 minutes (column A).

Compound **5ag,** Ar = 4-[3-(trifluoromethyl)-3H-diazirin-3-yl]phenyl, *(R)-N-[4-(3-(trifluoromethyl)-3H-diazirin-3-yl)benzyl]-3-methyl-N'-[(4-methoxy-7-azaindol-3-yl)-oxoacetyl]-piperazine:* MS *m*/*z:* (M+H)⁺ calcd for C₂₄H₂₂F₃N₆O₄ 515.17, found 515.02. HPLC retention time: 1.55 minutes (column A).

New Equation:

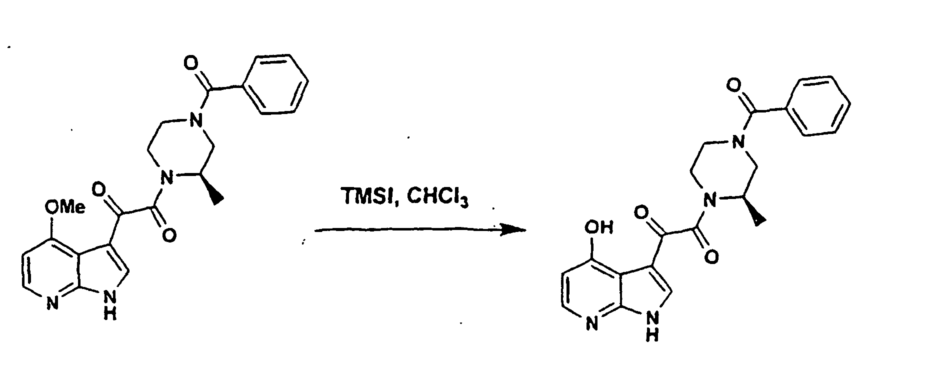

*Preparation of (R)-N-(benzoyl)-3-methyl-N'-[(4-hydroxyl-7-azaindol-3-yl)-oxoacetyl]-piperazine **5ah:*** The crude compound **17a** (100 mg) and an excess of TMSI (0.25 ml) were mixed in CHCl₃. The reaction mixture was stirred for 6 days. The solvent was removed under vacuum, he crude product was purified using a Shimadzu automated preparative HPLC System to give compound 4.4 mg of **5ah.**

### Characterization of compound 5ah:

Compound **5ah,** *(R)-N-(benzoyl)-3-methyl-N'-[(4-hydroxyl-7-azaindol3-yl)-oxoacetyl]-piperazine:* MS *m*/*z*: (M+H)⁺ calcd for C₂₁H₂₁N₄O₄: 393.16; found 393.11. HPLC retention time: 1.46 minutes (column B).

### Alternate procedures useful for the synthesis of Compound 39

Preparation of 5,7-dibromo-4-methoxy-6-azaindole **36:** Vinylmagnesium bromide (0.85 M in THF, 97.7 mL, 83.0 mmol) was added over 30 min. to a stirring solution of 2,6-dibromo-3-methoxy-5-nitropyridine (7.4 g, 23.7 mmol) in THF (160 mL) at -75°C. The solution was stirred 1h at -75 °C, overnight at -20 °C, recooled to -75 °C and quenched with saturated aqueous NH₄Cl (~100 mL). The reaction mixture was allowed to warm to rt, washed with brine (~100 mL) and extracted with Et₂O (150 mL) and CH₂Cl₂ (2 x 100 mL). The combined organics were dried (MgSO₄), filtered and concentrated. The residue was purified by flash column chromatography (SiO₂, 3:1 hexanes/EtOAc) to yield 5,7-dibromo-4-methoxy-6-azaindole **36** (1.10 g, 3.60 mmol, 15%) as a pale yellow solid.

Characterization of **36**: ¹H NMR (500 MHz, CDCl₃) 8.73 (br s, 1 H), 7.41 (dd, *J* = 3.1, 2.8 Hz, 1H), 6.69 (d, *J* = 3.1, 2.2 Hz, 1H), 4.13 (s, 3H); ¹³C NMR (125 MHz, CDCl₃) 146.6, 133.7, 128.8, 127.5, 120.2, 115.6, 101.9, 60.7. MS *m*/*z* (M+H)⁺ calcd for C₈H₇Br₂N₂O: 304.88; found 304.88. HPLC retention time: 1.31 minutes (column A).

Preparation of 4-methoxy-6-azaindole **37:** A solution of 5,7-Dibromo-4-methoxy-6-azaindole **36** (680 mg, 2.22 mmol), 5% Pd/C (350 mg, 0.17 mmol) and hydrazine (2.5 mL, 80 mmol) in EtOH was heated at reflux for 1 h. The reaction mixture was allowed to cool to rt, filtered through celite and the filtrate concentrated. Aqueous NH₄OH (11% in H₂O, 45 mL) was added to the residue and the solution was extracted with CH₂Cl₂ (3 x 30 mL). The combined organics were dried (MgSO₄), filtered and concentrated to yield 4-methoxy- 6-azaindole 37 (290 mg, 1.95 mmol, 88%) as an orange solid.

Characterization of **37:** ¹H NMR (500 MHz, CDCl₃) 8.61 (br s, 1H), 8.52 (s, 1H), 7.88 (s, 1H), 7.30 (d, *J* = 2.9 Hz, 1H), 6.69 (d, *J* = 2.9 Hz, 1H), 4.03 (s, 3H). MS *m*/*z* (M+H)⁺ calcd for C₈H₉N₂O: 149.06; found 148.99. HPLC retention time: 0.61 minutes (column A).

Preparation of **38:** Aluminum trichloride (67 mg, 0.50 mmol) was added to a solution of 4-methoxy-6-azaindole (15 mg, 0.10 mmol) in CH₂Cl₂ (2 mL) and stirred at rt for 30 min. Methyl chlorooxacetate (0.020 mL, 0.21 mmol) was added and the reaction mixture was stirred overnight. The reaction was quenched with MeOH (0.20 mL), stirred 5 h and filtered (flushing with CH₂Cl₂). The filtrate was washed with saturated aqueous NH₄OAc (2 x 10 mL) and H₂O (10 mL) and concentrated to yield **38** (5 mg) as a yellow solid.

Characterization of **38**: ¹H NMR (500 MHz, CDCl₃) 8.65 (s, 1H), 8.36 (s, 1H), 8.02 (s, 1H), 4.03 (s, 3H), 3.96 (s, 3H). MS m/z (M+H)⁺ calcd for C₁₁H₁₀N₂O₄: 235.06; found 234.96. HPLC retention time: 0.63 minutes (column A).

Preparation of N-benzoyl-N'-[(2-carboxaldehyde-pyrrole-4-yl)-oxoacetyl]-piperazine **41:** A solution of ethyl 4-oxoacetyl-2-pyrrolecarboxaldehyde **40** (17.0 g, 87.1 mmol) in 25 mL of KOH (3.56 M in H₂O, 88.8 mmol) and EtOH (400 mL) was stirred 2h. The white precipitate that formed was collected by filtration, washed with EtOH (~30 mL) and Et₂O (~30 mL) and dried under high vacuum to yield 15.9 g of potassium 2-pyrrolecarboxaldehyde-4-oxoacetate as a white solid that was used without further purification. A solution of potassium 2-pyrrolecarboxaldehyde-4-oxoacetate (3.96 g, 19.3 mmol), N-benzoylpiperazine hydrochloride (4.54 g, 19.7 mmol), 3-(diethoxyphosphoryloxy)-1,2,3-benzotriazin-4(3*H*)-one (5.88 g, 19.7 mmol) and triethylamine (3.2 mL, 23 mmol) in DMF (50 mL) was stirred 1d. The reaction mixture was filtered into H₂O (300 mL), extracted with CH₂Cl₂ (3 x 200 mL) and the combined organics were concentrated on a rotary evaporator to remove the CH₂Cl₂. The crude material (still in DMF) was then diluted with H₂O (200 mL) and allowed to recrystallize for 48 h. The solid was then collected by filtration and dried under high vacuum (P₂O₅) to yield N-benzoyl-N'-[(2-carboxaldehyde-pyrrole-4-yl)-oxoacetyl]-piperazine **41** (3.3 g, 9.7 mmol, 45% over two steps) as a light yellow solid. No further purification was required.

Characterization of **41:** ¹H NMR (500 MHz, CDCl₃) 9.79 (s, 1H), 9.63 (s, 1 H), 7.82 (s, 1H), 7.51-7.34 (m, 6H), 4.05-3.35 (m, 8H). MS *m*/*z* (M+H)⁺ calcd for C₁₈H₁₈N₃O₄: 340.12; found 340.11. HPLC retention time: 1.04 minutes (column A).

Preparation of **42:** N-benzoyl-N'-[(2-carboxaldehyde-pyrrole-4-yl)-oxoacetyl]-piperazine **41** (3.3 g, 9.7 mmol) was stirred as a slurry in EtOH (100 mL) for 15 min., cooled to 0°C and then reacted with glycine methyl ester hydrochloride (3.66 g, 29.2 mmol), triethylamine (1.50 mL, 11 mmol) and sodium cyanoborohydride (672 mg, 10.7 mmol). The reaction mixture was allowed to warm to rt, stirred 24 h and poured into ice (~400 mL). The solution was extracted with EtOAc (3 x 300 mL) and the combined organics were washed with brine (300 mL), dried (MgSO₄) and concentrated under reduced pressure. The residue was purified by preparative thin layer chromatography (SiO₂, 9:1 EtOAc/MeOH, R_{f} = 0.2) to yield **42** (2.4 g, 5.8 mmol, 60%) as a white solid.

Characterization of **42:** ¹H NMR (500 MHz, CDCl₃) 9.33 (s, 1H), 7.49 (s, 1H), 7.58-7.32 (m, 5H), 6.50 (s, 1H), 3.90-3.35 (m, 8H), 3.81 (s, 2H), 3.74 (s, 3H), 3.40 (s, 2H). MS *m*/*z* (M⁺H)⁺ calcd for C₂₁H₂₅N₄O₅: 413.17; found 413.17. HPLC retention time: 0.84 minutes (column A).

Preparation of **43:** Methyl ester **42** (485 mg, 1.17 mmol) and K₂CO₃ (325 mg, 2.35 mmol) in MeOH (6 mL) and H₂O (6 mL) were stirred at rt for 3h. The reaction mixture was then quenched with concentrated HCl (0.40 mL) and concentrated under high vacuum. Part of the solid residue (200 mg, 0.37 mmol) was added to a stirring solution of P₂O₅ (400 mg, 1.4 mmol) in methanesulfonic acid (4.0 g, 42 mmol) (which had already been stirred together at 110°C for 45 min.) at 110°C and stirred for 15 min. The reaction mixture was poured over crushed ice (~20 g), stirred 1 h, basified with K₂CO₃ (5.0 g, 38 mmol), diluted with CH₂Cl₂ (20 mL), and benzoyl chloride (1.0 mL, 8.5 mmol) and stirred 1 h. The reaction mixture was extracted with CH₂Cl₂ (3 x 20 mL) and the combined organics were dried (Na₂SO₄) and concentrated under reduced pressure. The residue was purified by preparative thin layer chromatography (SiO₂, EtOAc, R_{f} = 0.5) to yield **43** (101 mg g, 0.21 mmol, 57%) as an off white solid.

Characterization of **43:** MS *m*/*z* (M+H)⁺ calcd for C₂₇H₂₄N₄O₅: 485.17; found 485.07. HPLC retention time: 1.15 minutes (column A).

Preparation of **39.** R = OMe, *N-(benzoyl)-N'-[(*4*-methoxy-6-azaindol-3-yl*)*-oxoacetyl]-piperazine:*

In a flask affixed with a Dean-Stark trap, p-toluenesulfonic acid hydrate (55 mg, 0.29 mmol) and benzene (5 mL) were heated to reflux for 1 h. The solution was cooled to rt and reacted with 2,2-dimethoxypropane (0.10 mL, 0.81 mmol) and **43** (46 mg, 0.095 mmol). The reaction mixture was stirred 1 h, diluted with CH₂Cl₂ (2 mL), stirred 30 min. and then oxidized with tetrachlorobenzoquinone (150 mg, 0.61 mmol) and stirred overnight. The reaction mixture was poured into 5% aqueous NaOH (20 mL) and extracted with CH₂Cl₂ (3 x 25 mL). The combined organics were dried (Na₂SO₄) and concentrated under reduced pressure. The residue was subjected to preparative thin layer chromatography (Et₂O), the baseline material was extracted and resubjected to preparative thin layer chromatography (SiO₂, 9:1 EtOAc/MeOH, R_{f} = 0.15) to yield 39 (3 mg, 0.008 mmol, 6%) as a white solid.

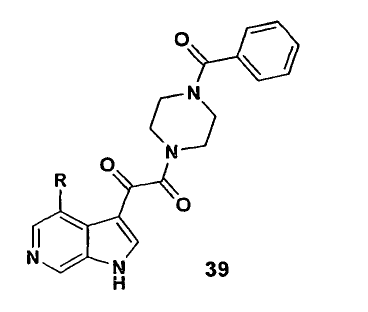

Compound **39,** R = OMe, *N-(benzoyl)-3-methyl-N'-[(4-methoxy-6-azaindol-3-yl)-oxoacetyl]-piperazine:*

Characterization of **39:** ¹H NMR (500 MHz, CD₃OD) 8.49 (s, 1H), 8.35 (s, 1H), 7.98 (s, 1H), 7.53-7.38 (m, 5H), 4.02 (s, 3H), 3.97-3.42 (m, 8H). MS *m*/*z* (M+H)⁺ calcd for C₂₁H₂₃N₄O₅: 393.15; found 393.13. HPLC retention time: 0.85 minutes (column A).

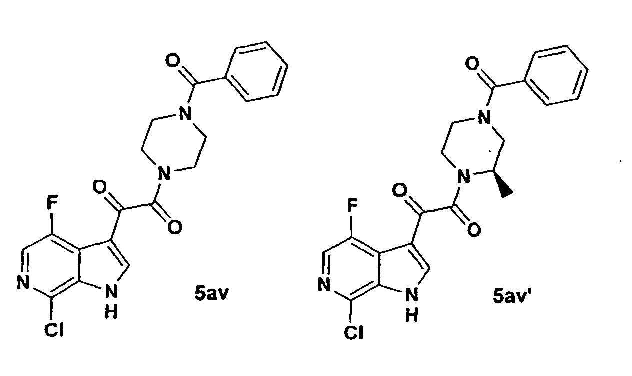

Preparation of **5av** *N-(benzoyl)-N'-[(4-fluoro-7-chloro-6-azaindol-3-yl)-oxoacetyl]-piperazine* and **5 av**'*(R)-N-(benzoyl)-3-methyl-N'-[(4-fluoro-7-chloro-6-azaindol-3-yl)-oxoacetyl]-piperazine*

It should be noted that 2-chloro-5-fluoro-3-nitro pyridine may be prepared by the method in example 5B of reference 59 Marfat et.al. The scheme below provides some details which enhance the yields of this route. The Bartoli chemistry in Scheme 1 was used to prepare the aza indole **1zz** which is also detailed below.

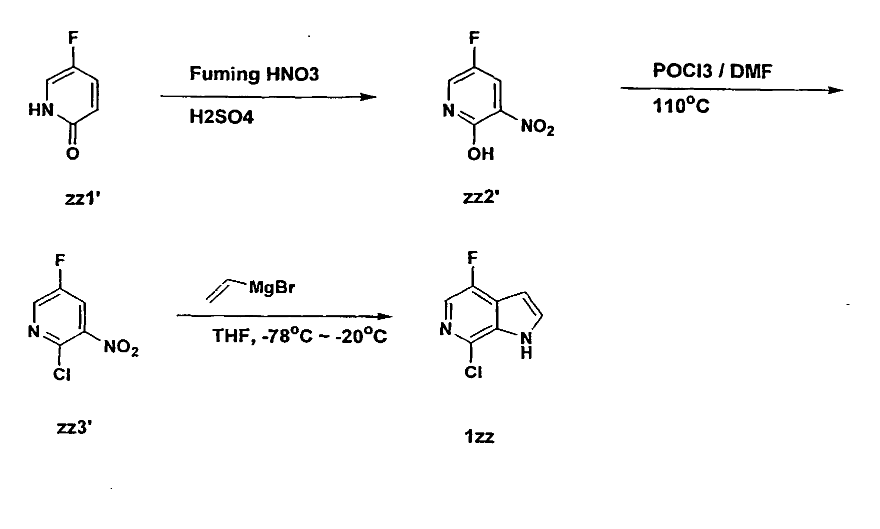

Compound **zz1'** (1.2g, 0.01mol) was dissolved in 2.7ml of sulphuric acid at room temperature. Premixed fuming nitric acid (1ml) and sulphuric acid was added dropwise at 5 - 10°C to the solution of compound **zz1'.** The reaction mixture was heated to 85°C for 1 hr, then cooled to room temperature and poured into ice (20g). The yellow solid product **zz2'** was collected by filtration, washed with water and dried in air to yield 1.01 g of compound **zz2'.**

Compound **zz2'** (500mg, 3.16mmol) was dissolved in Phosphorus oxychloride (1.7ml, 18.9mmol) and DMF (Cat) at room temperature. The reaction was heated to 110°C for 5 hr. The excess POCl3 was removed in vacuo. The residue was chromatographed on silica gel (CHCl3, 100%) to afford 176mg of product **zz3'.**

Compound **zz3'** (140mg, 0.79mmol) was dissolved in THF (5ml) and cooled to -78°C under N2. Vinyl magnesium bromide (1.0M in ether, 1.2ml) was added dropwise. After the addition was completed, the reaction mixture was kept at -20°C for about 15 hr. The reaction was then quenched with saturated NH4Cl, extracted with EtOAc. The combined organic layer was washed with brine, dried over MgSO4, concentrated and chromatographed to afford about 130mg of compound **1zz.**

The chemistry in Scheme 3 provided the derivatives which corresponds to general formula **5** and has a 6-aza ring and R₂=F and R₄ = Cl. In particular, reaction of 2-chloro-5-fluoro-3-nitro pyridine with 3 equivalents of vinyl Magnesium bromide using the typical conditions described herein will provide 4-fluoro-7-chloro-6-azaindole in high yield. Addition of this compound to a solution of aluminum trichloride in dichloromethane stirring at ambident temperature followed 30 minutes later with chloromethyl or chloroethyl oxalate provided an ester. Hydrolysis with KOH as in the standard procedures herein provided an acid salt which reacted with piperazines **4** (for example 1-benzoyl piperazine) in the presence of DEPBT under the standard conditions described herein to provide the compound **5** described just above. The compound with the benzoyl piperazine is *N-(benzoyl)-N'-[(4-fluoro-7-chloro-6-azaindol-3-yl)-oxoacetyl]-piperazine* and is compound **5av.** The compound with the (R)- methyl benzoyl piperazine is **5 av'**(*R*)-*N*-(*benzoyl*)*-3-methyl-N'-[(4-fluoro-7-chloro-6-azaindol-3-yl)-oxoacetyl]* - *piperazine* and is compound **5av'**

Characterization of **5av** *N-(benzoyl)-N'-[(4-fluoro-7-chloro-6* - *azaindol-3-yl)-oxoacetylJ-piperazine* and **5** ***av**'(R)-N-(benzoyl)-3-methyl-N'* - *[(4-fluoro-7-chloro-6-azaindol-3-yl)-oxoacetyl]-piperazine* 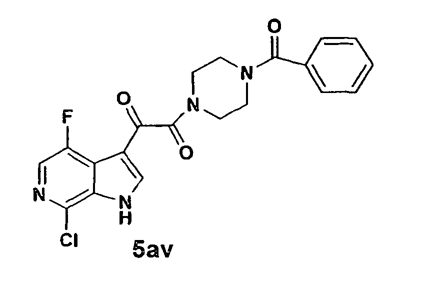 ¹H NMR (500 MHz, CD3OD): 8.40 (s, 1H), 8.04 (s, 1H), 7.46 (bs, 5H), 3.80 ~ 3.50 (m, 8H).
LC/MS: (ES+) m/z (M+H)⁺ = 415, RT = 1.247. 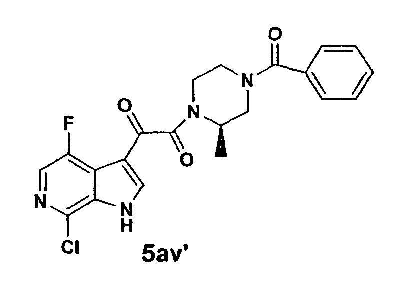 ¹H NMR (500 MHz, CD3OD): 8.42 (s, 1/2H), 8.37 (s, 1/2H), 8.03 (s, 1H), 7.71 ~ 7.45 (m, 5H), 4.72 ~ 3.05 (m, 7H), 1.45 ~ 1.28 (m, 3H).
LC/MS: (ES+) m/z (M+H)⁺ = 429, RT = 1.297.
LC/MS Column: YMC ODS-A C18 S7 3.0x50mm. Start %B = 0, Final %B = 100, Gradient Time = 2 min, Flow rate = 5 ml/min. Wavelength = 220nm. Solvent A = 10% MeOH - 90% H2O - 0.1 % TFA.
Solvent B = 90% MeOH - 10% H2O - 0.1% TFA.
Similarly compounds 5ay, 5az, 5abc and 5abd can be made:

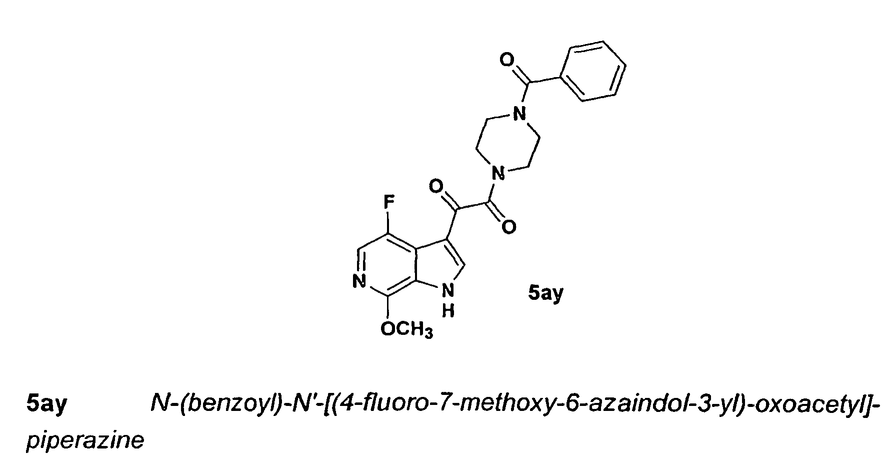

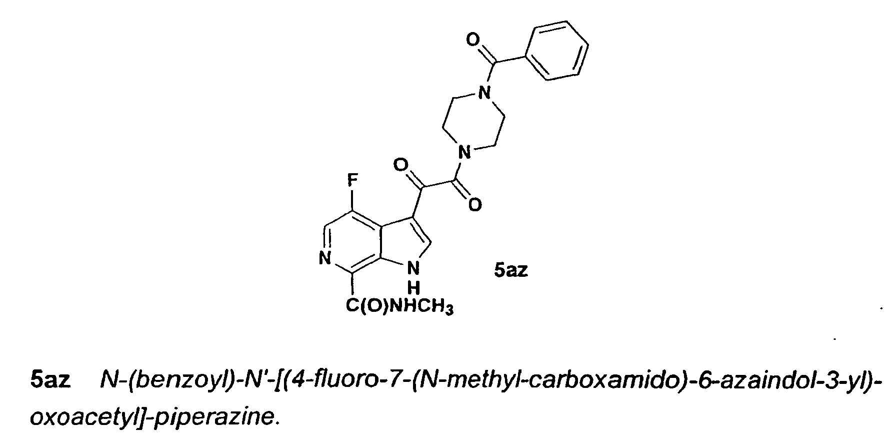

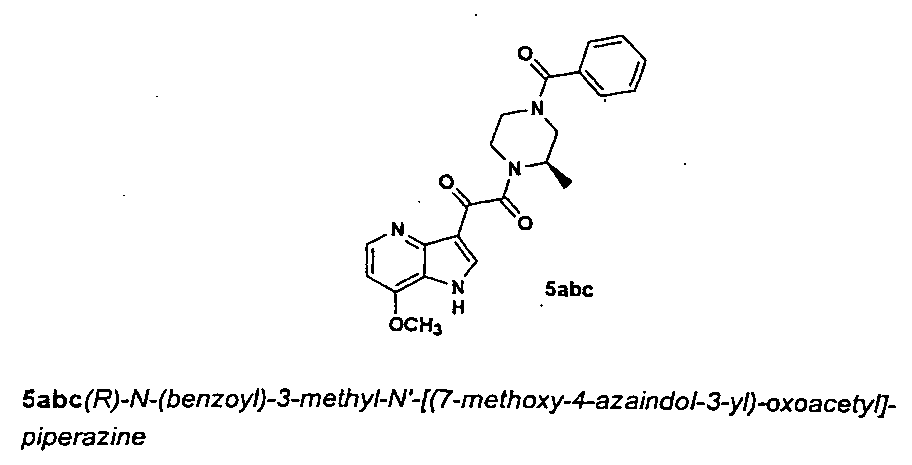

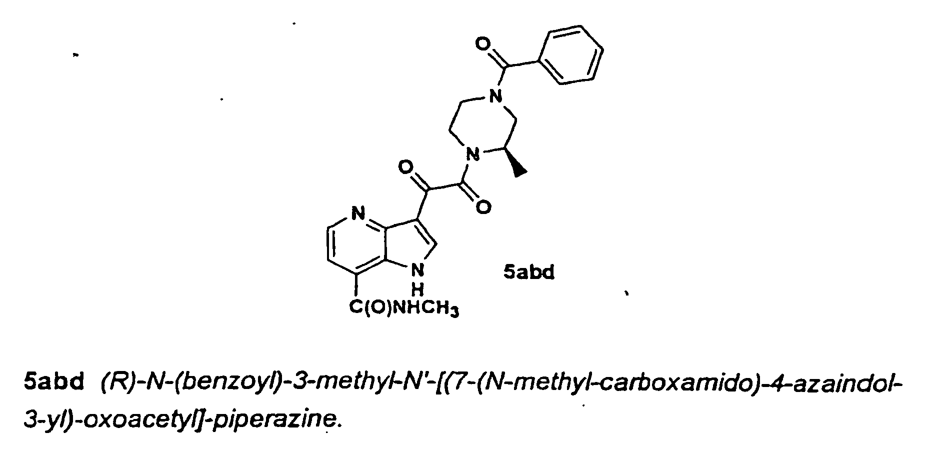

The compounds of the present invention may be administered orally, parenterally (including subcutaneous injections, intravenous, intramuscular, intrastemal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a pharmaceutical composition for treating viral infections such as HIV infection and AIDS. The treatment involves administering to a patient in need of such treatment a pharmaceutical composition comprising a pharmaceutical carrier and a therapeutically-effective amount of a compound of the present invention.

The pharmaceutical composition may be in the form of orally-administrable suspensions or tablets; nasal sprays, sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

### Abbreviations or Alternative Names

- TFA: Trifluoroacetic Acid
- DMF: *N*,*N* Dimethylformamide
- THF: Tetrahydrofuran
- MeOH: Methanol
- Ether: Diethyl Ether
- DMSO: Dimethyl Sulfoxide
- EtOAc: Ethyl Acetate
- Ac: Acetyl
- Bz: Benzoyl
- Me: Methyl
- Et: Ethyl
- Pr: Propyl
- Py: Pyridine
- Hunig's Base: *N*,*N*-Diisopropylethylamine
- DEPBT: 3-(Diethoxyphosphoryloxy)-1,2,3-benzotriazin-4(3*H*)-one
- DEPC: diethyl cyanophosphate
- DMP: 2,2-dimethoxypropane
- mCPBA: meta-Chloroperbenzoic Acid
- azaindole: 1*H*-Pyrrolo-pyridine
- 4-azaindole: 1*H*-pyrrolo[3,2-*b*]pyridine
- 5-azaindole: 1*H*-Pyrrolo[3,2-*c*]pyridine
- 6-azaindole: 1*H*-pyrrolo[2,3-*c*]pyridine
- 7-azaindole: 1*H*-Pyrrolo[2,3-*b*]pyridine

## Claims

1. A compound of formula I, or a pharmaceutically acceptable salt thereof, 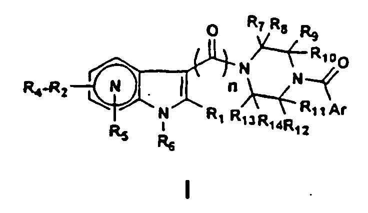 wherein: 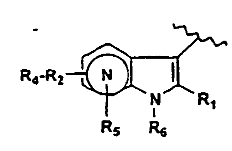 is selected from the group consisting of 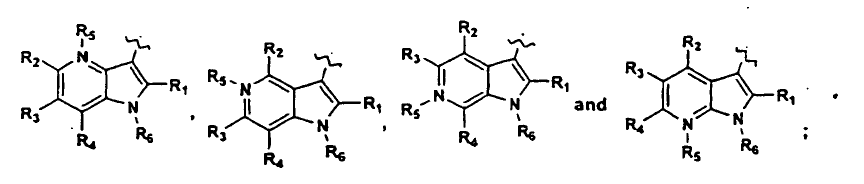 R₁, R₂, R₃, R₄ are each independently selected from the group consisting of H, C₁-C₆ alkyl, C₃-C₆ cycloalkyl, C₂-C₆ alkenyl, C₄-C₆ cycloalkenyl, C₂-C₆ alkynyl, halogen, CN, phenyl, nitro, OC(O)R₁₅, C(O)R₁₅, C(O)OR₁₆. C(O)NR₁₇R₁₈, OR₁₉, SR₂₀ and NR₂₁R₂₂;
R₁₅, is independently selected from the group consisting of H, C₁-C₆ alkyl, C₃-C₆ cycloalkyl, C₂-C₆ alkenyl and C₄-C₆ cycloalkenyl;
R₁₆, R₁₉, and R₂₀ are each independently selected from the group consisting of H, C₁-C₆ alkyl, C₁₋₆ alkyl substituted with one to three halogen atoms, C₃-C₆ cycloalkyl, C₂-C₆ alkenyl, C₄-C₆ cycloalkenyl, and C₃-C₆ alkynyl; provided the carbon atoms which comprise the carbon-carbon triple bond of said C₃-C₈ alkynyl are not the point of attachment to the oxygen or sulfur to which R₁₆, R₁₉, or R₂₀ is attached;
R₁₇ and R₁₈ are each independently selected from the group consisting of H, C₁-C₆ alkyl, C₃-C₆ cydoalkyl, C₃-C₆ alkenyl, C₄-C₆ cycloalkenyl, and C₃-C₆ alkynyl; provided the carbon atoms which comprise the carbon-carbon double bond of said C₃-C₆ alkenyl or the carbon-carbon triple bond of said C₃-C₆ alkynyl are not the point of attachment to the nitrogen to which R₁₇ and R₁₈ is attached;
R₂₁ and R₂₂ are each independently selected from the group consisting of H, OH, C₁-C₆ alkyl, C₃-C₆ cycloalkyl, C₃-C₆ alkenyl, C₅-C₆ cycloalkenyl, C₃-C₆ alkynyl, and C(O)R₂₃; provided the carbon atoms which comprise the carbon-carbon double bond of said C₃-C₆ alkenyl, C₄-C₆ cycloalkenyl, or the carbon-carbon triple bond of said C₃-C₆ alkynyl are not the point of attachment to the nitrogen to which R₂₁ and R₂₂ is attached;
R₂₃ is selected from the group consisting of H, C₁-C₆ alkyl, C₃-C₆ cycloalkyl, C₂-C₆ alkenyl, C₄-C₆ cycloalkenyl, and C₂-C₆ alkynyl;
R₅ is (O)ₘ, wherein m is 0 or 1;
n is 1 or 2;
R₆ is selected from the group consisting of H, C₁-C₆, alkyl, C₃-C₆ cycloalkyl, C₄-C₆ cycloalkenyl, C(O)R₂₄, C(O)OR₂₅, C(O)NR₂₆R₂₇, C₃-C₆ alkenyl, and C₃-C₆ alkynyl; provided the carbon atoms which comprise the carbon-carbon double bond of said C₃-C₆ alkenyl or the carbon-carbon triple bond of said C₃-C₆ alkynyl are not the point of attachment to the nitrogen to which R₆ is attached;
R₂₄ is selected from the group consisting of H, C₁-C₆ alkyl, C₃-C₆ cycloalkyl, C₃-C₆ alkenyl, C₄-C₆ cycloalkenyl, and C₃-C₆ alkynyl;
R₂₅ is selected from the group consisting of C₁-C₈ alkyl, C₃-C₈ cycloalkyl, C₂-C₆ alkenyl, C₄-C₆ cycloalkenyl, and C₃-C₆ alkynyl; provided the carbon atoms which comprise the carbon-carbon triple bond of said C₃-C₆alkynyl are not the point of attachment to the oxygen to which R₂₅ is attached;
R₂₆ and R₂₇ are each independently selected from the group consisting of H, C₁-C₆ alkyl, C₃-C₆ cycloalkyl, C₃-C₆ alkenyl, C₅-C₆ cycloalkenyl, and C₃-C₆ alkynyl; provided the carbon atoms which comprise the carbon-carbon double bond of said C₃-C₆ alkenyl, C₅-C₆ cycloalkenyl, or the carbon-carbon triple bond of said C₃-C₆ alkynyl are not the point of attachment to the nitrogen to which R₂₆ and R₂₇ are attached;
R₇, R₈, R₉, R₁₀, R₁₁, R₁₂, R₁₃, and R₁₄ are each independently selected from the group consisting of H, C₁-C₆ alkyl, C₃-C₆ cycloalkyl, C₂-C₆ alkenyl, C₄-C₆ cycloalkenyl, C₂-C₆ alkynyl, CR₂₈R₂₉OR₃₀, C(O)R₃₁, CR₃₂(OR₃₃)OR₃₄, CR₃₅NR₃₆R₃₇, C(O)OR₃₈, C(O)NR₃₉R₄₀, CR₄₁R₄₂F, CR₄₃F₂ and CF₃;
R₂₈, R₂₉, R₃₀, R₃₁, R₃₂, R₃₅, R₄₁, R₄₂ and R₄₃ are each independently selected from the group consisting of H, C₁-C₆ alkyl, C₃-C₆ cycloalkyl, C₂-C₆ alkenyl, C₄-C₆ cycloalkenyl, C₂-C₆ alkynyl and C(O)R₄₄;
R₃₃, R₃₄ and R₃₈ are each independently selected from the group consisting of H, C₁-C₆ alkyl, C₃-C₆ cycloalkyl, C₃-C₆ alkenyl, C₄-C₆ cycloalkenyl, and C₃-C₆ alkynyl; provided the carbon atoms which comprise the carbon-carbon triple bond of said C₃-C₆ alkynyl are not the point of attachment to the oxygen to which R₃₄ and R₃₈ are attached;
R₃₆ and R₃₇ are each independently selected from the group consisting of H, C₁-C₆ alkyl, C₃-C₆ cycloalkyl, C₃-C₆ alkenyl, C₄-C₆ cycloalkenyl, and C₃-C₆ alkynyl; provided the carbon atoms which comprise the carbon-carbon triple bond of said C₃-C₆ alkynyl are not the point of attachment to the nitrogen to which R₃₆ and R₃₇ are attached;
R₃₉ and R₄₀ are each independently selected from the group consisting of H, C₁-C₆ alkyl, C₃-C₆ cycloalkyl, C₂-C₆ alkenyl, C₄-C₆ cycloalkenyl, and C₃-C₆ alkynyl; provided the carbon atoms which comprise the carbon-carbon triple bond of said C₃-C₆ alkynyl are not the point of attachment to the nitrogen to which R₃₉ and R₄₀ are attached;
R₄₄ is selected from the group consisting of H, C₁-C₆ alkyl, C₃-C₆ cycloalkyl, C₂-C₆ alkenyl, C₄-C₆ cycloalkenyl, and C₂-C₆ alkynyl;
Ar is selected from the group consisting of 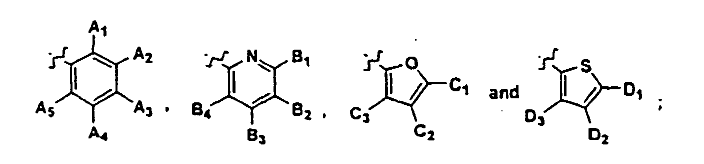 A₁, A₂, A₃, A₄, A₅, B₁, B₂, B₃, B₄, C₁, C₂, C₃, D₁, D₂, and D₃ are each independently selected from the group consisting of H, CN, halogen, NO₂, C₁-C₆ alkyl, C₃-C₆ cycloalkyl, C₂-C₆ alkenyl, C₄-C₆ cycloalkenyl, C₂-C₆ alkynyl, OR₄₅, NR₄₈R₄₇, SR₄₈, N₃ and CH(-N=N-)-CF₃;
R₄₅ is selected from the group consisting of H, C₁-C₆ alkyl, C₃-C₆ cycloalkyl, C₂-C₆ alkenyl, C₄-C₆ cycloalkenyl and C₃-C₆ alkynyl; provided the carbon atoms which comprise the carbon-carbon triple bond of said C₃-C₆ alkynyl are not the point of attachment to the oxygen to which R₄₅ is attached;
R₄₆ and R₄₇ are each independently selected from the group consisting of H, C₁-C₆ alkyl, C₃-C₆ cycloalkyl, C₃-C₆ alkenyl, C₅-C₆ cycloalkenyl, C₃-C₆ alkynyl and C(O)R₅₀; provided the carbon atoms which comprise the carbon-carbon double bond of said C₅-C₆ alkenyl, C₄-C₆ cycloalkenyl, or the carbon-carbon triple bond of said C₃-C₆ alkynyl are not the point of attachment to the nitrogen to which R₄₆ and R₄₇ are attached;
R₄₈ is selected from the group consisting of H, C₁-C₆ alkyl, C₃-C₆ cycloalkyl, C₂-C₆ alkenyl, C₄-C₆ cycloalkenyl, C₃-C₆ alkynyl and C(O)R₄₉; provided the carbon atoms which comprise the carbon-carbon triple bond of said C₃-C₆ alkynyl are not the point of attachment to the sulfur to which R₄₈ is attached;
R₄₉ is C₁-C₆ alkyl or C₃-C₆ cycloalkyl; and
R₅₀ is selected from the group consisting of H, C₁-C₆ alkyl, and C₃-C₆ cycloalkyl.

2. A compound of claim 1, or a pharmaceutically acceptable salt thereof, selected from the group consisting of compounds 5a, 5b, 5c, 5d, 5e, 5f, 5g, 5h, 5i and 5ai as identified below: 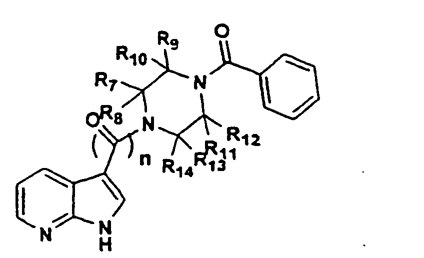
| **Compd #** | **n** | **R** |
|---|---|---|
| **5a** | 2 | R₇₋₁₃ = H, R₁₄ = (*R*)-Me |
| **5b** | 2 | R₇₋₈ = R₁₀₋₁₄ = H, R₉ = Et |
| **5c** | 1 | R₇₋₈ = R₁₀₋₁₄ = H, R₉ = Et |
| **5d** | 2 | R₇₋₁₄ = H |
| **5e** | 2 | R₇₋₈ = R₁₀₋₁₄ = H, R₉ = Me |
| **5f** | 2 | R₇₋₁₃ = H, R₁₄ = (*S*)-Me |
| **5g** | 2 | R₇₋₁₃ = H, R₁₄ = Et |
| **5h** | 2 | R₇₋₁₂ = H, R₁₃ = R₁₄ = Me |
| **5i** | 2 | R₇₋₈ = R₁₀₋₁₃ = H, R₉ = R₁₄ = Me |
| **5ai** | 2 | R₇₋₈ = R₉₋₁₃ = H, R₁₄ = Me |

3. A compound of claim 1, or a pharmaceutically acceptable salt thereof, selected from the group consisting of compounds 5j, 5k and 5l as identified below:

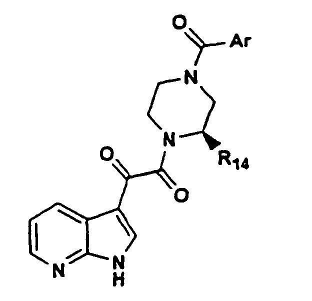

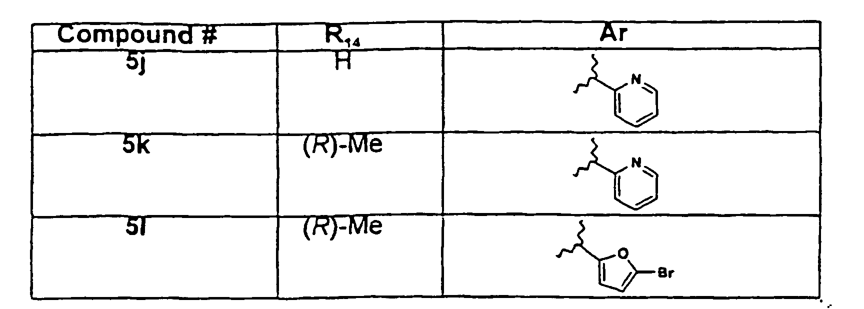

4. A compound of claim 1, or a pharmaceutically acceptable salt thereof, having the formula 5m identified below:

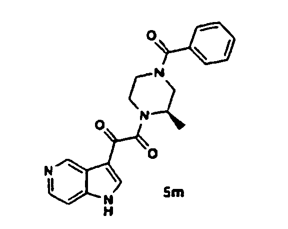

5. A compound of claim 1, or a pharmaceutically acceptable salt thereof, selected from the group consisting of compounds 8a, 15a, 16a, 16d and 16e identified below: 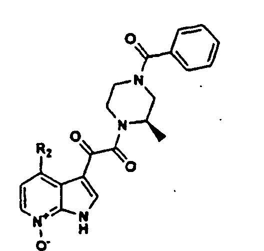
| **Compound #** | **R**_{**2**} |
|---|---|
| **8a** | H |
| **15a** | NO₂ |
| **16a** | OMe |
| **16d** | OEt |
| **16e** | SPr |

6. A compound of claim 1, or a pharmaceutically acceptable salt thereof, selected from the group consisting of compounds 9a, 9b, 10a, 11a, 11b, 11c, 12a, 14a, 17a-17f, 18a, 19a and 20a identified below: 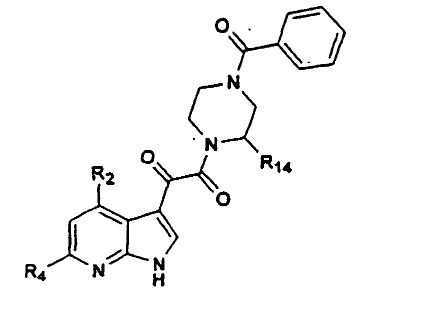
| **Compd #** | **R**_{**2**} | **R**_{**4**} | **R**_{**14**} |
|---|---|---|---|
| **9a** | Cl | H | (*R*)-Me |
| **9b** | H | Cl | (*R*)-Me |
| **10a** | NO₂ | F | (*R*)-Me |
| **11a** | H (when R₄=Me), Me (when R₄=H) | Me (when R₂=H), H (when R₂=Me) | (*R*)-Me |
| **11b** | H (when R₄=Ph), Ph (when R₄=H) | Ph (when R₂=H), H (when R₂=Ph) | (*R*)-Me |
| **11c** | H (when R₄=vinyl), Vinyl (when R₄=H) | Vinyl (when R₂=H), H (when R₂=Vinyl) | (*R*)-Me |
| **12a** | H | CN | (*R*)-Me |
| **14a** | H | OH | (*R*)-Me |
| **17a** | OMe | H | (*R*)-Me |
| **17d** | OMe | H | (*S*)-Me |
| **17e** | OMe | H | Me |
| **17b** | OCH₂CF₃ | H | (*R*)-Me |
| **17c** | O-*i-*Pr | H | (*R*)-Me |
| **17f** | H | PrS | (*R*)-Me |
| **18a** | NO₂ | H | (*R*)-Me |
| **19a** | NHOH | H | (*R*)-Me |
| **20a** | NH₂ | H | (*R*)-Me |

7. A compound of claim 6 or a pharmaceutically acceptable salt thereof, wherein R₂ is -OMe, R₄ is hydrogen, and R₁₄ is (*R*)-methyl.

8. A compound of claim 1, or a pharmaceutically acceptable salt thereof, selected from the group consisting of compounds 13a, 21a, and 21b identified below:

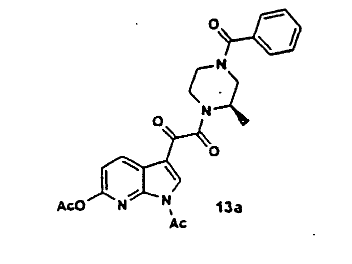

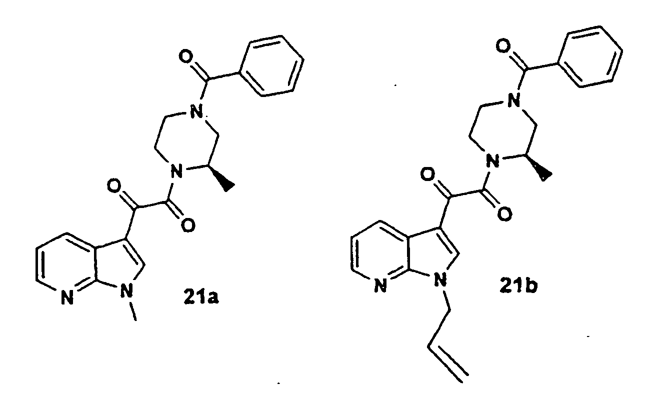

9. A compound of claim 1, or a pharmaceutically acceptable salt wherein R₂, R₃ and R₄ are each independently selected from the group consisting of H, -OCH₃, -OCH₂CF₃, -OiPr, -OnPr, halogen, CN, NO₂, C₁-C₆ alkyl, NHOH, NH₂, Ph, SR₂₀, and N(CH₃)₂.

10. A compound of claim 9, or a pharmaceutically acceptable salt wherein n is 2; R₁ is selected from the group consisting of H, C₁-C₆ alkyl and CH₂CH=CH₂; and R₅ is (O)ₘ wherein m is 0.

11. A compound of claim 10, or a pharmaceutically acceptable salt thereof, wherein R₇, R₈, R₉, R₁₀, R₁₁, R₁₂, R₁₃, and R₁₄ are each independently H or CH₃, provided one or two of the members of the group R₇-R₁₄ are CH₃ and the remaining members of the group R₇-R₁₄ are H.

12. A compound of claim 11, or a pharmaceutically acceptable salt thereof, wherein one of the members of the group A₁, A₂, A₃, A₄, A₅, B₁, B₂, B₃, B₄, C₁, C₂, C₃, D₁, D₂, and D₃ is selected from the group consisting of hydrogen, halogen and amino and the remaining members of the group A₁, A₂, A₃, A₄, A₅, B₁, B₂, B₃, B₄, C₁, C₂, C₃, D₁, D₂, and D₃ are hydrogen.

13. A compound of claim 1, or a pharmaceutically acceptable salt thereof, of the Formula below: 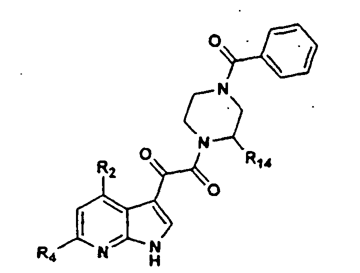 wherein:
R₂ is selected from the group consisting of H, -OCH₃, -OCH₂CF₃, -OPr, halogen, CN, NO₂, and NHOH;
R₄ is selected from the group consisting of H, -halogen, -CN, and hydroxy; and
R₁₄ is CH₃ or H.

14. A compound of claim 1, wherein R₄ is selected from the group consisting of OH, CN, halogen, -OCOCH₃ and C₁-C₆ alkyl.

15. A compound of claim 1, or a pharmaceutically acceptable salt thereof, of the formula identified below: 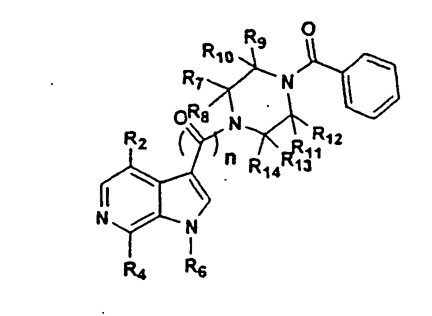 wherein:
R₂ is selected from the group consisting of H, F, Cl, Br, OMe, CN, and OH;
R₄ is selected from the group consisting of H, C₁-C₆ alkyl, C₂-C₆ alkenyl, C₃-C₈ cycloalkyl, C₅-C₆ cycloalkenyl, Cl, OMe, CN, OH, C(O)NH₂, C(O)NHMe, C(O)NHEt, phenyl and -C(O)CH₃:
n is 2;
R₈, R₉, R₁₀, R₁₁, R₁₂, R₁₃, and R₁₄ are each independently H or CH₃, provided 0-2 of the members of the group R₈, R₉, R₁₀, R₁₁, R₁₂, R₁₃, and R₁₄ may be CH₃ and the remaining members of the group R₈, R₉, R₁₀, R₁₁, R₁₂, R₁₃, and R₁₄ are H; and
R₆ is H or CH₃.

16. A compound of claim 1, or a pharmaceutically acceptable salt thereof, selected from the group consisting of compounds 5p, 5r, 5s, 5q, 5t, 5u, 5v and 27c identified below: 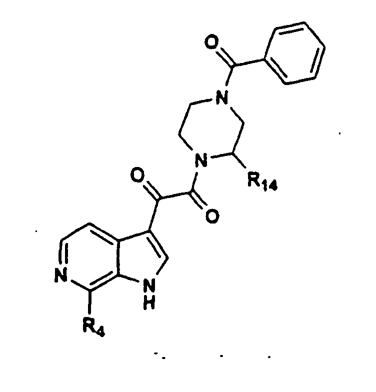
| **Compound #** | **R**_{**4**} | **R**_{**14**} |
|---|---|---|
| **5p** | H | H |
| **5r** | H | (*R*)-Me |
| **5s** | H | (*S*)-Me |
| **5q** | H | Me |
| **5t** | Cl | H |
| **5u** | Cl | (*R*)-Me |
| **5v** | OMe | (*R*)-Me |
| **27c** | NMe₂ | (*R*)-Me |

17. A compound of claim 1, or a pharmaceutically acceptable salt thereof of formula: 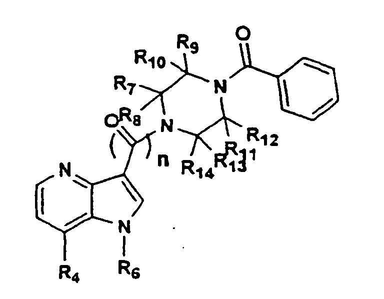 wherein:
R₄ is selected from the group consisting of H, C₁-C₆ alkyl, C₂-C₆ alkenyl, C₃-C₆ cycloalkyl, C₅-C₆ cycloalkenyl, Cl, OMe, CN, OH, C(O)NH₂, C(O)NHMe, C(O)NHEt, phenyl and -C(O)CH₃;
n is 2;
R₈, R₉, R₁₀, R₁₁, R₁₂, R₁₃, and R₁₄ are each independently H or CH₃, provided 0-2 of the members of the group R₈, R₉, R₁₀, R₁₁, R₁₂, R₁₃, and R₁₄ may be CH₃ and the remaining members of the group R₈, R₉, R₁₀, R₁₁, R₁₂, R₁₃, and R₁₄ are H; and
R₆ is H or CH₃.

18. A compound of claim 1, or a pharmaceutically acceptable salt thereof, selected from the group consisting of compounds 5w, 5x, 5y, 5z and 5ak identified below: 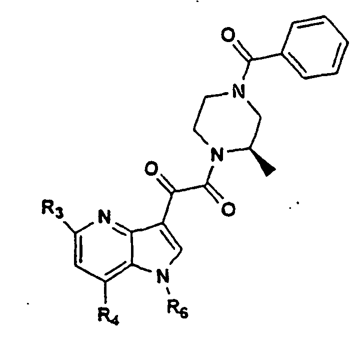
| **Compound #** | **R**_{**3**} | **R**_{**4**} | **R**_{**6**} |
|---|---|---|---|
| **5w** | **H** | **H** | **H** |
| **5x** | **H** | **Me** | **H** |
| **5y** | **H** | **Cl** | **H** |
| **5z** | **H** | **OMe** | **Me** |
| **5ak** | **Cl** | **Me** | **H** |

19. A compound of claim 15 wherein R₄, R₇, R₈, R₉, R₁₀, R₁₁, R₁₂, R₁₃ and R₁₄ are H; and R₂ is -OMe.

20. A compound of claim 15 wherein R₂, R₄, R₇, R₈, R₉, R₁₀, R₁₁, R₁₂, R₁₃ and R₁₄ are H.

21. A compound of claim 1, or a pharmaceutically acceptable salt thereof, having the formula 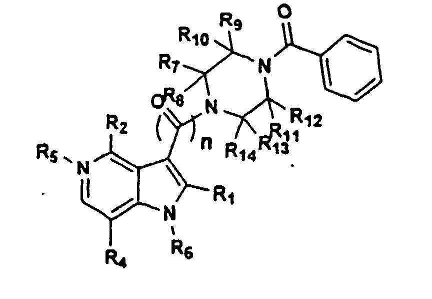 wherein:
R₂ is H, F, Cl, Br, OMe, CN, or OH;
R₄ is C₁-C₆ alkyl, C₂-C₆ alkenyl, C₃-C₆ cycloalkyl, C₅-C₆ cycloalkenyl, Cl, OMe, CN, OH, C(O)NH₂, C(O)NHMe, C(O)NHEt, Ph or -C(O)CH₃;
n is 2;
R₈, R₉, R₁₀, R₁₁, R₁₂, R₁₃ and R₁₄ are each independently H or CH₃, provided up to two of these substituents may be methyl;
R₁ is hydrogen;
R₅ is unsubstituted; and
R₆ is hydrogen or methyl.

22. A compound of claim 1 or pharmaceutically acceptable salts thereof, of the Formula 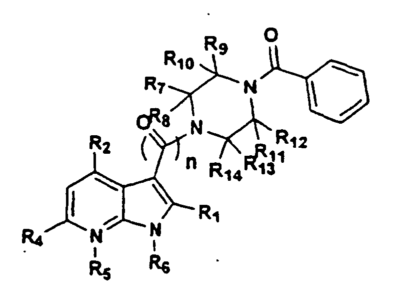 wherein:
R₂ is H, -OCH₃, -OCH₂CF₃, -OPr, halogen, CN, NO₂, or NHOH;
R₄ is H, -halogen, -CN, or hydroxy;
One or two members of R₇-R₁₄ is methyl and the remaining members are hydrogen;
n is 2;
R₁ is hydrogen;
R₅ is (O)ₘ, where m is O; and
R₆ is hydrogen, methyl, or allyl.

23. A pharmaceutical composition which comprises an antiviral effective amount of a compound of Formula I, including pharmaceutically acceptable salts thereof, as claimed in any of claims 1-22.

24. The pharmaceutical composition of claim 23, useful for treating infection by HIV, which additionally comprises an antiviral effective amount of an AIDS treatment agent selected from the group consisting of:
(a) an AIDS antiviral agent;
(b) an anti-infective agent;
(c) an immunomodulator; and
(d) HIV entry inhibitors.

25. Use of a compound of Formula I, including pharmaceutically acceptable salts thereof, as claimed in any of claims 1-22, for manufacturing a medicament for treating mammals infected with a virus by administration to said mammal of an antiviral effective amount.

26. The use of claim 25, wherein said antiviral effective amount of a compound of Formula I is in combination with an antiviral effective amount of an AIDS treatment agent selected from the group consisting of: an AIDS antiviral agent; an anti-infective agent; an immunomodulator; and HIV entry inhibitors.

27. The use of claim 25 or 26 wherein the virus is HIV.

28. A compound of any one of claims 1-22 for use as a medicament.

## Patentansprüche

1. Verbindung der Formel I oder ein pharmazeutisch verträgliches Salz davon 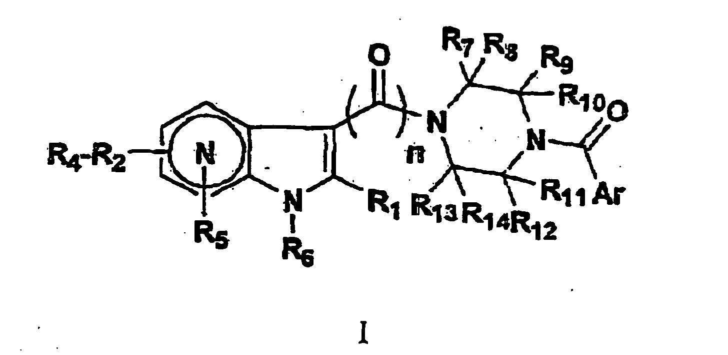 wobei 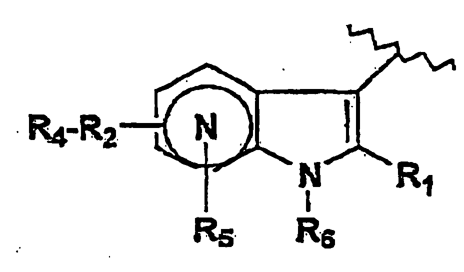 ausgewählt ist aus 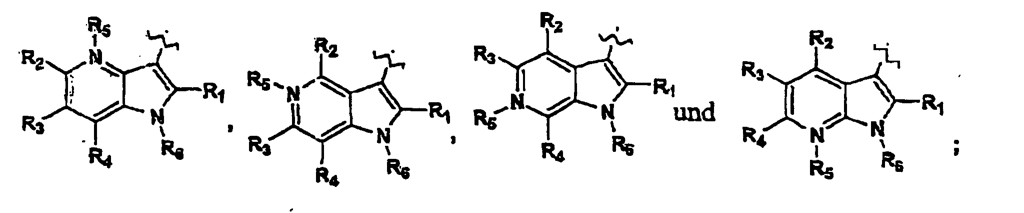 R₁, R₂, R₃, R₄ jeweils unabhängig voneinander ausgewählt sind aus H, C₁-C₆-Alkyl, C₃-C₆-Cycloalkyl, C₂-C₆-Alkenyl, C₄-C₆-Cycloalkenyl, C₂-C₆-Alkinyl, Halogen, CN, Phenyl, Nitro, OC(O)R₁₅, C(O)R₁₅, C(O)OR₁₆, C(O)NR₁₇R₁₈, OR₁₉, SR₂₀ und NR₂₁R₂₂; R₁₅ unabhängig ausgewählt ist aus H, C₁-C₆-Alkyl, C₃-C₆-Cycloalkyl, C₂-C₆-Alkenyl und C₄-C₆-Cycloalkenyl;
R₁₆, R₁₉ und R₂₀ jeweils unabhängig ausgewählt sind aus H, C₁-C₆-Alkyl, C₁₋₆-Alkyl, substituiert mit 1 bis 3 Halogenatomen, C₃-C₆-Cycloalkyl, C₂-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Sauerstoff oder Schwefel sind, an welchen R₁₆, R₁₉ oder R₂₀ gebunden sind;
R₁₇ und R₁₈ jeweils unabhängig ausgewählt sind aus H, C₁-C₆-Alkyl, C₃-C₆-Cycloalkyl, C₃-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Doppelbindung des C₃-C₆-Alkenyls oder die Kohlenstoff-Kohlenstoff-Dreifachbindung des C₃-C₆-Alkinyls umfassen, nicht der Verknüpfungspunkt zum Stickstoff sind, an welchen R₁₇ und R₁₈ gebunden sind;
R₂₁ und R₂₂ jeweils unabhängig ausgewählt sind aus H, OH, C₁-C₆-Alkyl, C₃-C₆-Cycloalkyl, C₃-C₆-Alkenyl, C₅-C₆-Cycloalkenyl, C₃-C₆-Alkinyl und C(O)R₂₃; mit der Maßgabe, dass die Kohlenstoffatome, welche die Kohlenstoff-Kohlenstoff-Doppelbindung des C₃-C₆-Alkenyls oder des C₄-C₆-Cycloalkenyls oder die Kohlenstoff-Kohlenstoff-Dreifachbindung des C₃-C₆-Alkinyls umfassen, nicht der Verknüpfungspunkt zum Stickstoff sind, an welchen R₂₁ und R₂₂ gebunden sind;
R₂₃ ausgewählt ist aus H, C₁-C₆-Alkyl, C₃-C₆-Cycloalkyl, C₂-C₆-Alkenyl, C₄-C₆-Cycloalkenyl und C₂-C₆-Alkinyl;
R₅ für (O)ₘ steht, wobei m gleich 0 oder 1 ist;
n gleich 1 oder 2 ist;
R₆ ausgewählt ist aus H, C₁-C₆-Alkyl, C₃-C₆-Cycloalkyl, C₄-C₆-Cycloalkenyl, C(O)R₂₄, C(O)OF₋₂₅, C(C)NR₂₆R₂₇, C₃-C₆-Alkenyl und C₃-C₆-Alkinyl; mit der Maßgabe, dass die Kohlenstoffatome, welche die Kohlenstoff-Kohlenstoff-Doppelbindung des C₃-C₆-Alkenyls oder die Kohlenstoff-Kohlenstoff-Dreifachbindung des C₃-C₆-Alkinyls umfassen, nicht der Verknüpfungspunkt zum Stickstoff sind, an welchen R₆ gebunden ist;
R₂₄ ausgewählt ist aus H, C₁-C₆-Alkyl, C₃-C₆-Cycloalkyl, C₃-C₆-Alkenyl, C₄-C₆-Cycloalkenyl und C₃-C₆-Alkinyl;
R₂₅ ausgewählt ist aus C₁-C₆-Alkyl, C₃-C₆-Cycloalkyl, C₂-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Sauerstoff sind, an welchen R₂₅ gebunden ist;
R₂₆ und R₂₇ jeweils unabhängig ausgewählt sind aus H, C₁-C₆-Alkyl, C₃-C₆-Cycloalkyl, C₃-C₆-Alkenyl, C₅-C₆-Cycloalkenyl und C₃-C₆-Alkinyl; mit der Maßgabe, dass die Kohlerenstoffatome, welche die Kohlenstoff-Kohlenstoff-Doppelbindung des C₃-C₆-Alkenyls oder des C₅-C₆-Cycloalkenyls oder die Kohlenstoff-Kohlenstoff-Dreifacabindung des C₃-C₆-Alkinyls umfassen, nicht der Verknüpfungspunkt zum Stickstoff sind, an welchen R₂₆ and R₂₇ gebunden sind;
R₇, R₈, R₉, R₁₀, R₁₁, R₁₂, R₁₃ und R₁₄ jeweils unabhängig ausgewählt sind aus H, C₁-C₆-Alkyl, C₃-C₆-Cycloalkyl, C₂-C₆-Alkenyl, C₄-C₆-Cycloalkenyl, C₂-C₆-Alkinyl, CR₂₈R₂₉OR₃₀, C(O)R₃₁, CR₃₂(OR₃₃)OR₃₄, CR₃₅NR₃₆R₃₇, C(O)OR₃₈, C(O)NR₃₉R₄₀, CR₄₁R₄₂F, CR₄₃F₂ und CF₃;
R₂₈, R₂₉, R₃₀, R₃₁, R₃₂, R₃₅, R₄₁, R₄₂ und R₄₃ jeweils unabhängig ausgewählt sind aus H, C₁-C₆-Alkyl, C₃-C₆-Cycloalkyl, C₂-C₆-Alkenyl, C₄-C₆-Cycloalkenyl, C₂-C₆-Alkinyl und C(O)R₄₄;
R₃₃, R₃₄ und R₃₈ jeweils unabhängig ausgewählt sind aus H, C₁-C₆-Alkyl, C₃-C₆-Cycloalkyl, C₃-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Sauerstoff sind, an welchen R₃₄ und R₃₈ gebunden sind;
R₃₆ und R₃₇ jeweils unabhängig ausgewählt sind aus H, C₁-C₆-Alkyl, C₃-C₆-Cycloalkyl, C₃-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Stickstoff sind, an welchen R₃₆ und R₃₇ gebunden sind;
R₃₉ und R₄₀ jeweils unabhängig ausgewählt sind aus H, C₁-C₆-Alkyl, C₃-C₆-Cycloalkyl, C₂-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Stickstoff sind, an welchen R₃₉ und R₄₀ gebunden sind;
R₄₄ ausgewählt ist aus H, C₁-C₆-Alkyl, C₃-C₆-Cycloalkyl, C₂-C₆-Alkenyl, C₄-C₆-Cycloalkenyl und C₂-C₆-Alkinyl;
Ar ausgewählt ist aus 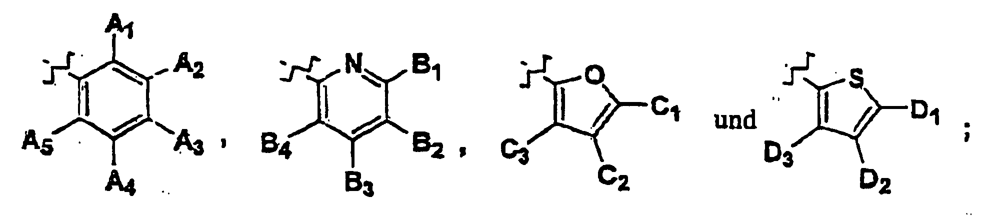 A₁, A₂, A₃, A₄, A₅, B₁, B₂, B₃, B₄, C₁, C₂, C₃, D₁, D₂ and D₃ jeweils unabhängig ausgewählt sind aus H, CN, Halogen, NO₂, C₁-C₆-Alkyl, C₃-C₆-Cycloalkyl, C₂-C₆-Alkenyl, C₄-C₆-Cycloalkenyl, C₂-C₆-Alkinyl, OR₄₅, NR₄₆R₄₇, SR₄₈, N₃ und CH(-N=N-)-CF₃;
R₄₅ ausgewählt ist aus H, C₁-C₆-Alkyl, C₃-C₆-Cycloalkyl, C₂-C₆-Alkenyl, C₄-C₆-Cycloalkenyl und C₃-C₆-Alkinyl; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Sauerstoff sind, an welchen R₄₅ gebunden ist;
R₄₆ und R₄₇ jeweils unabhängig ausgewählt sind aus H, C₁-C₆-Alkyl, C₃-C₆-Cycloalkyl, C₃-C₆-Alkenyl, C₅-C₆-Cycloalkenyl, C₃-C₆-Alkinyl und C(O)R₅₀; mit der Maßgabe, dass die Kohlenstoffatome, welche die Kohlenstoff-Kohlenstoff-Doppelbindung des C₅-C₆-Alkenyls oder des C₄-C₆-Cycloalkenyls oder die Kohlenstoff-Kohlenstoff-Dreifachbindung des C₃-C₆-Alkinyls umfassen, nicht der Verknüpfungspunkt zum Stickstoff sind, an welchen R₄₆ und R₄₇ gebunden sind;
R₄₈ ausgewählt ist aus H, C₁-C₆-Alkyl, C₃-C₆-Cycloalkyl, C₂-C₆-Alkenyl, C₄-C₆-Gycloalkenyl, C₃-C₆-Alkinyl und C(O)R₄₉; mit der Maßgabe, dass die Kohlenstoffatome, welche die Kohlenstoff-Kohlenstoff-Dreifachbindung des C₃-C₆-Alkinyls umfassen, nicht der Verknüpfungspunkt zum Schwefel sind, an welchen R₄₈ gebunden ist;
R₄₉ für C₁-C₆-Alkyl oder C₃-C₆-Cycloalkyl steht; und
R₅₀ ausgewählt ist aus H, C₁-C₆-Alkyl und C₃-C₆-Cycloalkyl.

2. Verbindung nach Anspruch 1 oder ein pharmazeutisch verträgliches Salz davon, ausgewählt aus den Verbindungen 5a, 5b, 5c, 5d, 5e, 5f, 5g, 5h, 5i und 5ai, wie nachstehend beschrieben: 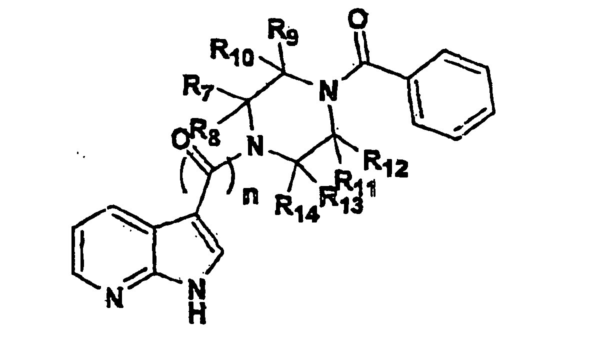
| **Verbindung#** | **n** | **R** |
|---|---|---|
| **5a** | 2 | R₇₋₁₃ = H, R₁₄ = (*R*)-Me |
| **5b** | 2 | R₇₋₈ = R₁₀₋₁₄ = H, R₉ = Et |
| **5c** | 1 | R₇₋₈ = R₁₀₋₁₄ = H, R₉ = Et |
| **5d** | 2 | R₇₋₁₄ = H |
| **5e** | 2 | R₇₋₈ = R₁₀₋₁₄ = H, R₉ = Me |
| **5f** | 2 | R₇₋₁₃ = H, R₁₄ = (*S*)-Me |
| **5g** | 2 | R₇₋₁₃ = H, R₁₄ = Et |
| **5h** | 2 | R₇₋₁₂ = H, R₁₃ = R₁₄ = Me |
| **5i** | 2 | R₇₋₈ = R₁₀₋₁₃ = H, R₉ = R₁₄ = Me |
| **5ai** | 2 | R₇₋₈ = R₉₋₁₃ = H, R₁₄ = Me |

3. Verbindung nach Anspruch 1 oder ein pharmazeutisch verträgliches Salz davon, ausgewählt aus den Verbindungen 5j, 5k und 5l, wie nachstehend beschrieben:

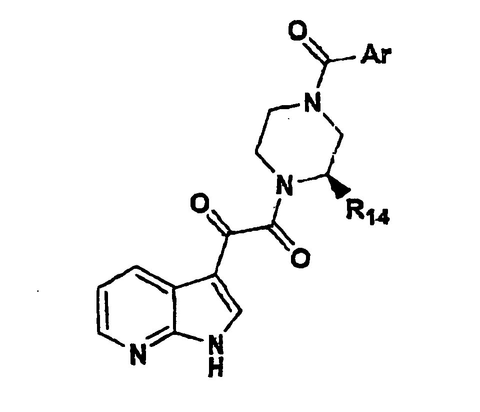

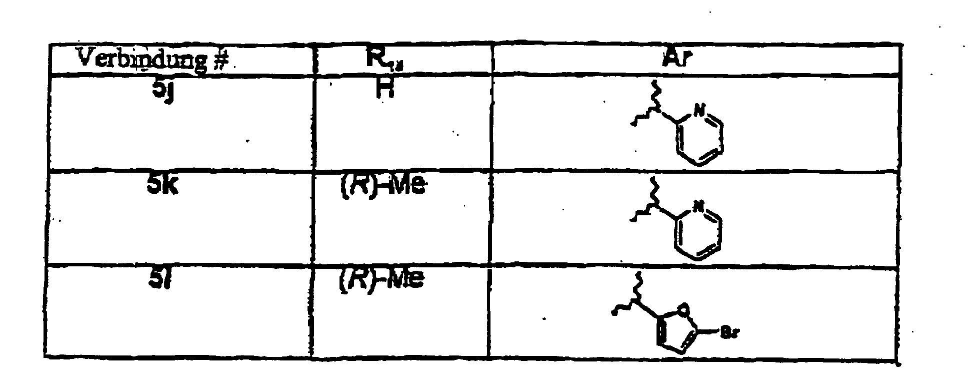

4. Verbindung nach Anspruch 1 oder ein pharmazeutisch verträgliches Salz davon mit der Formel 5m, wie nachstehend beschrieben:

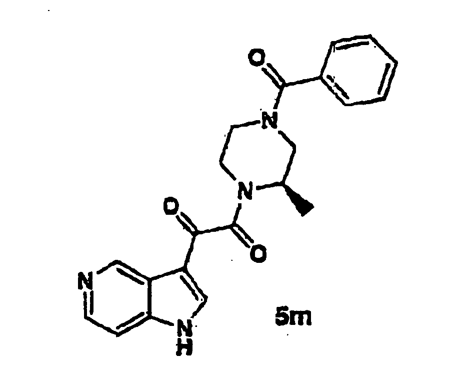

5. Verbindung nach Anspruch 1 oder ein pharmazeutisch verträgliches Salz davon, ausgewählt aus den Verbindungen 8a, 15a, 16a, 16d und 16e, wie nachstehend beschrieben: 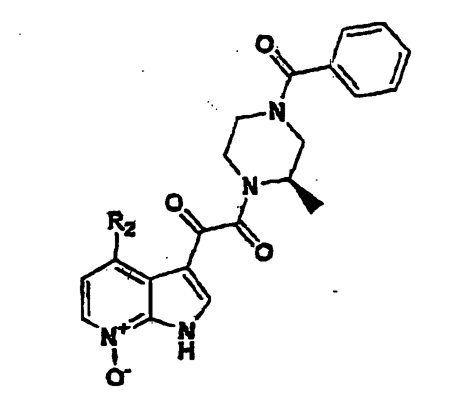
| **Verbindung #** | **R**_{**2**} |
|---|---|
| **8a** | H |
| **15a** | NO₂ |
| **16a** | OMe |
| **16d** | OEt |
| **16e** | SPr |

6. Verbindung nach Anspruch 1 oder ein pharmazeutisch verträgliches Salz davon, ausgewählt aus den Verbindungen 9a, 9b, 10a, 11a 11b, 11c, 12a, 14a, 17a-17f, 18a, 19a and 20a, wie nachstehend beschrieben: 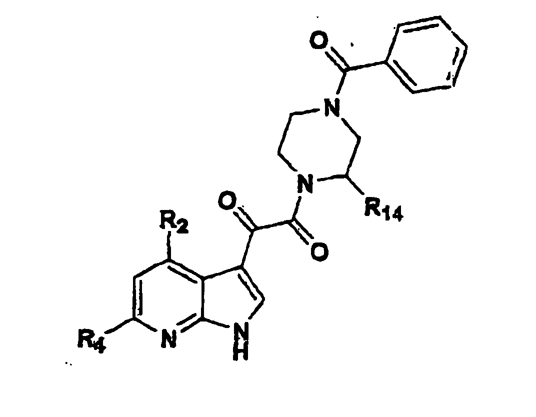
| **Verbindung #** | **R**_{**2**} | **R**_{**4**} | **R**_{**14**} |
|---|---|---|---|
| **9a** | Cl | H | (*R*)-Me |
| **9b** | H | Cl | (*R*)-Me |
| **10a** | NO₂ | F | (*R*)-Me |
| **11a** | H (when R₄=Me), Me (when R₄=H) | Me (when R₂=H), H (when R₂=Me) | (*R*)-Me |
| **11b** | H (when R₄=Ph), Ph (when R₄=H) | Ph (when R₂=H), H (when R₂=Ph) | (*R*)-Me |
| **11c** | H (when R₄=vinyl), Vinyl (when R₄=H) | Vinyl (when R₂=H), H (when R₂=Vinyl) | (*R*)-Me |
| **12a** | H | CN | (*R*)-Me |
| **14a** | H | OH | (*R*)-Me |
| **17a** | OMe | H | (*R*)-Me |
| **17d** | OMe | H | (*S*)-Me |
| **17e** | OMe | H | Me |
| **17b** | OCH₂CF₃ | H | (*R*)-Me |
| **17c** | O-*i-*Pr | H | (*R*)-Me |
| **17f** | H | PrS | (*R*)-Me |
| **18a** | NO₂ | H | (*R*)-Me |
| **19a** | NHOH | H | (*R*)-Me |
| **20a** | NH₂ | H | (*R*)-Me |

7. Verbindung nach Anspruch 6 oder ein pharmazeutisch verträgliches Salz davon, wobei R₂ für -OMe steht, R₄ für Wasserstoff steht und R₁₄ für (R)-Methyl steht.

8. Verbindung nach Anspruch 1 oder ein pharmazeutisch verträgliches Salz davon, ausgewählt aus den Verbindungen 13a, 21a und 21b, wie nachstehend beschrieben:

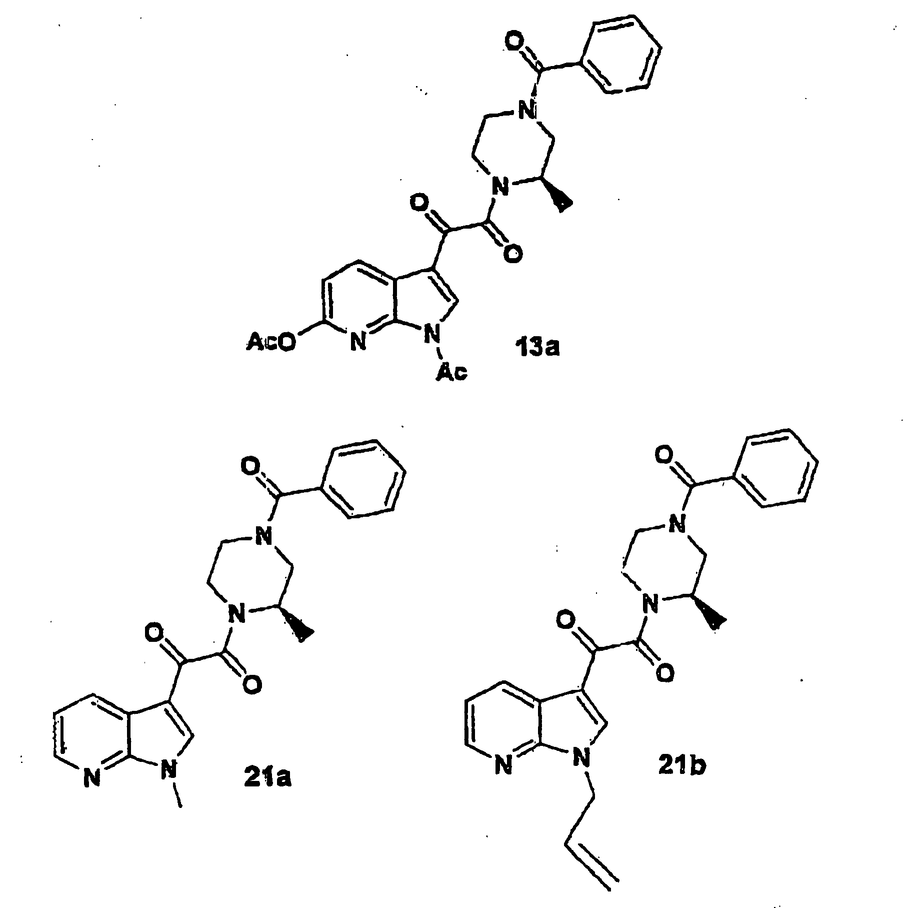

9. Verbindung nach Anspruch 1 oder ein pharmazeutisch verträgliches Salz, wobei R₂, R₃ und R₄ jeweils unabhängig voneinander ausgewählt sind aus H, -OCH₃, -OCH₂CF₃, -OiPr, -OnPr, Halogen, CN, NO₂, C₁-C₆-Alkyl, NHOH, NH₂, Ph, SR₂₀ und N(CH₃)₂.

10. Verbindung nach Anspruch 9 oder ein pharmazeutisch verträgliches Salz, wobei n gleich 2 ist; R₁ ausgewählt ist aus H, C₁-C₆-Alkyl und CH₂CH=CH₂; und R₅ für (O)ₘ, wobei m gleich 0 ist, steht.

11. Verbindung nach Anspruch 10 oder ein pharmazeutisch verträgliches Salz davon, wobei R₇₅ R₅, R₉, R_{10,} R₁₁, R₁₂, R₁₃ und R₁₄ jeweils unabhängig H oder CH₃ sind, mit der Maßgabe, dass ein oder zwei Mitglieder der Reste R₇-R₁₄ für CH₃ stehen und die restlichen Mitglieder der Reste R₇-R₁₄ für H stehen.

12. Verbindung nach Anspruch 11 oder ein pharmazeutisch verträgliches Salz davon, wobei eines der Mitglieder des Rests A₁, A₂, A₃, A₄, A₅, B₁, B₂, B₃, B₄, C₁, C₂, C₃, D₁, D₂ und D₃ ausgewählt sind aus Wasserstoff. Halogen und Amino und die restlichen Mitglieder des Rests A₁, A₂, A₃, A₄, As, B₁, B₂, B₃, B₄, C₁, C₂, C₃, D₁, D₂ und D₃ Wasserstoff sind.

13. Verbindung nach Ansprach 1 oder ein pharmazeutisch verträgliches Salz davon mit der nachstehenden Formel: 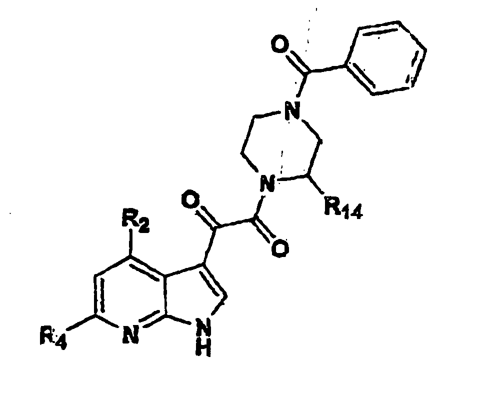 wobei
R₂ ausgewählt ist aus H, -OCH₃, -OCH₂CF₃, -OPr, Halogen, CN, NO₂ und NHOH;
R₄ ausgewählt ist aus H, -Halogen, -CN und Hydroxy; und
R₁₄ für CH₃ oder H steht.

14. Verbindung nach Anspruch 1, wobei R₄ ausgewählt ist aus OH, CN, Halogen, -OCOCH₃ und C₁-C₆-Alkyl.

15. Verbindung nach Anspruch 1 oder ein pharmazeutisch verträgliches Salz davon mit der nachstehend beschriebenen Formel: 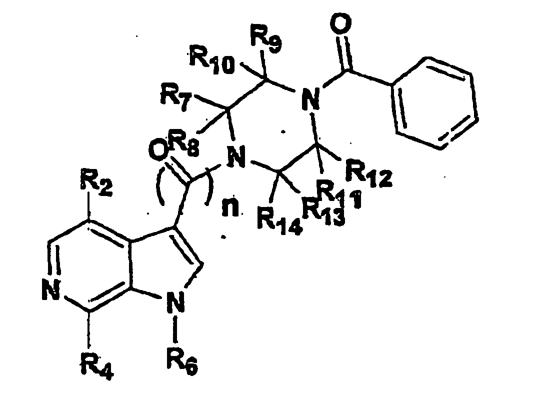 wobei
R₂ ausgewählt ist aus H, F, Cl, Br, OMe, CN und OH;
R₄ ausgewählt ist aus H, C₁-C₆-Alkyl, C₂-C₆-Alkenyl, C₃-C₆-Cycloalkyl, C₅-C₆-Cycloalkenyl, Cl, OMe, CN, OH, C(O)NH₂, C(O)NHMe, C(O)NHEt, Phenyl und -C(O)CH₃;
m gleich 2 ist;
R₈, R₉, R₁₀, R₁₁, R₁₂, R₁₃ und R₁₄ jeweils unabhängig H oder CH₃ sind, mit der Maßgabe, dass 0 bis 2 Mitglieder des Rests R₈, R₉, R₁₀, R₁₁, R₁₂, R₁₃ und R₁₄ für CH₃ stehen können und die restlichen Mitglieder des Rests R₈, R₉, R₁₀, R₁₁, R₁₂, R₁₃ und R₁₄ für H stehen; und
R₆ für H oder CH₃ steht.

16. Verbindung nach Anspruch 1 oder ein pharmazeutisch verträgliches Salz davon, ausgewählt aus den Verbindungen 5p, 5r, 5s, 5q, 5t, 5u, 5v und 27c, wie nachstehend beschrieben: 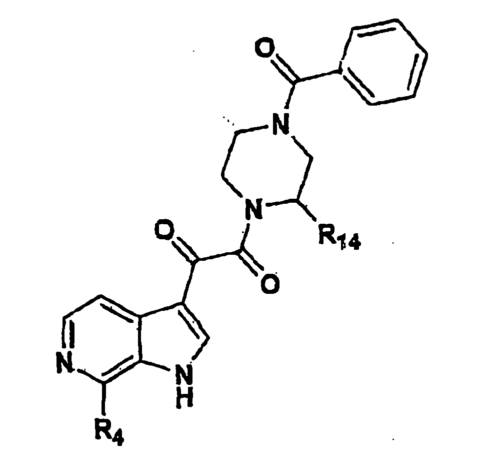
| **Verbindung #** | **R**_{**4**} | **R**_{**14**} |
|---|---|---|
| **5p** | H | H |
| **5r** | H | (*R*)-Me |
| **5s** | H | (*S*)-Me |
| **5q** | H | Me |
| **5t** | Cl | H |
| **5u** | Cl | (*R*)-Me |
| **5v** | OMe | (*R*)-Me |
| **27c** | NMe₂ | (*R*)-Me |

17. Verbindung nach Anspruch 1 oder ein pharmazeutisch verträgliches Salz davon mit der formel: 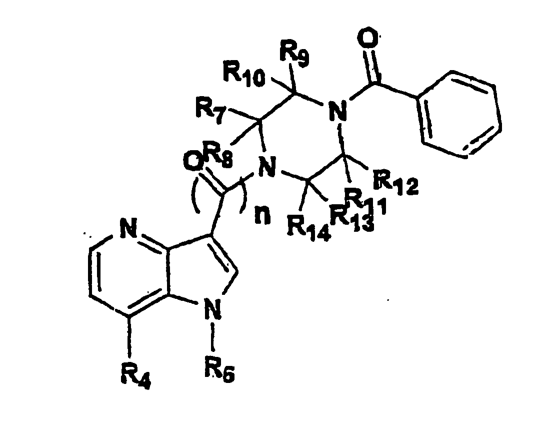 wobei
R₄ ausgewählt ist aus H, C₁-C₆-Alkyl, C₂-C₆-Alkenyl, C₃-C₆-Cycloalkyl, C₅-C₆-Cycloalkenyl, Cl, OMe, CN, OH, C(O)NH₂, C(O)NHMe, C(O)NHEt, Phenyl und -C(O)CH₃;
n gleich 2 ist;
R₈, R₉, R₁₀, R₁₁, R₁₂, R₁₃ und R₁₄ jeweils unabhängig H oder CH₃ sind, mit der Maßgabe, dass 0 bis 2 Mitglieder des Rests R₈, R₉, R₁₀, R₁₁, R₁₂, R₁₃ und R₁₄ für CH₃ stehen können und die restlichen Mitglieder des Rests R₈, R₉, R₁₀, R₁₁, R₁₂, R₁₃ und R₁₄ für H stehen; und
R₆ für H oder CH₃ steht.

18. Verbindung nach Anspruch 1 oder ein pharmazeutisch verträgliches Salz davon, ausgewählt aus den Verbindungen 5w, 5x, 5y, 5z und 5ak, wie nachstehend beschrieben: 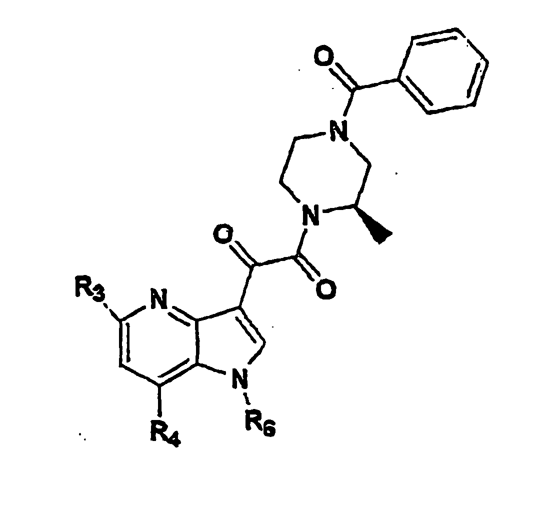
| **Verbindung #** | | **R**_{**4**} | **R**_{**6**} |
|---|---|---|---|
| **5w** | **H** | **H** | **H** |
| **5x** | **H** | **Me** | **H** |
| **5y** | **H** | **C** | **H** |
| **5z** | **H** | **OMe** | **Me** |
| **5ak** | **Cl** | **Me** | **H** |

19. Verbindung nach Anspruch 15, wobei R₄, R₇, R₈, R₉, R₁₀, R₁₁, R₁₂, R₁₃ und R₁₄ für H stehen; nud R₂ für -OMe steht.

20. Verbindung nach Anspruch 15, wobei R₂, R₄, R₇, R₈, R₉, R₁₀, R₁₁, R₁₂, R₁₃ und R₁₄ für H stehen.

21. Verbindung nach Anspruch 1 oder ein pharmazeutisch verträgliches Salz davon mit der Formel: 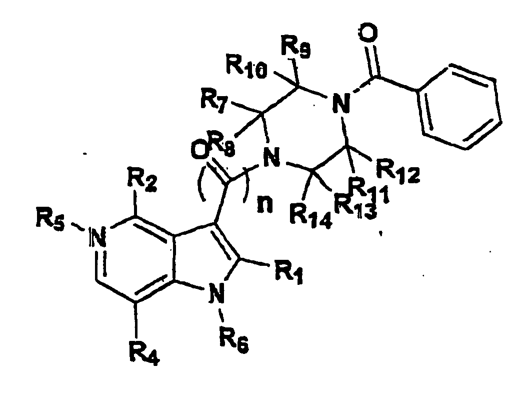 wobei
R₂ für H, F, Cl, Br, OMe, CN oder OH steht;
R₄ für C₁-C₆-Alkyl, C₂-C₆-Alkenyl, C₃-C₆-Cycloalkyl, C₅-C₆-Cycloalkenyl, Cl, OMe, CN, OH, C(O)NH₂, C(O)NHMe, C(O)NHEt, Ph oder -C(O)CH₃ steht;
n gleich 2 ist;
R₈, R₉, R₁₀, R₁₁, R₁₂, R₁₃ und R₁₄ jeweils unabhängig H oder CH₃ sind, mit der Maßgabe, dass bis zu 2 dieser Substituenten Methyl sein können;
R₁ für Wasserstoff steht;
R₅ unsubstituiert ist; und
R₆ für Wasserstoff oder Methyl steht.

22. Verbindung nach Anspruch 1 oder pharmazeutisch verträgliche Salze davon mit der Formel 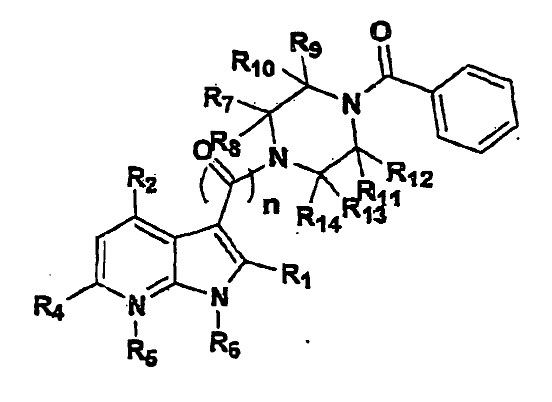 wobei
R₂ für H, -OCH₃, -OCH₂CF₃, -OPr, Halogen, CN, NO₂ oder NHOH steht;
R₄ für H, -Halogen, -CN oder Hydroxy steht;
ein oder zwei Mitglieder der Reste R₇-R₁₄ für Methyl stehen und die restlichen Mitglieder Wasserstoff sind;
n gleich 2 ist;
R₁ für Wasserstoff steht;
R₅ für (O)m steht, wobei m für O steht; und
R₆ für Wasserstoff, Methyl oder Allyl steht.

23. Arzneimittel, umfassend eine antiviral wirksame Menge einer Verbindung der Formel I, einschließlich pharmazeutisch verträglicher Salze davon nach einem der Ansprüche 1 bis 22.

24. Arzneimittel nach Anspruch 23, welches zur Behandlung einer Infektion mit HIV von Nutzen ist, das zusätzlich eine antiviral wirksame Menge eines AIDS Behandlungsmittels, ausgewählt aus:
(a) einem antiviralen Mittel gegen AIDS;
(b) einem infektionsverhindernden Mittel;
(c) einem Immunmodulator; und
(d) Hemmem gegen das Eindringen von HIV
umfasst.

25. Verwendung einer Verbindung der Formel I, einschließlich pharmazeutisch verträglicher Salze davon, nach einem der Ansprüche 1 bis 22, zur Herstellung eines Medikaments zur Behandlung von Säugern, die mit einem Virus infiziert sind, durch Verabreichung einer antiviral wirksamen Menge an den Säuger.

26. Verwendung nach Anspruch 25, wobei die antiviral wirksame Menge einer Verbindung der Formel I in Kombination mit einer antiviral wirksamen Menge eines AIDS Behandlungsmittels, ausgewählt aus: einem antiviralen Mittel gegen AIDS, einem infektionsverhindernden Mittel; einem Immunmodulator und Hemmern gegen das Eindringen von HIV, vorliegt.

27. Verwendung nach Anspruch 25 oder 26, wobei der Virus HIV ist.

28. Verbindung nach einem der Ansprüche 1 bis 22 zur, Verwendung als Medikament.

## Revendications

1. Composé de formule I, ou son sel pharmaceutiquement acceptable, 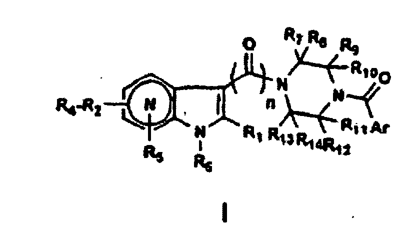 où: 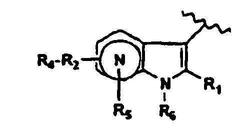 est sélectionné dans le groupe consistant en: 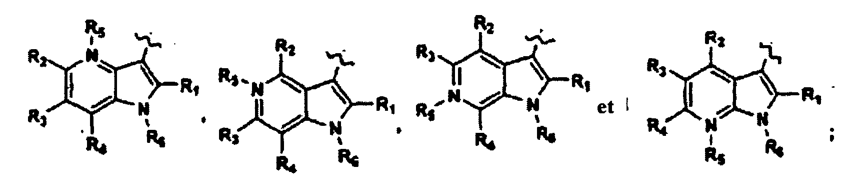 R₁,R₂,R₃,R₄ sont chacun indépendamment sélectionnés dans le groupe consistant en H, alkyle C₁-C₆, cycloalkyle C₃-C₆, alcényle C₂-C₆, cycloalcényle C₄-C₆, alkynyle C₂-C₆, halogène, CN, phényle,nitro, OC(O)R₁₅, C(O)R₁₅, C(O)OR₁₆, C(O)NR₁₇R₁₈, OR₁₉, SR₂₀ et NR₂₁R₂₂;
R₁₅, est indépendamment sélectionné dans le groupe consistant en H, alkyle C₁-C₆, cycloalkyle C₃-C₆, alcényle C₂-C₆ et cycloalcényle C₄-C₆;
R₁₆, R₁₉ et R₂₀ sont chacun indépendamment sélectionnés dans le groupe consistant en H, alkyle C₁-C₆, alkyle C₁-C₆ substitué par un à trois atomes d'halogène, cycloalkyle C₃-C₆, alcényle C₂-C₆, cycloalcényle C₄-C₆, et alkynyle C₃-C₆; à condition que les atomes de carbone qui forment la triple liaison carbone-carbone dudit alkynyle C₃-C₆ ne soient pas le point d'attachement à l'oxygène ou au soufre auquel est attaché R₁₆, R₁₉ ou R₂₀;
R₁₇ et R₁₈ sont chacun indépendamment sélectionnés dans le groupe consistant en H, alkyle C₁-C₆, cycloalkyle C₃-C₆, alcényle C₃-C₆, cycloalcényle C₄-C₆, et alkynyle C₃-C₆; à condition que les atomes de carbone qui forment la double laison carbone-carbone dudit alcényle C₃-C₆ ou la triple liaison carbone-carbone dudit alkynyle C₃-C₆ ne soient pas le point d'attachement à l'azote auquel R₁₇ et R₁₈ est attaché;
R₂₁ et R₂₂ sont chacun indépendamment sélectionnés dans le groupe consistant en H, OH, alkyle C₁-C₆, cycloalkyle C₃-C₆, alcényle C₃-C₆, cycloalcényle C₅-C₆, alkynyle C₃-C₆ et C(O)R₂₃; à condition que les atomes de carbone qui forment la double liaison carbone-carbone dudit alcényle C₃-C₆, cycloalcényle C₄-C₆, ou la triple liaison carbone-carbone dudit alkynyle C₃-C₆ ne soient pas le point d'attachement à l'azote auquel est attaché R₂₁ et R₂₂;
R₂₃ est sélectionné dans le groupe consistant en H, alkyle C₁-C₆, cycloalkyle C₃-C₆, alcényle C₂-C₆, cycloalcényle C₄-C₆ et alkynyle C₂-C₆;
R₅ est (O)m, où m est 0 ou 1;
n est 1 ou 2;
R₆ est sélectionné dans le groupe consistant en H, alkyle C₁-C₆, cycloalkyle C₃-C₆, cycloalcényle C₄-C₆, C(O)R₂₄, C (O) OR₂₅, C (O) NR₂₆R₂₇, alcényle C₃-C₆, et alkynyle C₃-C₆; à condition que les atomes de carbone qui forment la double liaison carbone-carbone dudit alcényle C₃-C₆ ou la triple liaison carbone-carbone dudit alkynyle C₃-C₆ ne soient pas le point d'attachement à l'azote auquel R₆ est attaché;
R₂₄ est sélectionné dans le groupe consistant en H, alkyle C₁-C₆, cycloalkyle C₃-C₆-; alcényle C₃-C₆, cycloalcényle C₄-C₆, et alkynyle C₃-C₆;
R₂₅ est sélectionné dans le groupe consistant en alkyle C₁-C₆, cycloalkyle C₃-C₆, alcényle C₂-C₆, cycloalcényle C₄-C₆, et alkynyle C₃-C₆; à condition que les atomes de carbone qui forment la triple liaison carbone-carbone dudit alkynyle C₃-C₆ ne soient pas le point d'attachement à l'oxygène auquel R₂₅ est attaché;
R₂₆ et R₂₇ sont chacun indépendamment sélectionnés dans le groupe consistant en H, alkyle C₁-C₆, cycloalkyle C₃-C₆, alcényle C₃-C₆, cycloalcényle C₅-C₆, et alkynyle C₃-C₆; à condition que les atomes de carbone qui forment la double liaison carbone-carbone dudit alcényle C₃-C₆, cycloalcényle C₅-C₆, ou la triple liaison carbone-carbone dudit alkynyle C₃-C₆ ne soient pas le point d'attachement à l'azote auquel R₂₆ et R₂₇ sont attachés;
R₇, R₈, R₉, R₁₀, R₁₁, R₁₂, R₁₃ et R₁₄ sont chacun indépendamment sélectionnés dans le groupe consistant en H, alkyle C₁-C₆, cycloalkyle C₃-C₆, alcényle C₂-C₆, cycloalcényle C₄-C₆, alkynyle C₂-C₆, CR₂₈R₂₉OR₃₀, C(O)R₃₁, CR₃₂(OR₃₃)OR₃₄, CR₃₅NR₃₆R₃₇, C (O) OR₃₈, C(O)NR₃₉R₄₀, CR₄₁R₄₂F, CR₄₃F₂ et CF₃;
R₂₈, R₂₉, R₃₀, R₃₁, R₃₂, R₃₅, R₄₁, R₄₂ et R₄₃ sont chacun indépendamment sélectionnés dans le groupe consistant en H, alkyle C₁-C₆, cycloalkyle C₃-C₆, alcényle C₂-C₆, cycloalcényle C₄-C₆, alkynyle C₂-C₆ et C (O) R₄₄;
R₃₃, R₃₄ et R₃₈ sont chacun indépendamment sélectionnés dans le groupe consistant en H, alkyle C₁-C₆, cycloalkyle C₃-C₆, alcényle C₃-C₆, cycloalkyle C₄-C₆, alkynyle C₃-C₆; à condition que les atomes de carbone qui forment la triple liaison carbone-carbone dudit alkynyle C₃-C₆ ne soient pas le point d'attachement à l'oxygène auquel sont attachés R₃₄ et R₃₈;
R₃₆ et R₃₇ sont chacun indépendamment sélectionnés dans le groupe consistant en H, alkyle C₁-C₆, cycloalkyle C₃-C₆, alcényle C₃-C₆, cycloalcényle C₄-C₆ et alkynyle C₃-C₆; à condition que les atomes de carbone qui forment la triple liaison carbone-carbone dudit alkynyle C₃-C₆ ne soient pas le point d'attachement à l'azote auquel sont attachés R₃₆ et R₃₇ ;
R₃₉ et R₄₀ sont chacun indépendamment sélectionnés dans le groupe consistant en H, alkyle C₁-C₆, cycloalkyle C₃-C₆, alcényle C₂-C₆, cycloalcényle C₄-C₆, et alkynyle C₃-C₆; à condition que les atomes de carbone qui forment la triple liaison carbone-carbone dudit alkynyle C₃-C₆ ne soient pas le point d'attachement à l'azote auquel sont attachés R₃₉ et R₄₀ ;
R₄₄ est sélectionné dans le groupe consistant en H, alkyle C₁-C₆, cycloalkyle C₃-C₆, alcényle C₂-C₆, cycloalcényle C₄-C₆ et alkynyle C₂-C₆;
Ar est sélectionné dans le groupe consistant en 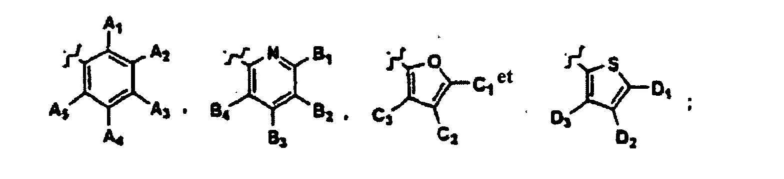 A₁, A₂, A₃, A₄, A₅, B₁, B₂, B₃, B₄, C₁, C₂, C₃, D₁, D₂, et D₃ sont chacun indépendamment sélectionnés dans le groupe consistant en H, CN, halogène, NO₂, alkyle C₁-C₆, cycloalkyle C₃-C₆, alcényle C₂-C₆, cycloalcényle C₄-C₆, alkynyle C₂-C₆, OR₄₅, NR₄₆R₄₇, SR₄₈, N₃ et CH (-N=N-) -CF₃;
R₄₅ est sélectionné dans le groupe consistant en H, alkyle C₁-C₆, cycloalkyle C₃-C₆, alcényle C₂-C₆, cycloalcényle C₄-C₆ et alkynyle C₃-C₆; à condition que les atomes de carbone qui forment la triple liaison carbone-carbone dudit alkynyle C₃-C₆ ne soient pas le point d'attachement à l'oxygène auquel est attaché R₄₅;
R₄₆ et R₄₇ sont chacun indépendamment sélectionnés dans le groupe consistant en H, alkyle C₁-C₆, cycloalkyle C₃-C₆, alcényle C₃-C₆, cycloalcényle C₅-C₆, alkynyle C₃-C₆ et C (O) R₅₀; à condition que les atomes de carbone qui forment la double liaison carbone-carbone dudit alcényle C₅-C₆, cycloalcényle C₄-C₆, ou la triple liaison carbone-carbone dudit alkynyle C₃-C₆ ne soient pas le point d'attachement à l'azote auquel R₄₆ et R₄₇ sont attachés;
R₄₈ est sélectionné dans le groupe consistant en H, alkyle C₁-C₆, cycloalkyle C₃-C₆, alcényle C₂-C₆, cycloalcényle C₄-C₆, alkynyle C₃-C₆ et C(O)R₄₉; à condition que les atomes de carbone qui forment la triple liaison carbone-carbone dudit alkynyle C₃-C₆ ne soient pas le point d'attachement au soufre auquel R₄₈ est attaché;
R₄₉ est alkyle C₁-C₆ ou cycloalkyle C₃-C₆; et
R₅₀ est sélectionné dans le groupe consistant en H, alkyle C₁-C₆, et cycloalkyle C₃-C₆.

2. Composé de la revendication 1, ou son sel pharmaceutiquement acceptable sélectionné dans le groupe consistant en composés 5a, 5b, 5c, 5d, 5e, 5f, 5g, 5h, 5i et 5ai comme identifiés ci-dessous: 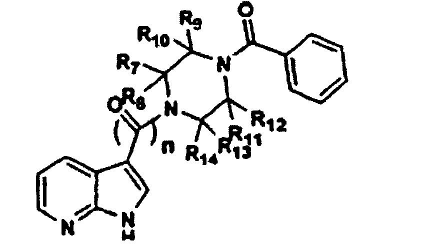
| Composé # | **n** | **R** |
|---|---|---|
| **5a** | **2** | **R**_{**7-13**} **= H, R**_{**14**} **= (*R*)-Me** |
| **5b** | **2** | **R**_{**7-8**} **= R**_{**10-14**} **= H, R**_{**9**} **= ET** |
| **5c** | **1** | **R**_{**7-8**} **= R**_{**10-14**} **= H, R**_{**9**} **= Et** |
| **5d** | **2** | **R**_{**7-14**} **= H** |
| **5e** | **2** | **R**_{**7-8**} **= R**_{**10-14**} **= H, R**_{**9**} **= Me** |
| **5f** | **2** | **R**_{**7-13**} **= H, R**_{**14**} **= (S)-Me** |
| **5g** | **2** | **R**_{**7-13**} **= H, R**_{**14**} **= Et** |
| **5h** | **2** | **R**_{**7-12**}**= H, R**_{**13**} **= R**_{**14**} **= Me** |
| **5i** | **2** | **R**_{**7-8**} **= R**_{**10-13**} **= H, R**_{**9**} **= R**_{**14**} **= Me** |
| **5ai** | **2** | **R**_{**7-8**} **= R**_{**9-13**} **= H, R**_{**14**} **= Me** |

3. Composé de la revendication 1, ou son sel pharmaceutiquement acceptable, sélectionné dans le groupe consistant en composés 5j, 5k et 5l comme identifiés ci-dessous:

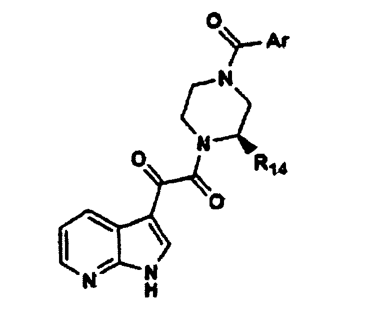

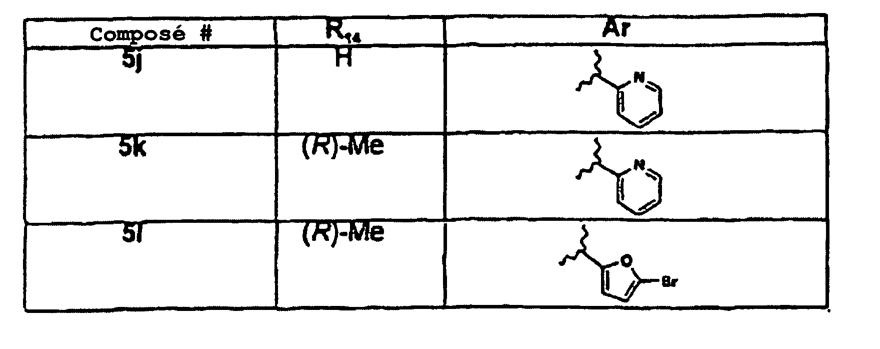

4. Composé de la revendication 1, ou son sel pharmaceutiquement acceptable, ayant la formule 5m identifiée ci-dessous:

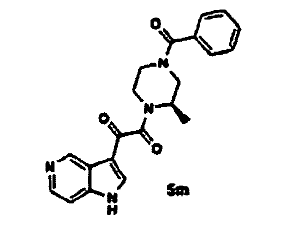

5. Composé de la revendication 1, ou son sel pharmaceutiquement acceptable, est sélectionné dans le groupe consistant en composés 8a, 15a, 16a, 16d et 16e identifiés ci-dessous: 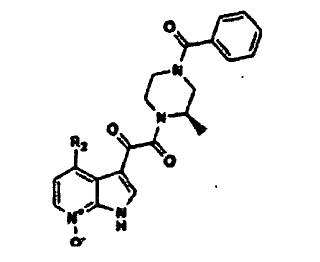
| Composé # | **R**_{**2**} |
|---|---|
| **8a** | **H** |
| **15a** | **NO**_{**2**} |
| **16a** | **OMe** |
| **16d** | **OEt** |
| **16e** | **SPr** |

6. Composé de la revendication 1, ou son sel pharmaceutiquement acceptable, sélectionné dans le groupe consistant en composés 9a, 9b, 10a, 11a, 11b, 11c, 12a, 14a, 17a-17f, 18a, 19a et 20a identifiés ci-dessous: 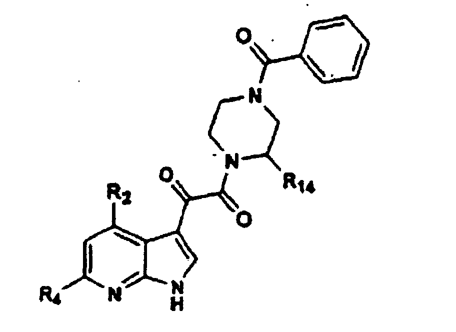
| Comp. **#** | **R**_{**2**} | **R**_{**4**} | **R**_{**14**} |
|---|---|---|---|
| **9a** | Cl | H | (*R*)-Me |
| **9b** | H | Cl | (*R*)-Me |
| **10a** | NO₂ | F | (*R*)-Me |
| **11a** | H (when R₄=Me), Me (when R₄=H) | Me (when R₂=H), H (when R₂=Me) | (*R*)-Me |
| **11b** | H (when R₄=Ph), Ph (when R₄=H) | Ph (when R₂=H), H (when R₂=Ph) | (*R*)-Me |
| **11c** | H (when R₄=vinyl), Vinyl (when R₄=H) | Vinyl (when R₂=H), H (when R₂=Vinyl) | (*R*)-Me |
| **12a** | H | CN | (*R*)-Me |
| **14a** | H | OH | (*R*)-Me |
| **17a** | OMe | H | (*R*)-Me |
| **17d** | OMe | H | (*S*)-Me |
| **17e** | OMe | H | Me |
| **17b** | OCH₂CF₃ | H | (*R*)-Me |
| **17c** | O-*i-*Pr | H | (*R*)-Me |
| **17f** | H | PrS | (*R*)-Me |
| **18a** | NO₂ | H | (*R*)-Me |
| **19a** | NHOH | H | (*R*)-Me |
| **20a** | NH₂ | H | (*R*)-Me |

7. Composé de la revendication 6 ou son sel pharmaceutiquement acceptable, où R₂ est -OMe, R₄ est hydrogène, et R₁₄ est (R)-méthyle.

8. Composé de la revendication 1, ou son sel pharmaceutiquement acceptable, sélectionné dans le groupe consistant en composés 13a, 21a, et 21b identifiés ci-dessous:

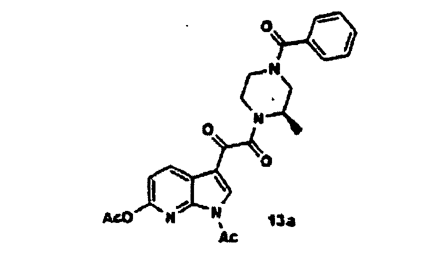

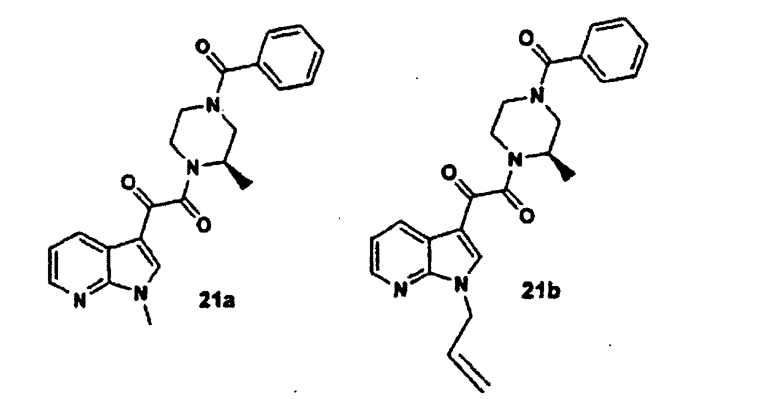

9. Composé de la revendication 1, ou son sel pharmaceutiquement acceptable où R₂, R₃ et R₄ sont chacun indépendamment sélectionnés dans le groupe consistant en H, -OCH₃, -OCH₂CF₃, -OiPr, -OnPr, halogène, CN, NO₂, alkyle C₁-C₆, NHOH, NH₂, Ph, SR₂₀, et N(CH₃)₂.

10. Composé de la revendication 9, ou son sel pharmaceutiquement acceptable, où n est 2; R₁ est sélectionné dans le groupe consistant en H, alkyle C₁-C₆, CH₂CH=CH₂; et R₅ est (O)ₘ où m est 0.

11. Composé de la revendication 10, ou son sel pharmaceutiquement acceptable, où R₇, R₈, R₉, R₁₀, R₁₁, R₁₂, R₁₃ et R₁₄ sont chacun indépendamment H ou CH₃, à condition qu'un ou deux des membres du groupe R₇-R₁₄ soit CH₃ et que les membres restants du groupe R₇-R₁₄ soient H.

12. Composé de la revendication 11, ou son sel pharmaceutiquement acceptable, où l'un des membres du groupe A₁, A₂, A₃, A₄, A₅, B₁, B₂, B₃, B₄, C₁, C₂, C₃, D₁, D₂, et D₃ est sélectionné dans le groupe consistant en hydrogène, halogène et amino et les membres restants du groupe A₁, A₂, A₃, A₄, A₅, B₁, B₂, B₃, B₄, C₁, C₂, C₃, D₁, D₂, et D₃ sont hydrogène.

13. Composé de la revendication 1, ou son sel pharmaceutiquement acceptable de la formule ci-dessous: 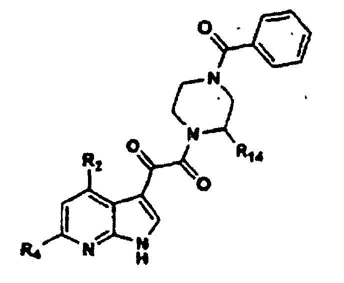 où:
R₂ est sélectionné dans le groupe consistant en H, -OCH₃, -OCH₂CF₃, -OPr, halogène, CN, NO₂- et NHOH;
R₄ est sélectionné dans le groupe consistant en H, -halogène, -CN, et hydroxy; et
R₁₄ est CH₃ ou H.

14. Composé de la revendication 1, où R₄ est sélectionné dans le groupe consistant en OH, CN, halogène, -OCOCH₃ et alkyle C₁-C₆.

15. Composé de la revendication 1, ou son sel pharmaceutiquement acceptable de la formule identifiée ci-dessous: 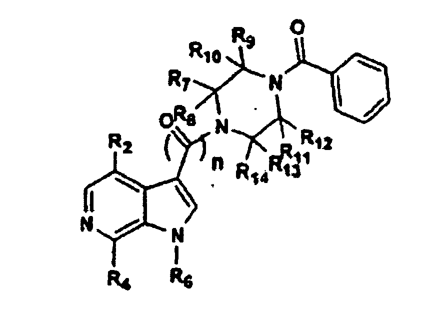 où:
R₂ est sélectionné dans le groupe consistant en H, F, Cl, Br, OMe, CN, et OH;
R₄ est sélectionné dans le groupe consistant en H, alkyle C₁-C₆, alcényle C₂-C₆, cycloalkyle C₃-C₆, cycloalcényle C₅-C₆, Cl, OMe, CN, OH, C(O)NH₂, C(O)NHMe, C(O)NHEt, phényle et -C (O) CH₃;
n est 2;
R₈, R₉, R₁₀, R₁₁, R₁₂, R₁₃, et R₁₄ sont chacun indépendamment H ou CH₃, à condition que 0-2 des membres du groupe R₈, R₉, R₁₀, R₁₁, R₁₂, R₁₃, et R₁₄ puissent être CH₃ et que les membres restants du groupe R₈, R₉, R₁₀, R₁₁, R₁₂, R₁₃, et R₁₄ soient H; et
R₆ est H ou CH₃.

16. Composé de la revendication 1, ou son sel pharmaceutiquement acceptable, sélectionné dans le groupe consistant en composés 5p, 5r, 5s, 5q, 5t, 5u, 5v et 27c identifiés ci-dessous: 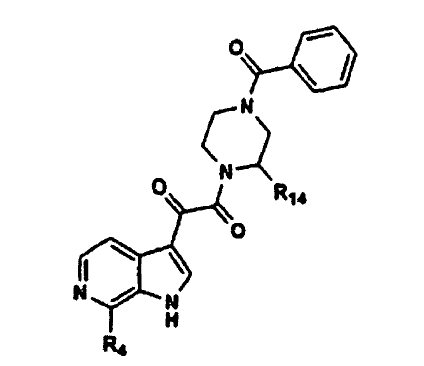
| Composé # | **R**_{**4**} | **R**_{**14**} |
|---|---|---|
| **5p** | **H** | **H** |
| **5r** | **H** | **(*****R*****)-Me** |
| **5s** | **H** | **(*****S*****)-Me** |
| **5q** | **H** | **Me** |
| **5t** | **Cl** | **H** |
| **5u** | **Cl** | **(*****R*****)-Me** |
| **5v** | **OMe** | **(*****R*****)-Me** |
| **27c** | **NMe**_{**2**} | **(*****R*****)-Me** |

17. Composé de la revendication 1, ou son sel pharmaceutiquement acceptable de la formule: 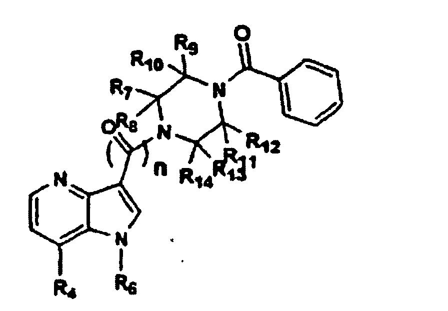 où:
R₄ est sélectionné dans le groupe consistant en H, alkyle C₁-C₆, alcényle C₂-C₆, cycloalkyle C₃-C₆, cycloalcényle C₅-C₆, Cl, OMe, CN, OH, C (O) NH₂, C(O)NHMe, C (O) NHEt, phényle et -C (O) CH₃;
n est 2;
R₈, R₉, R₁₀, R₁₁, R₁₂, R₁₃, et R₁₄ sont chacun indépendamment H ou CH₃, à condition que 0-2 des membres du groupe R₈, R₉, R₁₀, R₁₁, R₁₂, R₁₃, et R₁₄ puissent être CH₃ et que les membres restants du groupe R₈, R₉, R₁₀, R₁₁, R₁₂, R₁₃, et R₁₄ soient H; et
R₆ est H ou CH₃.

18. Composé de la revendication 1, ou son sel pharmaceutiquement acceptable, sélectionné dans le groupe consistant en composés 5w, 5x, 5y, 5z et 5ak identifiés ci-dessous: 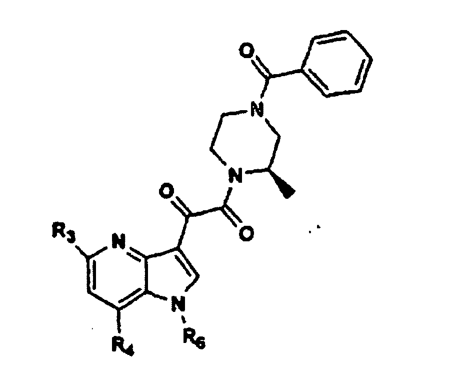
| Composé # | **R**_{**3**} | **R**_{**4**} | **R**_{**6**} |
|---|---|---|---|
| **5w** | **H** | **H** | **H** |
| **5x** | **H** | **Me** | **R** |
| **5y** | **H** | **Cl** | **H** |
| **5z** | **H** | **OMe** | **Me** |
| **5ak** | **Cl** | **Me** | **H** |

19. Composé de la revendication 15 où R₄, R₇, R₈, R₉, R₁₀, R₁₁, R₁₂, R₁₃ et R₁₄ sont H; et R₂ est -OMe.

20. Composé de la revendication 15 où R₂, R₄, R₇, R₈, R₉, R₁₀, R₁₁, R₁₂, R₁₃ et R₁₄ sont H.

21. Composé de la revendication 1, ou son sel pharmaceutiquement acceptable, ayant la formule 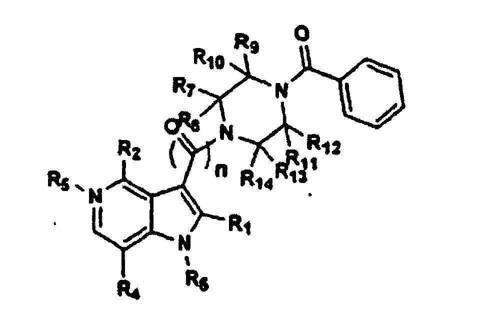 OÙ:
R₂ est H, F, Cl, Br, OMe, CN, ou OH;
R₄ est alkyle C₁-C₆, alcényle C₂-C₆, cycloalkyle C₃-C₆, cycloalcényle C₅-C₆, Cl, OMe, CN, OH, C (O) NH₂, C (O) NHMe, C (O) NHEt, Ph ou -C (O) CH₃;
n est 2;
R₈, R₉, R₁₀, R₁₁, R₁₂, R₁₃ et R₁₄ sont chacun indépendamment H ou CH₃, à condition que jusqu'à deux de ces substituants puissent être méthyle;
R₁ est hydrogène;
R₅ est non substitué; et
R₆ est hydrogène ou méthyle.

22. Composé de la revendication 1, ou ses sels pharmaceutiquement acceptables, de la Formule 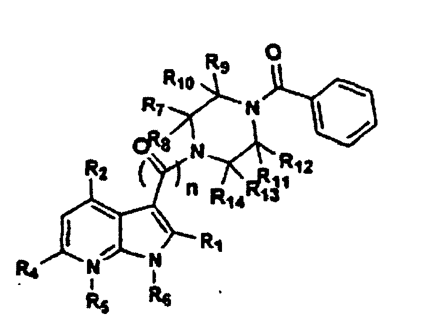 où:
R₂ est H, -OCH₃, -OCH₂CF₃, -OPr, halogène, CN, NO₂, ou NHOH;
R₄ est H, -halogène, -CN, ou hydroxy;
Un ou deux membres de R₇-R₁₄ est méthyle et les membres restants sont hydrogène;
n est 2;
R₁ est hydrogène;
R₅ est (O)ₘ, où m est O; et
R₆ est hydrogène, méthyle ou allyle.

23. Composition pharmaceutique qui comprend une quantité antivirale efficace d'un composé de Formule I, comprenant ses sels pharmaceutiquement acceptables, selon l'une quelconque des revendications 1-22.

24. Composition pharmaceutique de la revendication 23, utile pour le traitement d'une infection par VIH, qui comprend additionnellement une quantité antivirale efficace d'un agent de traitement du SIDA sélectionné dans le groupe consistant en:
(a) un agent antiviral du SIDA;
(b) un agent anti-infectieux;
(c) un immunomodulateur; et
(d) des inhibiteurs d'entrée de VIH.

25. Utilisation d'un composé de Formule I, comprenant ses sels pharmaceutiquement acceptables selon l'une quelconque des revendications 1-22, pour la fabrication d'un médicament pour le traitement des mammifères infectés par un virus, par administration audit mammifère d'une quantité antivirale efficace.

26. Utilisation de la revendication 25, où ladite quantité antivirale efficace d'un composé de Formule I est en combinaison avec une quantité antivirale efficace d'un agent de traitement du SIDA sélectionné dans le groupe consistant en un agent antiviral du SIDA; un agent anti-infectieux; un immunomodulateur; et des inhibiteurs d'entrée de VIH.

27. Utilisation de la revendication 25 ou 26 où le virus est VIH.

28. Composé de l'une quelconque des revendications 1-22 pour une utilisation comme médicament.
